# EUROPEAN PATENT APPLICATION

(11) **EP 3 483 272 A1**
(43) Date of publication of application: **15.05.2019**
(21) Application number: 18179176.5
(22) Date of filing: 21.06.2018
(51) Int. Cl.: C12N 15/113

(54) **PRODUCTS AND COMPOSITIONS**

(30) Priority: 13.11.2017 EP 17201447
(71) Applicant: Silence Therapeutics GmbH, 13125 Berlin (DE)
(72) Inventor: DAMES, Sibylle, 12435 Berlin (DE); SCHAEPER, Ute, 12125 Berlin (DE); SCHUBERT, Steffen, 14199 Berlin (DE); TENBAUM, Stephan, 88400 Biberach an der Riss (DE); BETHGE, Lucas, 14482 Potsdam (DE); FRAUENDORF, Christian, 13127 Berlin (DE); HAUPTMANN, Judith, 13125 Berlin (DE); WEINGÄRTNER, Adrien, 10318 Berlin (DE)
(74) Representative: CMS Cameron McKenna Nabarro Olswang LLP

(57) **Abstract**

The present invention relates to products and compositions and their uses. In particular the invention relates to nucleic acid products that interfere with the *ALDH2* gene expression or inhibits its expression and therapeutic uses such as for the treatment or prevention of alcohol use disorder.

SEQUENCE LISTING

<110>  Silence Therapeutics GmbH

<120>  Products and Compositions

<130>  P11530EP02

<105>  EP17201447.4
<151>  13.11.2017

<160>  54

<170>  PatentIn version 3.5

<210>  1
<211>  19
<212>  RNA
<213>  Artificial seq

<220>
<223>  siRNA sequence

<400>  1
uuguuuauga aaaucuggu                                                    19


<210>  2
<211>  19
<212>  RNA
<213>  Artificial seq

<220>
<223>  siRNA sequence

<400>  2
accagauuuu cauaaacaa                                                    19


<210>  3
<211>  19
<212>  RNA
<213>  Artificial seq

<220>
<223>  siRNA sequence

<400>  3
aauguuuucc ugcugacgg                                                    19


<210>  4
<211>  19
<212>  RNA
<213>  Artificial seq

<220>
<223>  siRNA sequence

<400>  4
ccgucagcag gaaaacauu                                                    19

<210>  5
<211>  19
<212>  RNA
<213>  Artificial seq

<220>
<223>  siRNA sequence

<400>  5
uugaacuuca ggaucugca                                                    19


<210>  6
<211>  19
<212>  RNA
<213>  Artificial seq

<220>
<223>  siRNA sequence

<400>  6
ugcagauccu gaaguucaa                                                    19


<210>  7
<211>  19
<212>  RNA
<213>  Artificial seq

<220>
<223>  siRNA sequence

<400>  7
uuucacuuca guguaugcc                                                    19


<210>  8
<211>  19
<212>  RNA
<213>  Artificial seq

<220>
<223>  siRNA sequence

<400>  8
ggcauacacu gaagugaaa                                                    19


<210>  9
<211>  19
<212>  RNA
<213>  Artificial seq

<220>
<223>  siRNA sequence

<400>  9
uucuuaugag uucuucuga                                                    19


<210>  10
<211>  19
<212>  RNA

<213>  Artificial seq

<220>
<223>  siRNA sequence

<400>  10
ucagaagaac ucauaagaa                                                    19


<210>  11
<211>  19
<212>  RNA
<213>  Artificial seq

<220>
<223>  siRNA sequence

<400>  11
ucuucuuaaa cugaguuuc                                                    19


<210>  12
<211>  19
<212>  RNA
<213>  Artificial seq

<220>
<223>  siRNA sequence

<400>  12
gaaacucagu uuaagaaga                                                    19


<210>  13
<211>  19
<212>  RNA
<213>  Artificial seq

<220>
<223>  siRNA sequence

<400>  13
uccuugauca gguuggcca                                                    19


<210>  14
<211>  19
<212>  RNA
<213>  Artificial seq

<220>
<223>  siRNA sequence

<400>  14
uggccaaccu gaucaagga                                                    19


<210>  15
<211>  19
<212>  RNA
<213>  Artificial seq

<220>

<223>  siRNA sequence

<400>  15
uugaagaaca gggcgaagu                                                    19


<210>  16
<211>  19
<212>  RNA
<213>  Artificial seq

<220>
<223>  siRNA sequence

<400>  16
acuucgcccu guucuucaa                                                    19


<210>  17
<211>  19
<212>  RNA
<213>  Artificial seq

<220>
<223>  siRNA sequence

<400>  17
auguagccga ggaucuucu                                                    19


<210>  18
<211>  19
<212>  RNA
<213>  Artificial seq

<220>
<223>  siRNA sequence

<400>  18
agaagauccu cggcuacau                                                    19


<210>  19
<211>  19
<212>  RNA
<213>  Artificial seq

<220>
<223>  siRNA sequence

<400>  19
aucugguugc agaagaccu                                                    19


<210>  20
<211>  19
<212>  RNA
<213>  Artificial seq

<220>
<223>  siRNA sequence

<400>  20

aggucuucug caaccagau                                                    19


<210>  21
<211>  19
<212>  RNA
<213>  Artificial seq

<220>
<223>  siRNA sequence

<400>  21
aaauccacca gguaggaga                                                    19


<210>  22
<211>  19
<212>  RNA
<213>  Artificial seq

<220>
<223>  siRNA sequence

<400>  22
ucuccuaccu gguggauuu                                                    19


<210>  23
<211>  19
<212>  RNA
<213>  Artificial seq

<220>
<223>  siRNA sequence

<400>  23
acucucuuga gguugcugc                                                    19


<210>  24
<211>  19
<212>  RNA
<213>  Artificial seq

<220>
<223>  siRNA sequence

<400>  24
gcagcaaccu caagagagu                                                    19


<210>  25
<211>  19
<212>  RNA
<213>  Artificial seq

<220>
<223>  siRNA sequence

<400>  25
auguccaaau ccaccaggu                                                    19

<210>  26
<211>  19
<212>  RNA
<213>  Artificial seq

<220>
<223>  siRNA sequence

<400>  26
accuggugga uuuggacau                                                    19


<210>  27
<211>  19
<212>  RNA
<213>  Artificial seq

<220>
<223>  siRNA sequence

<400>  27
ccaugcuugc aucaggagc                                                    19


<210>  28
<211>  19
<212>  RNA
<213>  Artificial seq

<220>
<223>  siRNA sequence

<400>  28
gcuccugaug caagcaugg                                                    19


<210>  29
<211>  22
<212>  DNA
<213>  Homo sapien/Macaca fascicularis

<400>  29
ggcaagccct atgtcatctc ct                                                22


<210>  30
<211>  22
<212>  DNA
<213>  Macaca fascicularis

<400>  30
ggatggtttt cccgtggtac tt                                                22


<210>  31
<211>  29
<212>  DNA
<213>  Artificial seq

<220>
<223>  siRNA sequence

<400>  31

tggtcctcaa atgtctccgg tattatgcc                                          29

<210>  32
<211>  22
<212>  DNA
<213>  Murine

<400>  32
ggcaagcctt atgtcatctc gt                                                22

<210>  33
<211>  23
<212>  DNA
<213>  Murine

<400>  33
ggaatggttt tcccatggta ctt                                               23

<210>  34
<211>  28
<212>  DNA
<213>  Artificial seq

<220>
<223>  siRNA sequence

<400>  34
tgaaatgtct ccgctattac gctggctg                                          28

<210>  35
<211>  22
<212>  DNA
<213>  Homo sapien/Macaca fascicularis

<400>  35
tcattccttc cccaaagaga cc                                                22

<210>  36
<211>  22
<212>  DNA
<213>  Homo sapien

<400>  36
cacctccgtt ttggtggtag ag                                                22

<210>  37
<211>  28
<212>  DNA
<213>  Artificial seq

<220>
<223>  siRNA sequence

<400>  37
caagctgctc agtggaggca acacatta                                          28

<210>  38
<211>  22
<212>  DNA
<213>  Macaca fascicularis

<400>  38
tcattccttc cccaaagaaa cc                                                22


<210>  39
<211>  28
<212>  DNA
<213>  Artificial seq

<220>
<223>  siRNA sequence

<400>  39
tcaagctgtt aagtggcagc aacacgtt                                          28


<210>  40
<211>  23
<212>  DNA
<213>  Homo sapien

<400>  40
gcatgggtca gaaggattcc tat                                               23


<210>  41
<211>  23
<212>  DNA
<213>  Homo sapien

<400>  41
tgtagaaggt gtggtgccag att                                               23


<210>  42
<211>  22
<212>  DNA
<213>  Artificial seq

<220>
<223>  siRNA sequence

<400>  42
tcgagcacgg catcgtcacc aa                                                22


<210>  43
<211>  18
<212>  DNA
<213>  Macaca fascicularis

<400>  43
aaggccaacc gcgagaag                                                     18


<210>  44
<211>  20
<212>  DNA

<213>  Macaca fascicularis

<400>  44
agaggcgtac agggacagca                                                    20


<210>  45
<211>  28
<212>  DNA
<213>  Artificial seq

<220>
<223>  siRNA sequence

<400>  45
tgagaccttc aacaccccag ccatgtac                                           28


<210>  46
<211>  21
<212>  DNA
<213>  Murine

<400>  46
cctaaggcca accgtgaaaa g                                                  21


<210>  47
<211>  21
<212>  DNA
<213>  Murine

<400>  47
aggcatacag ggacagcaca g                                                  21


<210>  48
<211>  22
<212>  DNA
<213>  Homo sapien

<400>  48
caccgccaaa tttaactgca ga                                                 22


<210>  49
<211>  24
<212>  DNA
<213>  Homo sapien

<400>  49
aagggtttga taagttctag ctgt                                               24


<210>  50
<211>  31
<212>  DNA
<213>  Artificial seq

<220>
<223>  siRNA sequence

<400>  50

tgcacagtat ccttttgaag accataaccc a                                    31

<210>  51
<211>  19
<212>  RNA
<213>  Artificial seq

<220>
<223>  siRNA sequence

<400>  51
aauguuuucc ugcugacgg                                                  19

<210>  52
<211>  18
<212>  RNA
<213>  Artificial seq

<220>
<223>  siRNA sequence

<400>  52
aauguuuucc ugcugacg                                                   18

<210>  53
<211>  18
<212>  RNA
<213>  Artificial seq

<220>
<223>  siRNA sequence

<400>  53
ccgucagcag gaaaacau                                                   18

<210>  54
<211>  18
<212>  RNA
<213>  Artificial seq

<220>
<223>  siRNA sequence

<400>  54
gaaacucagu uuaagaag                                                   18

## Description

The present invention relates to products and compositions and their uses. In particular the invention relates to nucleic acid products that interfere with or inhibit the aldehyde dehydrogenase-2 (*ALDH2*) gene expression and therapeutic uses of such repression such as for the treatment of alcohol use disorder and associated pathologies or syndromes.

### Background

Double-stranded RNA (dsRNA) able to complementarily bind expressed mRNA has been shown to be able to block gene expression (Fire et a.l, 1998, Nature. 1998 Feb 19;391(6669):806-11 and Elbashir et al., 2001, Nature. 2001 May 24;411 (6836):494-8) by a mechanism that has been termed RNA interference (RNAi). Short dsRNAs direct gene-specific, post-transcriptional silencing in many organisms, including vertebrates, and have become a useful tool for studying gene function. RNAi is mediated by the RNA-induced silencing complex (RISC), a sequence-specific, multi-component nuclease that degrades messenger RNAs homologous to the silencing trigger loaded into the RISC complex. Interfering RNA (termed herein iRNA) such as siRNAs, antisense RNA, and micro RNA are oligonucleotides that prevent the formation of proteins by gene-silencing i.e. inhibiting gene translation of the protein through degradation of mRNA molecules. Gene-silencing agents are becoming increasingly important for therapeutic applications in medicine.

According to Watts and Corey in the Journal of Pathology (2012; Vol 226, p 365-379) there are algorithms that can be used to design nucleic acid silencing triggers, but all of these have severe limitations. It may take various experimental methods to identify potent iRNAs, as algorithms do not take into account factors such as tertiary structure of the target mRNA or the involvement of RNA binding proteins. Therefore, the discovery of a potent nucleic acid silencing trigger with minimal off-target effects is a complex process. For the pharmaceutical development of these highly charged molecules it is necessary that they can be synthesised economically, distributed to target tissues, enter cells and function within acceptable limits of toxicity.

Compulsive, excessive alcohol consumption beyond social drinking (aka. alcoholism, alcohol abuse, alcohol dependence) is a worldwide increasing health problem with severe personal and socioeconomic impact. Current medications that aid alcohol dishabituation are either not very effective or they have considerable undesired side effects. As a therapeutic approach, reduction of activity of a rate-limiting key enzyme of the hepatic alcohol metabolism pathway, the aldehyde dehydrogenase-2 (ALDH2), leads to accumulation of the toxic metabolite acetaldehyde. This accumulation results in unpleasant physiological effects in alcohol-consuming individuals. The association of unpleasant to severe feelings affects the patient's alcohol seeking behaviour, reduces alcohol consumption and, thereby, assists dishabituation. This therapeutic avenue has been exploited successfully for decades using small molecule ALDH-inhibiting compounds that act systemically (Kragh et al., 2008, Bull. Hist. Chem., Vol. 33, 2). The safety profile of those systemically acting inhibitors is worsened by the unwanted side effects even in individuals that do not consume alcohol or are produced by involuntary exposure to environmental aldehydes present in e.g. food, perfumes or other forms harmless to the organism in normal circumstances.

The current antidipsotropic medications that are aimed at keeping alcohol dependent patients away from drinking have low molecular and tissue specificity and show, therefore, a bad safety profile with considerable effects even in non-alcohol consuming patients. Treatment failure of those therapies is high due to high abandonment and cheating (not taking the medicine in order to be able to resume the consumption of alcohol). So there is a medical need to improve such therapeutic approach.

A first aspect of the invention relates to a nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of a RNA transcribed from the *ALDH2* gene, wherein said first strand comprises a nucleotide sequence selected from the following sequences: SEQ ID NOs:1, 3, 5, 7, 9, 11, 13, 15, 17, 19, 21, 23, 25 or 27.

The second strand may comprise a nucleotide sequence of SEQ ID NO:2, 4, 6, 8, 10, 12, 14, 16, 18, 20, 22, 24, 26 or 28.

The first strand may comprise the nucleotide sequence of SEQ ID NO:3, SEQ ID NO:11 or SEQ ID NO:17 and/or the second strand may comprise the nucleotide sequence of SEQ ID NO:4, SEQ ID NO:12 or SEQ ID NO:18.

The first strand and/or the second strand may each be from 17-35 nucleotides in length and at least one duplex region may be from 10-25 nucleotides in length. The duplex may comprise two separate strands or it may comprise a single strand which comprises the first strand and the second strand.

The nucleic acid may: a) be blunt ended at both ends; b) have an overhang at one end and a blunt end at the other; or c) have an overhang at both ends.

One or more nucleotides on the first and/or second strand may be modified, to form modified nucleotides. One or more of the odd numbered nucleotides of the first strand may be modified. One or more of the even numbered nucleotides of the first strand may be modified by at least a second modification, wherein the at least second modification is different from the modification on the one or more odd nucleotides. At least one of the one or more modified even numbered nucleotides may be adjacent to at least one of the one or more modified odd numbered nucleotides.

A plurality of odd numbered nucleotides in the first strand may be modified in the nucleic acid of the invention. A plurality of even numbered nucleotides in the first strand may be modified by a second modification. The first strand may comprise adjacent nucleotides that are modified by a common modification. The first strand may also comprise adjacent nucleotides that are modified by a second different modification.

One or more of the odd numbered nucleotides of the second strand may be modified by a modification that is different to the modification of the odd numbered nucleotides on the first strand and/or one or more of the even numbered nucleotides of the second strand may be modified by the same modification of the odd numbered nucleotides of the first strand. At least one of the one or more modified even numbered nucleotides of the second strand may be adjacent to the one or more modified odd numbered nucleotides. A plurality of odd numbered nucleotides of the second strand may be modified by a common modification and/or a plurality of even numbered nucleotides may be modified by the same modification that is present on the first strand odd numbered nucleotides. A plurality of odd numbered nucleotides on the second strand may be modified by a second modification, wherein the second modification is different from the modification of the first strand odd numbered nucleotides.

The second strand may comprise adjacent nucleotides that are modified by a common modification, which may be a second modification that is different from the modification of the odd numbered nucleotides of the first strand.

In the nucleic acid of the invention, each of the odd numbered nucleotides in the first strand and each of the even numbered nucleotides in the second strand may be modified with a common modification and, each of the even numbered nucleotides may be modified in the first strand with a second modification and each of the odd numbered nucleotides may be modified in the second strand with a second different modification.

The nucleic acid of the invention may have the modified nucleotides of the first strand shifted by at least one nucleotide relative to the unmodified or differently modified nucleotides of the second strand.

The modification and / or modifications may each and individually be selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide or a non-natural base comprising nucleotide. At least one modification may be 2'-O-methyl and/or at least one modification may be 2'-F.

The invention further provides, as a second aspect, a nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of an RNA transcribed from the *ALDH2* gene, wherein said first strand comprises a nucleotide sequence selected from the following sequences: SEQ ID NOs:1, 3, 5, 7, 9, 11, 13, 15, 17, 19, 21, 23, 25 or 27, wherein the nucleotides of first strand are modified by a first modification on the odd numbered nucleotides, and modified by a second modification on the even numbered nucleotides, and nucleotides of the second strand are modified by a third modification on the even numbered nucleotides and modified by a fourth modification the odd numbered nucleotides, wherein at least the first modification is different to the second modification and the third modification is different to the fourth modification. The second strand may comprise a nucleotide sequence of SEQ ID NO:2, 4, 6, 8, 10, 12, 14, 16, 18, 20, 22, 24, 26 or 28. The third modification and the first modification may be the same and/or the second modification and the fourth modification may be the same.

The first modification may be 2'OMe and the second modification may be 2'F.

In the nucleic acid of the second aspect, the first strand may comprise the nucleotide sequence of SEQ ID NO:3, SEQ ID NO:11 or SEQ ID NO:17 and the second strand may comprise the nucleotide sequence of SEQ ID NO:4, SEQ ID NO:12 or SEQ ID NO:18.

The sequence and modifications may be as shown in the Table below; which shows preferred sequences based on an extract of Table 1 as provided herein:

| **Sequence ID No** | **siRNA ID** | **sequence** | **modifications** |
|---|---|---|---|
| 3 | ALDH2-hcm-2 | 5'-AAUGUUUUCCUGCUGACGG-3' | 6254515173547182748 |
| 4 | | 5'-CCGUCAGCAGGAAAACAUU-3' | 3745364728462627251 |
| 11 | ALDH2-hcm-6 | 5'-UCUUCUUAAACUGAGUUUC-3' | 5351715262718281517 |
| 12 | | 5'-GAAACUCAGUUUAAGAAGA-3' | 4626353645152646282 |
| 17 | ALDH2-hcm-9 | 5'-AUGUAGCCGAGGAUCUUCU-3' | 6181647382846171535 |
| 18 | | 5'-AGAAGAUCCUCGGCUACAU-3' | 2826461735384716361 |

wherein the specific modifications are depicted by the following numbers
1=2'F-dU,
2=2'F-dA,
3=2'F-dC,
4=2'F-dG,
5=2'-OMe-rU;
6=2'-OMe-rA;
7=2'-OMe-rC;
8=2'-OMe-rG.

A nucleic acid of the invention may comprise a phosphorothioate linkage between the terminal one, two or three 3' nucleotides and/or one, two or three 5' nucleotides of the first and/or the second strand. It may comprise two phosphorothioate linkages between each of the three terminal 3' and between each of the three terminal 5' nucleotides on the first strand, and two phosphorothioate linkages between the three terminal nucleotides of the 3' end of the second strand.

Such a nucleic acid may be conjugated to a ligand.

The invention further provides, as a third aspect, a nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of a RNA transcribed from the *ALDH2* gene, wherein said first strand comprises a nucleotide sequence selected from the following sequences: SEQ ID NOs:1, 3, 5, 7, 9, 11, 13, 15, 17, 19, 21, 23, 25 or 27, and wherein the nucleic acid is conjugated to a ligand.

The ligand may comprise (i) one or more N-acetyl galactosamine (GalNac) moieties and derivatives thereof, and (ii) a linker, wherein the linker conjugates the GalNac moieties to a sequence as defined in any preceding aspects. The linker may be a bivalent or trivalent or tetravalent branched structure. The nucleotides may be modified as defined herein.

The ligand may comprise the formula I:

[S-X¹-P-X²]₃-A-X³- (I)

wherein:
S represents a saccharide, wherein the saccharide is N-acetyl galactosamine;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is alkylene or an alkylene ether of the formula (-CH₂)ₙ-O-CH₂- where n = 1- 6;
A is a branching unit;
X³ represents a bridging unit;
wherein a nucleic acid according to the present invention is conjugated to X³ via a phosphate or modified phosphate (preferably a thiophosphate).

The present invention therefore additionally provides a conjugated nucleic acid having one of the following structures 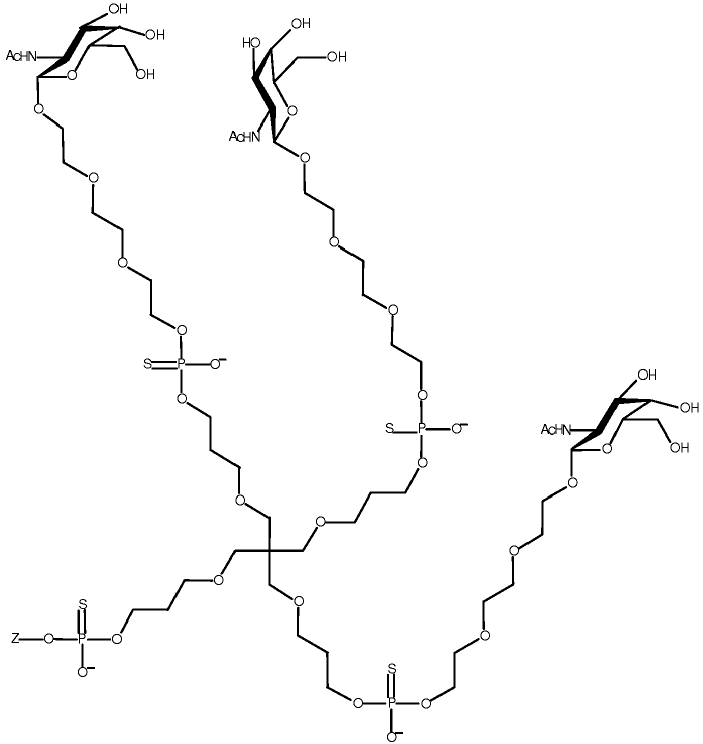 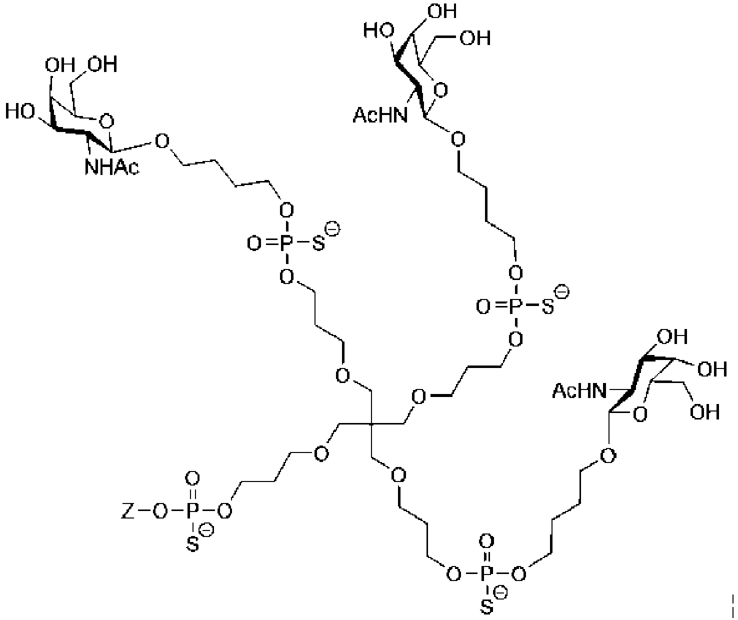 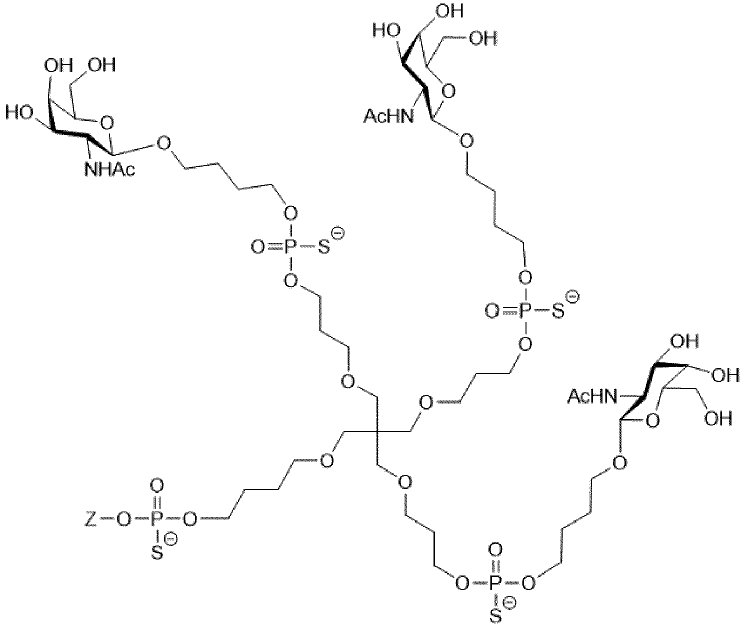 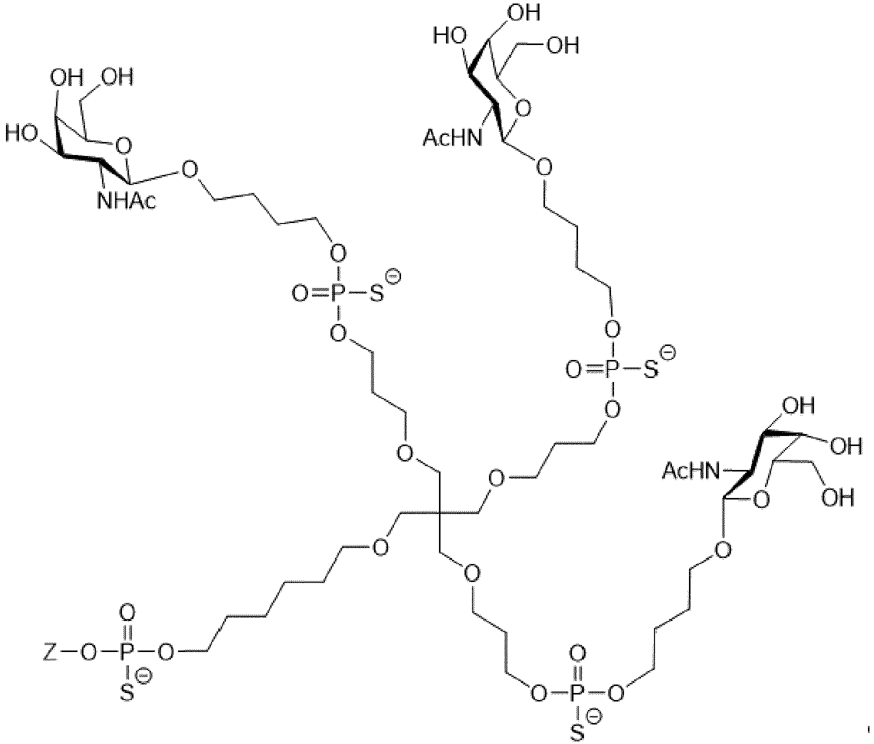 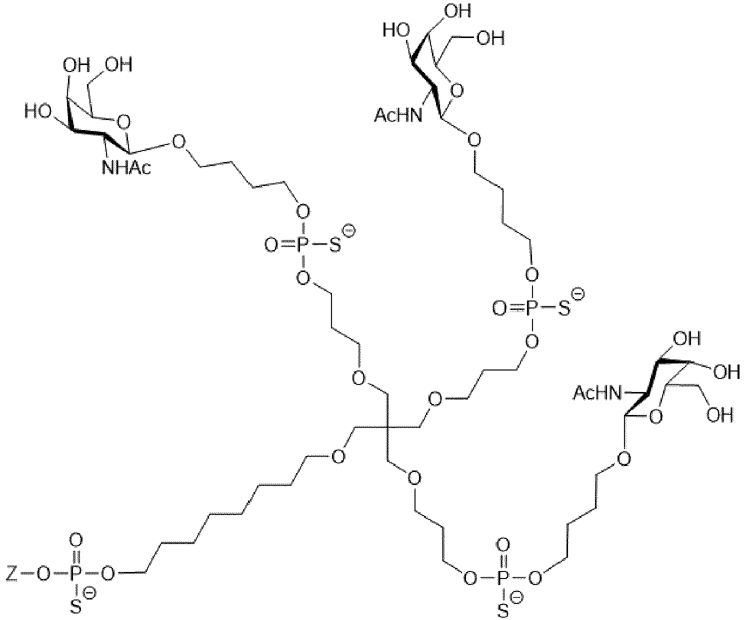 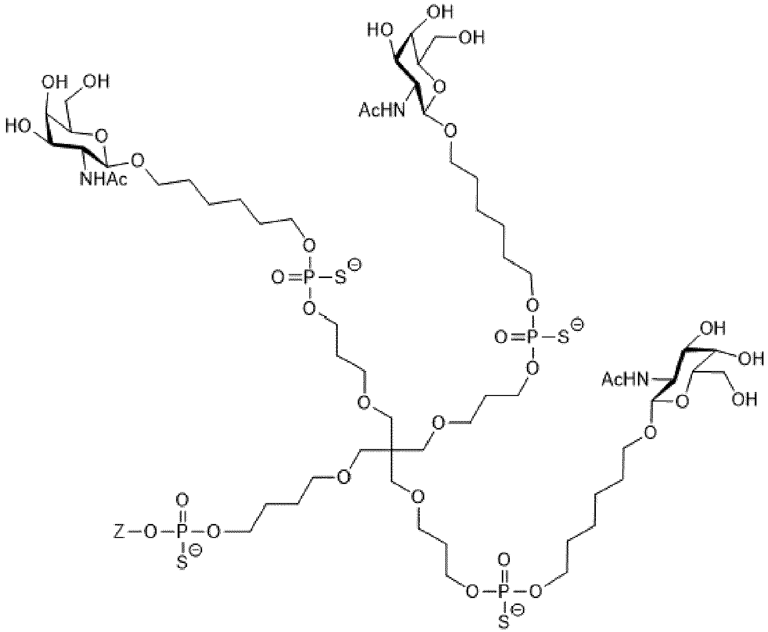 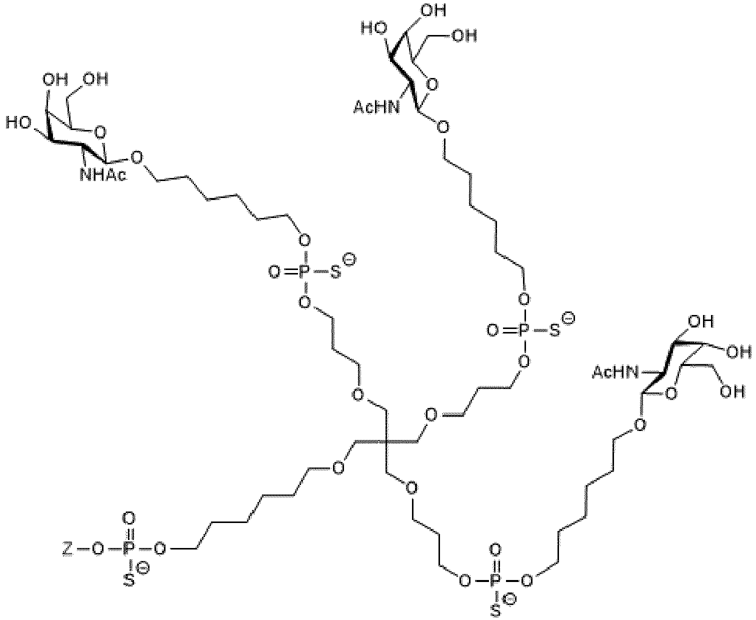 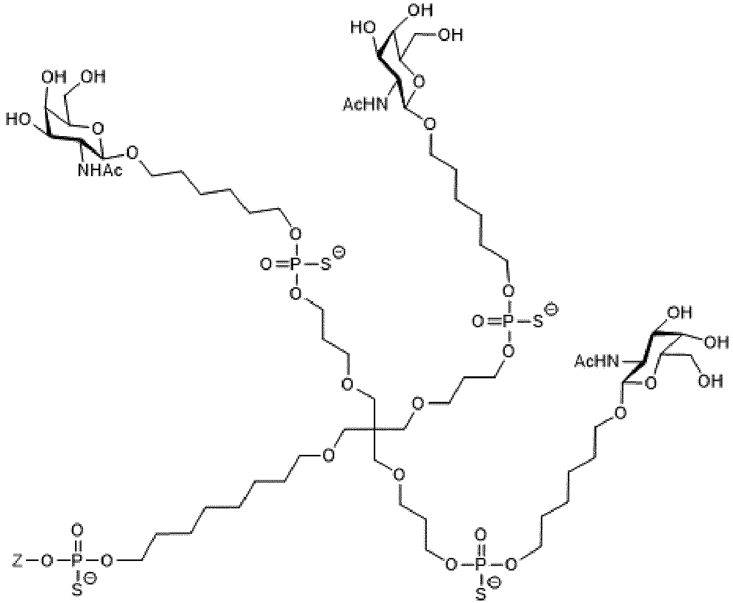 wherein Z represents a nucleic acid as defined herein before.

Alternatively, a nucleic acid according to the present invention may be conjugated to a ligand of the following structure

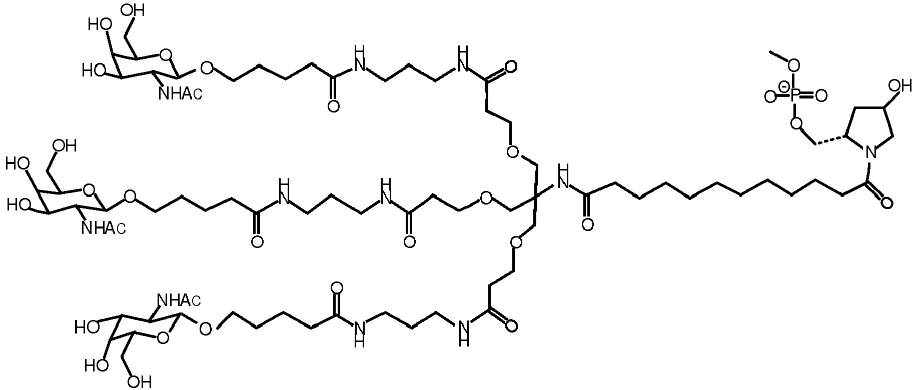

The invention also provides a composition comprising the nucleic acid or conjugated nucleic acid of any aspect of the invention, and a physiologically acceptable excipient.

Also provided is a nucleic acid or conjugated nucleic acid according to any aspect of the invention for use in the treatment of a disease, disorder or syndrome and/or in the manufacture of a medicament for treating a disease, disorder, or syndrome.

The invention provides a method of treating or preventing a disease, disorder or syndrome comprising administration of a composition comprising a nucleic acid or conjugated nucleic acid according to any aspect of the invention to an individual in need of treatment. The nucleic acid or conjugated nucleic acid may be administered to the subject subcutaneously, intravenously or using any other application routes such as oral, rectal or intraperitoneal.

After subcutaneous application, the invention may be delivered in a tissue specific manner to liver (hepatocytes) and target specifically *ALDH2,* in order to reduce unwanted side effects and achieve a lower therapeutic dose necessary to achieve the desired effect.

The disease, disorder or syndrome may be alcohol use disorder which includes acute alcohol sensitivity (hangover), alcoholic neuropathy (alcohol-related polyneuropathy) alcohol dependence, alcohol abuse (alcoholism) or fetal alcohol syndrome (alcohol-related neurodevelopmental disorder) or any other pathology associated to acute or prolonged excessive alcohol consumption.

A method of making the nucleic acid or conjugated nucleic acid according to the invention is also included.

### Detailed Description of Invention

The present invention relates to a nucleic acid which is double stranded and directed to an expressed RNA transcript of *ALDH2* and compositions thereof. These nucleic acids or conjugated nucleic acids can be used in the treatment and prevention of a variety of diseases, disorders and syndromes where reduced expression of *ALDH2* gene product is desirable.

A first aspect of the invention relates to a nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of a RNA transcribed from the *ALDH2* gene, wherein said first strand comprises a nucleotide sequence selected from the following sequences: SEQ ID NOs:1, 3, 5, 7, 9, 11, 13, 15, 17, 19, 21, 23, 25 or 27.

By nucleic acid it is meant a nucleic acid comprising two strands comprising nucleotides, that is able to interfere with gene expression. Inhibition may be complete or partial and results in down regulation of gene expression in a targeted manner. The nucleic acid comprises two separate polynucleotide strands; the first strand, which may also be a guide strand; and a second strand, which may also be a passenger strand. The first strand and the second strand may be part of the same polynucleotide molecule that is self-complementary which 'folds' back to form a double stranded molecule. The nucleic acid may be an siRNA molecule.

The nucleic acid may comprise ribonucleotides, modified ribonucleotides, deoxynucleotides, deoxyribonucleotides, or nucleotide analogues non-nucleotides that are able to mimic nucleotides such that they may 'pair' with the corresponding base on the target sequence or complementary strand. The nucleic acid may further comprise a double-stranded nucleic acid portion or duplex region formed by all or a portion of the first strand (also known in the art as a guide strand) and all or a portion of the second strand (also known in the art as a passenger strand). The duplex region is defined as beginning with the first base pair formed between the first strand and the second strand and ending with the last base pair formed between the first strand and the second strand, inclusive.

By duplex region it is meant the region in two complementary or substantially complementary oligonucleotides that form base pairs with one another, either by Watson-Crick base pairing or any other manner that allows for a duplex between oligonucleotide strands that are complementary or substantially complementary. For example, an oligonucleotide strand having 21 nucleotide units can base pair with another oligonucleotide of 21 nucleotide units, yet only 19 nucleotides on each strand are complementary or substantially complementary, such that the "duplex region" consists of 19 base pairs. The remaining base pairs may exist as 5' and 3' overhangs, or as single stranded regions. Further, within the duplex region, 100% complementarity is not required; substantial complementarity is allowable within a duplex region. Substantial complementarity refers to complementarity between the strands such that they are capable of annealing under biological conditions. Techniques to empirically determine if two strands are capable of annealing under biological conditions are well known in the art. Alternatively, two strands can be synthesised and added together under biological conditions to determine if they anneal to one another.

The portion of the first strand and second strand that form at least one duplex region may be fully complementary and is at least partially complementary to each other. Depending on the length of a nucleic acid, a perfect match in terms of base complementarity between the first strand and the second strand is not necessarily required. However, the first and second strands must be able to hybridise under physiological conditions.

The complementarity between the first strand and second strand in the at least one duplex region may be perfect in that there are no nucleotide mismatches or additional/deleted nucleotides in either strand. Alternatively, the complementarity may not be perfect. The complementarity may be from about 70% to about 100%. More specifically, the complementarity may be at least 70%, 75%, 80%, 85%, 90% or 95% and intermediate values.

The first strand and the second strand may each comprise a region of complementarity which comprises at least 15 contiguous nucleotides differing by no more than 3 nucleotides from any one of the sequences listed in Table 1.

The nucleic acid may comprise a second sequence comprising a nucleotide sequence of SEQ ID NO:2, 4, 6, 8, 10, 12, 14, 16, 18, 20, 22, 24, 26 or 28.

Use of a nucleic acid according to the present invention involves the formation of a duplex region between all or a portion of the first strand and a portion of a target nucleic acid. The portion of the target nucleic acid that forms a duplex region with the first strand, defined as beginning with the first base pair formed between the first strand and the target sequence and ending with the last base pair formed between the first strand and the target sequence, inclusive, is the target nucleic acid sequence or simply, target sequence. The duplex region formed between the first strand and the second strand need not be the same as the duplex region formed between the first strand and the target sequence. That is, the second strand may have a sequence different from the target sequence; however, the first strand must be able to form a duplex structure with both the second strand and the target sequence.

The complementarity between the first strand and the target sequence may be perfect (no nucleotide mismatches or additional/deleted nucleotides in either nucleic acid).

The complementarity between the first strand and the target sequence may not be perfect. The complementarity may be from about 70% to about 100%. More specifically, the complementarity may be at least 70%, 80%, 85%, 90% or 95% and intermediate values.

The identity between the first strand and the complementary sequence of the target sequence may range from about 75% to about 100%. More specifically, the complementarity may be at least 75%, 80%, 85%, 90% or 95% and intermediate values, provided a nucleic acid is capable of reducing or inhibiting the expression of *ALDH2.*

A nucleic acid having less than 100% complementarity between the first strand and the target sequence may be able to reduce the expression of *ALDH2* to the same level as a nucleic acid having perfect complementarity between the first strand and target sequence. Alternatively, it may be able to reduce expression of *ALDH2* to a level that is 15% - 100% of the level of reduction achieved by the nucleic acid with perfect complementarity.

The nucleic acid may comprise a first strand and a second strand that are each from 19-25 nucleotides in length. The first strand and the second strand may be of different lengths.

The nucleic acid may be 15-25 nucleotide pairs in length. The nucleic acid may be 17-23 nucleotide pairs in length. The nucleic acid may be 17-25 nucleotide pairs in length. The nucleic acid may be 23-24 nucleotide pairs in length. The nucleic acid may be 19-21 nucleotide pairs in length. The nucleic acid may be 21-23 nucleotide pairs in length.

The nucleic acid may comprise a duplex region that consists of 19-25 nucleotide base pairs. The duplex region may consist of 17, 18, 19, 20, 21, 22, 23, 24 or 25 base pairs, which may be contiguous.

The nucleic acid may comprise a first strand sequence of SEQ ID NO:3, SEQ ID NO:11 or SEQ ID NO:17. The nucleic acid may comprise a second strand sequence of SEQ ID NO:4, SEQ ID NO:12 or SEQ ID NO:18.

In a further aspect the nucleic acid or conjugated nucleic acid as described may reduce the expression of *ALDH2* by at least 15% compared to the expression observed in the absence of the nucleic acid or conjugated nucleic acid. All preferred features of any of the previous aspects also apply to this aspect. In particular, the expression of *ALDH2* may be reduced to at least the following given % or less than 90%, 80%, 70%, 60%, 50%, 40%, 30%, 20%, 15% or less, and intermediate values, than that observed in the absence of the nucleic acid or conjugated nucleic acid or in the presence of a non-silencing control.

The nucleic acid may be blunt ended at both ends; have an overhang at one end and a blunt end at the other end; or have an overhang at both ends.

An "overhang" as used herein has its normal and customary meaning in the art, i.e. a single stranded portion of a nucleic acid that extends beyond the terminal nucleotide of a complementary strand in a double strand nucleic acid. The term "blunt end" includes double stranded nucleic acid whereby both strands terminate at the same position, regardless of whether the terminal nucleotide(s) are base-paired. The terminal nucleotide of a first strand and a second strand at a blunt end may be base paired. The terminal nucleotide of a first strand and a second strand at a blunt end may not be paired. The terminal two nucleotides of a first strand and a second strand at a blunt end may be base-paired. The terminal two nucleotides of a first strand and a second strand at a blunt end may not be paired.

The nucleic acid may have an overhang at one end and a blunt end at the other. The nucleic acid may have an overhang at both ends. The nucleic acid may be blunt ended at both ends. The nucleic acid may be blunt ended at the end with the 5'-end of the first strand and the 3'-end of the second strand or at the 3'-end of the first strand and the 5'-end of the second strand.

The nucleic acid may comprise an overhang at a 3'- or 5'-end. The nucleic acid may have a 3'-overhang on the first strand. The nucleic acid may have a 3'-overhang on the second strand. The nucleic acid may have a 5'-overhang on the first strand. The nucleic acid may have a 5'-overhang on the second strand. The nucleic acid may have an overhang at both the 5'-end and 3'-end of the first strand. The nucleic acid may have an overhang at both the 5'-end and 3'-end of the second strand. The nucleic acid may have a 5' overhang on the first strand and a 3' overhang on the second strand. The nucleic acid may have a 3' overhang on the first strand and a 5' overhang on the second strand. The nucleic acid may have a 3' overhang on the first strand and a 3' overhang on the second strand. The nucleic acid may have a 5' overhang on the first strand and a 5' overhang on the second strand.

An overhang at the 3'-end or 5' end of the second strand or the first strand may be selected from consisting of 1, 2, 3, 4 and 5 nucleotides in length. Optionally, an overhang may consist of 1 or 2 nucleotides, which may or may not be modified.

Unmodified polynucleotides, particularly ribonucleotides, may be prone to degradation by cellular nucleases, and, as such, modifications/ modified nucleotides may be included in the nucleic acid of the invention.

One or more nucleotides on the second and/or first strand of the nucleic acid of the invention may be modified.

Modifications of the nucleic acid of the present invention generally provide a powerful tool in overcoming potential limitations including, but not limited to, *in vitro* and *in vivo* stability and bioavailability inherent to native RNA molecules. The nucleic acid according to the invention may be modified by chemical modifications. Modified nucleic acid can also minimise the possibility of inducing interferon activity in humans. Modification can further enhance the functional delivery of a nucleic acid to a target cell. The modified nucleic acid of the present invention may comprise one or more chemically modified ribonucleotides of either or both of the first strand or the second strand. A ribonucleotide may comprise a chemical modification of the base, sugar or phosphate moieties. The ribonucleic acid may be modified by substitution or insertion with analogues of nucleic acids or bases.

One or more nucleotides of a nucleic acid of the present invention may be modified. The nucleic acid may comprise at least one modified nucleotide. The modified nucleotide may be on the first strand. The modified nucleotide may be in the second strand. The modified nucleotide may be in the duplex region. The modified nucleotide may be outside the duplex region, i.e., in a single stranded region. The modified nucleotide may be on the first strand and may be outside the duplex region. The modified nucleotide may be on the second strand and may be outside the duplex region. The 3'-terminal nucleotide of the first strand may be a modified nucleotide. The 3'-terminal nucleotide of the second strand may be a modified nucleotide. The 5'-terminal nucleotide of the first strand may be a modified nucleotide. The 5'-terminal nucleotide of the second strand may be a modified nucleotide.

A nucleic acid of the invention may have 1 modified nucleotide or a nucleic acid of the invention may have about 2-4 modified nucleotides, or a nucleic acid may have about 4-6 modified nucleotides, about 6-8 modified nucleotides, about 8-10 modified nucleotides, about 10-12 modified nucleotides, about 12-14 modified nucleotides, about 14-16 modified nucleotides about 16-18 modified nucleotides, about 18-20 modified nucleotides, about 20-22 modified nucleotides, about 22-24 modified nucleotides, 24-26 modified nucleotides or about 26-28 modified nucleotides. In each case the nucleic acid comprising said modified nucleotides retains at least 50% of its activity as compared to the same nucleic acid but without said modified nucleotides or vice versa. The nucleic acid may retain 55%, 60%, 65%, 70%, 75%, 80%, 85%, 90%, 95% or 100% and intermediate values of its activity as compared to the same nucleic acid but without said modified nucleotides, or may have more than 100% of the activity of the same nucleic acid without said modified nucleotides.

The modified nucleotide may be a purine or a pyrimidine. At least half of the purines may be modified. At least half of the pyrimidines may be modified. All of the purines may be modified. All of the pyrimidines may be modified. The modified nucleotides may be selected from the group consisting of a 3'-terminal deoxy-thymine (dT) nucleotide, a 2'-O-methyl modified nucleotide, a 2' modified nucleotide, a 2'-deoxy-modified nucleotide, a locked nucleotide, an abasic nucleotide, a 2'-amino-modified nucleotide, a 2'-alkyl-modified nucleotide, a morpholino nucleotide, a phosphoramidate, a non-natural base comprising nucleotide, a nucleotide comprising a 5'-phosphorothioate group, a nucleotide comprising a 5' phosphate or 5' phosphate mimic and a terminal nucleotide linked to a cholesteryl derivative or a dodecanoic acid bisdecylamide group.

The nucleic acid may comprise a nucleotide comprising a modified nucleotide, wherein the base is selected from 2-aminoadenosine, 2,6-diaminopurine,inosine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidine (e.g., 5-methylcytidine), 5-alkyluridine (e.g., ribothymidine), 5-halouridine (e.g., 5-bromouridine), 6-azapyrimidine, 6-alkylpyrimidine (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueosine, 1-methyladenosine, 1-methylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid and 2-thiocytidine.

Nucleic acids discussed herein include unmodified RNA as well as RNA which has been modified, e.g., to improve efficacy, and polymers of nucleoside surrogates. Unmodified RNA refers to a molecule in which the components of the nucleic acid, namely sugars, bases, and phosphate moieties, are the same or essentially the same as that which occur in nature, for example as occur naturally in the human body. Modified nucleotide as used herein refers to a nucleotide in which one or more of the components of the nucleic acid, namely sugars, bases, and phosphate moieties, are different from those which occur in nature. While they are referred to as modified nucleotides they will of course, because of the modification, include molecules which are not nucleotides, for example a polynucleotide molecule in which the ribophosphate backbone is replaced with a non-ribophosphate construct that allows hybridisation between strands i.e. the modified nucleotides mimic the ribophosphate backbone.

Many of the modifications described below that occur within a nucleic acid will be repeated within a polynucleotide molecule, such as a modification of a base, or a phosphate moiety, or a non-linking O of a phosphate moiety. In some cases the modification will occur at all of the possible positions/nucleotides in the polynucleotide but in many cases it will not. A modification may only occur at a 3' or 5' terminal position, may only occur in a terminal regions, such as at a position on a terminal nucleotide or in the last 2, 3, 4, 5, or 10 nucleotides of a strand. A modification may occur in a double strand region, a single strand region, or in both. A modification may occur only in the double strand region of a nucleic acid of the invention or may only occur in a single strand region of a nucleic acid of the invention. A phosphorothioate modification at a non-linking O position may only occur at one or both termini, may only occur in a terminal region, e.g., at a position on a terminal nucleotide or in the last 2, 3, 4 or 5 nucleotides of a strand, or may occur in duplex and/or in single strand regions, particularly at termini. The 5' end or 3' ends may be phosphorylated.

Stability of a nucleic acid of the invention may be increased by including particular bases in overhangs, or to include modified nucleotides, in single strand overhangs, e.g., in a 5' or 3' overhang, or in both. Purine nucleotides may be included in overhangs. All or some of the bases in a 3' or 5' overhang may be modified. Modifications can include the use of modifications at the 2' OH group of the ribose sugar, the use of deoxyribonucleotides, instead of ribonucleotides, and modifications in the phosphate group, such as phosphorothioate modifications. Overhangs need not be homologous with the target sequence.

Nucleases can hydrolyze nucleic acid phosphodiester bonds. However, chemical modifications to nucleic acids can confer improved properties, and, can render oligoribonucleotides more stable to nucleases.

Modified nucleic acids, as used herein, can include one or more of:
(i) alteration, e.g., replacement, of one or both of the non-linking phosphate oxygens and/or of one or more of the linking phosphate oxygens (referred to as linking even if at the 5' and 3' terminus of the nucleic acid of the invention);
(ii) alteration, e.g., replacement, of a constituent of the ribose sugar, e.g., of the 2' hydroxyl on the ribose sugar;
(iii) replacement of the phosphate moiety with "dephospho" linkers;
(iv) modification or replacement of a naturally occurring base;
(v) replacement or modification of the ribose-phosphate backbone;
(vi) modification of the 3' end or 5' end of the RNA, e.g., removal, modification or replacement of a terminal phosphate group or conjugation of a moiety, e.g., a fluorescently labelled moiety, to either the 3' or 5' end of RNA.

The terms replacement, modification, alteration, indicate a difference from a naturally occurring molecule.

Specific modifications are discussed in more detail below.

Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulphur. One, each or both non-linking oxygens in the phosphate group can be independently any one of S, Se, B, C, H, N, or OR (R is alkyl or aryl).

The phosphate linker can also be modified by replacement of a linking oxygen with nitrogen (bridged phosphoroamidates), sulfur (bridged phosphorothioates) and carbon (bridged methylenephosphonates). The replacement can occur at a terminal oxygen. Replacement of the non-linking oxygens with nitrogen is possible.

A modified nucleotide can include modification of the sugar groups. The 2' hydroxyl group (OH) can be modified or replaced with a number of different "oxy" or "deoxy" substituents.

Examples of "oxy"-2' hydroxyl group modifications include alkoxy or aryloxy (OR, e.g., R=H, alkyl, cycloalkyl, aryl, aralkyl, heteroaryl or sugar); polyethyleneglycols (PEG), O(CH2CH2O)nCH2CH2OR; "locked" nucleic acids (LNA) in which the 2' hydroxyl is connected, e.g., by a methylene bridge, to the 4' carbon of the same ribose sugar; O-AMINE (AMINE=NH2; alkylamino, dialkylamino, heterocyclyl, arylamino, diaryl amino, heteroaryl amino, or diheteroaryl amino, ethylene diamine, polyamino) and aminoalkoxy, O(CH2)nAMINE, (e.g., AMINE=NH2; alkylamino, dialkylamino, heterocyclyl, arylamino, diaryl amino, heteroaryl amino, or diheteroaryl amino, ethylene diamine, polyamino).

"Deoxy" modifications include hydrogen halo; amino (e.g., NH2; alkylamino, dialkylamino, heterocyclyl, arylamino, diaryl amino, heteroaryl amino, diheteroaryl amino, or amino acid); NH(CH2CH2NH)nCH2CH2-AMINE (AMINE=NH2; alkylamino, dialkylamino, heterocyclyl, arylamino, diaryl amino, heteroaryl amino, or diheteroaryl amino), -NHC(O)R (R=alkyl, cycloalkyl, aryl, aralkyl, heteroaryl or sugar), cyano; mercapto; alkylthio-alkyl; thioalkoxy; and alkyl, cycloalkyl, aryl, alkenyl and alkynyl, which may be optionally substituted with e.g., an amino functionality. Other substitutents of certain embodiments include 2'-methoxyethyl, 2'-OCH3, 2'-O-allyl, 2'-C-allyl, and 2'-fluoro. The sugar group can also contain one or more carbons that possess the opposite stereochemical configuration than that of the corresponding carbon in ribose. Thus, a modified nucleotides may contain a sugar such as arabinose.

Modified nucleotides can also include "abasic" sugars, which lack a nucleobase at C-I'. These abasic sugars can further contain modifications at one or more of the constituent sugar atoms.
The 2' modifications may be used in combination with one or more phosphate linker modifications (e.g., phosphorothioate).

The phosphate group can be replaced by non-phosphorus containing connectors.

Examples of moieties which can replace the phosphate group include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino. In certain embodiments, replacements may include the methylenecarbonylamino and methylenemethylimino groups.

The phosphate linker and ribose sugar may be replaced by nuclease resistant nucleotides.

Examples include the morpholino, cyclobutyl, pyrrolidine and peptide nucleic acid (PNA) nucleoside surrogates. In certain embodiments, PNA surrogates may be used.

The 3' and 5' ends of an oligonucleotide can be modified. Such modifications can be at the 3' end or the 5' end or both ends of the molecule. They can include modification or replacement of an entire terminal phosphate or of one or more of the atoms of the phosphate group. For example, the 3' and 5' ends of an oligonucleotide can be conjugated to other functional molecular entities such as labelling moieties, e.g., fluorophores (e.g., pyrene, TAMRA, fluorescein, Cy3 or Cy5 dyes) or protecting groups (based e.g., on sulfur, silicon, boron or ester). The functional molecular entities can be attached to the sugar through a phosphate group and/or a linker. The terminal atom of the linker can connect to or replace the linking atom of the phosphate group or the C-3' or C-5' O, N, S or C group of the sugar. Alternatively, the linker can connect to or replace the terminal atom of a nucleotide surrogate (e.g., PNAs). These spacers or linkers can include e.g., -(CH₂)ₙ-, -(CH₂)ₙN-, -(CH₂)ₙO-, -(CH₂)ₙS-, O(CH₂CH₂O)ₙCH₂CH₂OH (e.g., n=3 or 6), abasic sugars, amide, carboxy, amine, oxyamine, oxyimine, thioether, disulfide, thiourea, sulfonamide, or morpholino, or biotin and fluorescein reagents. The 3' end can be an - OH group.

Other examples of terminal modifications include dyes, intercalating agents (e.g., acridines), cross-linkers (e.g., psoralene, mitomycin C), porphyrins (TPPC4, texaphyrin, Sapphyrin), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles).

Terminal modifications can be added for a number of reasons, including to modulate activity or to modulate resistance to degradation. Terminal modifications useful for modulating activity include modification of the 5' end with phosphate or phosphate analogues. Nucleic acids of the invention, on the first or second strand, may be 5' phosphorylated or include a phosphoryl analogue at the 5' prime terminus. 5'-phosphate modifications include those which are compatible with RISC mediated gene silencing. Suitable modifications include: 5'-monophosphate ((HO)₂(O)P-O-5'); 5'-diphosphate ((HO)₂(O)P-O-P(HO)(O)-O-5'); 5'-triphosphate ((HO)₂(O)P-O-(HO)(O)P-OP(HO)(O)-O-5'); 5'-guanosine cap (7-methylated or non-methylated) (7m-G-O-5'-(HO)(O)P-O-(HO)(O)P-O-P(HO)(O)-O-5'); 5'-adenosine cap (Appp), and any modified or unmodified nucleotide cap structure (N-O-5'-(HO)(O)P-O-(HO)(O)P-OP(HO)(O)-O-5'); 5'-monothiophosphate (phosphorothioate; (HO)₂(S)P-O-5'); 5'-monodithiophosphate (phosphorodithioate; (HO)(HS)(S)P-O-5'), 5'-phosphorothiolate ((HO)2(O)P-S-5'); any additional combination of oxygen/sulfur replaced monophosphate, diphosphate and triphosphates (e.g., 5'-alpha-thiotriphosphate, 5'-gamma-thiotriphosphate, etc.), 5'-phosphoramidates ((HO)2(O)P-NH-5', (HO)(NH2)(O)P-O-5'), 5'-alkylphosphonates (R=alkyl=methyl, ethyl, isopropyl, propyl, etc., e.g., RP(OH)(O)-O-5'-, (OH)₂(O)P-5'-CH₂-), 5'vinylphosphonate, 5'-alkyletherphosphonates (R=alkylether=methoxymethyl (MeOCH2-), ethoxymethyl, etc., e.g., RP(OH)(O)-O-5'-).

The nucleic acid of the present invention may include one or more phosphorothioate modifications on one or more of the terminal ends of the first and/or the second strand. Optionally, each or either end of the first strand may comprise one or two or three phosphorothioate modified nucleotides. Optionally, each or either end of the second strand may comprise one or two or three phosphorothioate modified nucleotides. Optionally, both ends of the first strand and the 5' end of the second strand may comprise two phosphorothioate modified nucleotides. By phosphorothioate modified nucleotide it is meant that the linkage between the nucleotide and the adjacent nucleotide comprises a phosphorothioate group instead of a standard phosphate group.

Terminal modifications can also be useful for monitoring distribution, and in such cases the groups to be added may include fluorophores, e.g., fluorscein or an Alexa dye. Terminal modifications can also be useful for enhancing uptake, useful modifications for this include cholesterol. Terminal modifications can also be useful for cross-linking an RNA agent to another moiety.

Adenine, guanine, cytosine and uracil are the most common bases found in RNA. These bases can be modified or replaced to provide RNA's having improved properties. E.g., nuclease resistant oligoribonucleotides can be prepared with these bases or with synthetic and natural nucleobases (e.g., inosine, thymine, xanthine, hypoxanthine, nubularine, isoguanisine, or tubercidine) and any one of the above modifications. Alternatively, substituted or modified analogues of any of the above bases and "universal bases" can be employed. Examples include 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, N6,N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3-carboxypropyl)uracil, 3-methylcytosine, 5-methylcytosine, N<4>-acetyl cytosine, 2-thiocytosine, N6-methyladenine, N6-isopentyladenine, 2-methylthio-N6-isopentenyladenine, N-methylguanines, or O-alkylated bases.

As used herein, the terms "non-pairing nucleotide analogue" means a nucleotide analogue which includes a non-base pairing moiety including but not limited to: 6 des amino adenosine (Nebularine), 4-Me-indole, 3-nitropyrrole, 5-nitroindole, Ds, Pa, N3-Me ribo U, N3-Me riboT, N3-Me dC, N3-Me-dT, N1-Me-dG, N1-Me-dA, N3-ethyl-dC, N3-Me dC. In some embodiments the non-base pairing nucleotide analogue is a ribonucleotide. In other embodiments it is a deoxyribonucleotide.

As used herein, the term, "terminal functional group" includes without limitation a halogen, alcohol, amine, carboxylic, ester, amide, aldehyde, ketone, ether groups.

Certain moieties may be linked to the 5' terminus of the first strand or the second strand. These include abasic ribose moiety, abasic deoxyribose moiety, modifications abasic ribose and abasic deoxyribose moieties including 2'O alkyl modifications; inverted abasic ribose and abasic deoxyribose moieties and modifications thereof, C6-imino-Pi; a mirror nucleotide including L-DNA and L-RNA; 5'OMe nucleotide; and nucleotide analogs including 4',5'-methylene nucleotide; 1-(β-D-erythrofuranosyl)nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 12-aminododecyl phosphate; hydroxypropyl phosphate; 1,5-anhydrohexitol nucleotide; alpha-nucleotide; threo-pentofuranosyl nucleotide; acyclic 3',4'-seco nucleotide; 3,4-dihydroxybutyl nucleotide; 3,5-dihydroxypentyl nucleotide, 5'-5'-inverted abasic moiety; 1,4-butanediol phosphate; 5'-amino; and bridging or non-bridging methylphosphonate and 5'-mercapto moieties. The nucleic acids of the invention may be included one or more inverted nucleotides, for example inverted thymidine or inverted adenine (for example see Takei, et al., 2002. JBC 277 (26):23800-06).

As used herein, the term "inhibit", "down-regulate", or "reduce" with respect to gene expression means the expression of the gene, or level of RNA molecules or equivalent RNA molecules encoding one or more proteins or protein subunits (e.g., mRNA), or activity of one or more proteins or protein subunits, is reduced below that observed in the absence of the nucleic acid or conjugated nucleic acid of the invention or in reference to an siRNA molecule with no known homology to human transcripts (herein termed non-silencing control). Such control may be conjugated and modified in an analogous manner to the molecule of the invention and delivered into the target cell by the same route; for example the expression may be reduced to 90%, 80%, 70%, 60%, 50%, 40%, 30%, 20%, 15%, or to intermediate values, or less than that observed in the absence of the nucleic acid or conjugated nucleic acid or in the presence of a non-silencing control.

The nucleic acid of the present invention may comprise an abasic nucleotide. The term "abasic" as used herein, refers to moieties lacking a base or having other chemical groups in place of a base at the 1' position, for example a 3',3'-linked or 5',5'-linked deoxyabasic ribose derivative.

The nucleic acid may comprise one or more nucleotides on the second and/or first strands that are modified. Alternating nucleotides may be modified, to form modified nucleotides.

Alternating as described herein means to occur one after another in a regular way. In other words, alternating means to occur in turn repeatedly. For example if one nucleotide is modified, the next contiguous nucleotide is not modified and the following contiguous nucleotide is modified and so on. One nucleotide may be modified with a first modification, the next contiguous nucleotide may be modified with a second modification and the following contiguous nucleotide is modified with the first modification and so on, where the first and second modifications are different.

One or more of the odd numbered nucleotides of the first strand of the nucleic acid of the invention may be modified wherein the first strand is numbered 5' to 3'. The term "odd numbered" as described herein means a number not divisible by two. Examples of odd numbers are 1, 3, 5, 7, 9, 11 and so on. One or more of the even numbered nucleotides of the first strand of the nucleic acid of the invention may be modified, wherein the first strand is numbered 5' to 3'. The term "even numbered" as described herein means a number which is evenly divisible by two. Examples of even numbers are 2, 4, 6, 8, 10, 12, 14 and so on. One or more of the odd numbered nucleotides of the second strand of the nucleic acid of the invention may be modified wherein the second strand is numbered 3' to 5'. One or more of the even numbered nucleotides of the second strand of the nucleic acid of the invention may be modified, wherein the second strand is numbered 3' to 5'.

One or more nucleotides on the first and/or second strand may be modified, to form modified nucleotides. One or more of the odd numbered nucleotides of the first strand may be modified. One or more of the even numbered nucleotides of the first strand may be modified by at least a second modification, wherein the at least second modification is different from the modification on the one or more odd nucleotides. At least one of the one or more modified even numbered nucleotides may be adjacent to at least one of the one or more modified odd numbered nucleotides.

A plurality of odd numbered nucleotides in the first strand may be modified in the nucleic acid of the invention. A plurality of even numbered nucleotides in the first strand may be modified by a second modification. The first strand may comprise adjacent nucleotides that are modified by a common modification. The first strand may also comprise adjacent nucleotides that are modified by a second different modification.

One or more of the odd numbered nucleotides of the second strand may be modified by a modification that is different to the modification of the odd numbered nucleotides on the first strand and/or one or more of the even numbered nucleotides of the second strand may be modified by the same modification of the odd numbered nucleotides of the first strand. At least one of the one or more modified even numbered nucleotides of the second strand may be adjacent to the one or more modified odd numbered nucleotides. A plurality of odd numbered nucleotides of the second strand may be modified by a common modification and/or a plurality of even numbered nucleotides may be modified by the same modification that is present on the first stand odd numbered nucleotides. A plurality of odd numbered nucleotides on the second strand may be modified by a second modification, wherein the second modification is different from the modification of the first strand odd numbered nucleotides.

The second strand may comprise adjacent nucleotides that are modified by a common modification, which may be a second modification that is different from the modification of the odd numbered nucleotides of the first strand.

In the nucleic acid of the invention, each of the odd numbered nucleotides in the first strand and each of the even numbered nucleotides in the second strand may be modified with a common modification and, each of the even numbered nucleotides may be modified in the first strand with a second modification and each of the odd numbered nucleotides may be modified in the second strand with the second modification.

The nucleic acid of the invention may have the modified nucleotides of the first strand shifted by at least one nucleotide relative to the unmodified or differently modified nucleotides of the second strand.

One or more or each of the odd numbered nucleotides may be modified in the first strand and one or more or each of the even numbered nucleotides may be modified in the second strand. One or more or each of the alternating nucleotides on either or both strands may be modified by a second modification. One or more or each of the even numbered nucleotides may be modified in the first strand and one or more or each of the even numbered nucleotides may be modified in the second strand. One or more or each of the alternating nucleotides on either or both strands may be modified by a second modification. One or more or each of the odd numbered nucleotides may be modified in the first strand and one or more of the odd numbered nucleotides may be modified in the second strand by a common modification. One or more or each of the alternating nucleotides on either or both strands may be modified by a second modification. One or more or each of the even numbered nucleotides may be modified in the first strand and one or more or each of the odd numbered nucleotides may be modified in the second strand by a common modification. One or more or each of the alternating nucleotides on either or both strands may be modified by a second modification.

The nucleic acid of the invention may comprise single or double stranded constructs that comprise at least two regions of alternating modifications in one or both of the strands. These alternating regions can comprise up to about 12 nucleotides but preferably comprise from about 3 to about 10 nucleotides. The regions of alternating nucleotides may be located at the termini of one or both strands of the nucleic acid of the invention. The nucleic acid may comprise from 4 to about 10 nucleotides of alternating nucleotides at each termini (3' and 5') and these regions may be separated by from about 5 to about 12 contiguous unmodified or differently or commonly modified nucleotides.

The odd numbered nucleotides of the first strand may be modified and the even numbered nucleotides may be modified with a second modification. The second strand may comprise adjacent nucleotides that are modified with a common modification, which may be the same as the modification of the odd numbered nucleotides of the first strand. One or more nucleotides of second strand may also be modified with the second modification. One or more nucleotides with the second modification may be adjacent to each other and to nucleotides having a modification that is the same as the modification of the odd numbered nucleotides of the first strand. The first strand may also comprise phosphorothioate linkages between the two nucleotides at the 3' end and at the 5' end. The second strand may comprise a phosphorothioate linkage between the two nucleotides at 5' end. The second strand may also be conjugated to a ligand at the 5' end.

The nucleic acid of the invention may comprise a first strand comprising adjacent nucleotides that are modified with a common modification. One or more of such nucleotides may be adjacent to one or more nucleotides which may be modified with a second modification. One or more nucleotides with the second modification may be adjacent. The second strand may comprise adjacent nucleotides that are modified with a common modification, which may be the same as one of the modifications of one or more nucleotides of the first strand. One or more nucleotides of second strand may also be modified with the second modification. One or more nucleotides with the second modification may be adjacent. The first strand may also comprise phosphorothioate linkages between the two nucleotides at the 5' end and at the 3' end. The second strand may comprise a phosphorothioate linkage between the two nucleotides at 3' end. The second strand may also be conjugated to a ligand at the 5' end.

The nucleotides numbered from 5' to 3' on the first strand and 3' and 5' on the second strand, 1, 3, 5, 7, 9, 11, 13, 15, 17, 19, 21, 23 and 25 may be modified by a modification on the first strand. The nucleotides numbered 2, 4, 6, 8, 10, 12, 14, 16, 18, 20, 22 and 24 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand. Nucleotides are numbered for the sake of the nucleic acid of the present invention from 5' to 3' on the first strand and 3' and 5' on the second strand

The nucleotides numbered 2, 4, 6, 8, 10, 12, 14, 16, 18, 20, 22 and 24 may be modified by a modification on the first strand. The nucleotides numbered 1, 3, 5, 7, 9, 11, 13, 15, 17, 19, 21, 23 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand.

Clearly, if the first and/or the second strand are shorter than 25 nucleotides in length, such as 19 nucleotides in length, there are no nucleotides numbered 20, 21, 22, 23, 24 and 25 to be modified. The skilled person understands the description above to apply to shorter strands, accordingly.

One or more modified nucleotides on the first strand may be paired with modified nucleotides on the second strand having a common modification. One or more modified nucleotides on the first strand may be paired with modified nucleotides on the second strand having a different modification. One or more modified nucleotides on the first strand may be paired with unmodified nucleotides on the second strand. One or more modified nucleotides on the second strand may be paired with unmodified nucleotides on the first strand. In other words, the alternating nucleotides can be aligned on the two strands such as, for example, all the modifications in the alternating regions of the second strand are paired with identical modifications in the first strand or alternatively the modifications can be offset by one nucleotide with the common modifications in the alternating regions of one strand pairing with dissimilar modifications (i.e. a second or further modification) in the other strand. Another option is to have dissimilar modifications in each of the strands.

The modifications on the first strand may be shifted by one nucleotide relative to the modified nucleotides on the second strand, such that common modified nucleotides are not paired with each other.

The modification and/or modifications may each and individually be selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide or a non-natural base comprising nucleotide. At least one modification may be 2'-O-methyl and/or at least one modification may be 2'-F. Further modifications as described herein may be present on the first and/or second strand.

Throughout the description of the invention, "same or common modification" means the same modification to any nucleotide, be that A, G, C or U modified with a group such as a methyl group or a fluoro group. Is it not taken to mean the same addition on the same nucleotide. For example, 2'F-dU, 2'F-dA, 2'F-dC, 2'F-dG are all considered to be the same or common modification, as are 2'-OMe-rU, 2'-OMe-rA; 2'-OMe-rC; 2'-OMe-rG. A 2'F modification is a different modification to a 2'OMe modification.

Some representative modified nucleic acid sequences of the present invention are shown in the examples. These examples are meant to be representative and not limiting.

Preferably, the nucleic acid may comprise a modification and a second or further modification which are each and individually selected from the group comprising 2'-O-methyl modification and 2'-F modification. The nucleic acid may comprise a modification that is 2'-O-methyl (2'OMe) that may be a first modification, and a second modification that is 2'-F. The nucleic acid of the invention may also include a phosphorothioate modification and/or a deoxy modification which may be present in or between the terminal 1, 2 or 3 nucleotides of each or any end of each or both strands.

The invention provides as a further aspect, a nucleic acid for inhibiting expression of *ALDH2* in a cell, comprising a nucleotide sequence of SEQ ID NO:1, 3, 5, 7, 9, 11, 13, 15, 17, 19, 21, 23, 25 or 27, wherein the nucleotides of first strand are modified by a first modification on the odd numbered nucleotides, and modified by a second modification on the even numbered nucleotides, and nucleotides of the second strand are modified by a third modification on the even numbered nucleotides and modified by a fourth modification the odd numbered nucleotides, wherein at least the first modification is different to the second modification and the third modification is different to the fourth modification.. The third and first modifications may be the same or different, the second and fourth modifications may be the same or different. The first and second modifications may be different to each other and the third and fourth modifications may be different to each other.

The second strand may comprise a nucleotide sequence of SEQ ID NO:2, 4, 6, 8, 10, 12, 14, 16, 18, 20, 22, 24, 26 or 28. The nucleotides of first strand may be modified by a first modification on the odd numbered nucleotides, and modified with a second modification on the even numbered nucleotides, and the second strand may be modified on the odd numbered nucleotides with the second modification and modified with the first modification on the even numbered nucleotides. The first modification may be 2'OMe and the second modification may be 2' F. The first strand may comprise the nucleotide sequence of SEQ ID NO:3, SEQ ID NO:11 or SEQ ID NO:17 and/or the second strand may comprise the nucleotide sequence of SEQ ID NO:4, SEQ ID NO:12 or SEQ ID NO:18. The modifications may be those as set out in Table 1.

The nucleic acid of the invention may be conjugated to a ligand. Efficient delivery of oligonucleotides, in particular double stranded nucleic acids of the invention, to cells *in vivo* is important and requires specific targeting and substantial protection from the extracellular environment, particularly serum proteins. One method of achieving specific targeting is to conjugate a ligand to the nucleic acid. The ligand helps in targeting the nucleic acid to the required target site. There is a need to conjugate appropriate ligands for the desired receptor molecules in order for the conjugated molecules to be taken up by the target cells by mechanisms such as different receptor-mediated endocytosis pathways or functionally analogous processes.

One example is the Asialoglycoprotein receptor complex (ASGP-R) composed by varying ratios of multimers of membrane ASGR1 and ASGR2 receptors, which is highly abundant on hepatocytes and has high affinity to the here described GalNAc moiety. One of the first disclosures of the use of triantennary cluster glycosides as conjugated ligands was in US patent number US 5,885,968. Conjugates having three GalNAc ligands and comprising phosphate groups are known and are described in Dubber et al. (Bioconjug. Chem. 2003 Jan-Feb;14(1):239-46.). The ASGP-R complex shows a 50-fold higher affinity for N-Acetyl-D-Galactosylamine (GalNAc) than D-Gal.

The asialoglycoprotein receptor complex (ASGP-R), which recognizes specifically terminal β-galactosyl subunits of glycosylated proteins or other oligosaccharides (Weigel, P.H. et. al., Biochim. Biophys. Acta. 2002 Sep 19;1572(2-3):341-63) can be used for delivering a drug to the liver's hepatocytes expressing the receptor complex by covalent coupling of galactose or galactoseamine to the drug substance (Ishibashi,S.; et. al., J Biol. Chem. 1994 Nov 11;269(45):27803-6). Furthermore the binding affinity can be significantly increased by the multi-valency effect, which is achieved by the repetition of the targeting moiety (Biessen EA, et al., J Med Chem. 1995 Apr 28;38(9):1538-46.).

The ASGP-R complex is a mediator for an active uptake of terminal β-galactosyl containing glycoproteins to the cell's endosomes. Thus, the ASGPR is highly suitable for targeted delivery of drug candidates conjugated to such ligands like, e.g., nucleic acids into receptor-expressing cells (Akinc et al., Mol Ther. 2010 Jul;18(7):1357-64).

More generally the ligand can comprise a saccharide that is selected to have an affinity for at least one type of receptor on a target cell. In particular, the receptor is on the surface of a mammalian liver cell, for example, the hepatic asialoglycoprotein receptor complex described before (ASGP-R).

The saccharide may be selected from N-acetyl galactoseamine, mannose, galactose, glucose, glucosamine and fucose. The saccharide may be N-acetyl galactoseamine (GalNAc).

A ligand for use in the present invention may therefore comprise (i) one or more N-acetyl galactosamine (GalNac) moieties and derivatives thereof, and (ii) a linker, wherein the linker conjugates the GalNac moieties to a sequence as defined in any preceding aspects. The linker may be a bivalent or trivalent or tetravalent branched structure. The nucleotides may be modified as defined herein.

"GalNAc" refers to 2-(Acetylamino)-2-deoxy-D- galactopyranose, commonly referred to in the literature as N-acetyl galactosamine. Reference to "GalNAc" or "N-acetyl galactoseamine" includes both the β- form: 2-(Acetylamino)-2-deoxy-β-D-galactopyranose and the α-form: 2-(Acetylamino)-2-deoxy-α-D-galactopyranose. Both the β-form: 2-(Acetylarnino)-2-deoxy-β-D-galactopyranose and α-form: 2-(Acetylamino)-2-deoxy-α-D-galactopyranose may be used interchangeably. Preferably, the compounds of the invention comprise the β-form, 2-(Acetylarnino)-2-deoxy-β-D-galactopyranose.

The ligand may therefore comprise GalNAc.

The ligand may comprise a compound of formula I:

[S-X¹-P-X²]₃-A-X³- (I)

wherein:
S represents a saccharide, wherein the saccharide is N-acetyl galactosamine;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is alkylene or an alkylene ether of the formula (-CH₂)ₙ-O-CH₂- where n = 1- 6;
A is a branching unit;
X³ represents a bridging unit;
wherein a nucleic acid according to the present invention is conjugated to X³ via a phosphate or modified phosphate (preferably a thiophosphate).

In formula I, branching unit "A" branches into three in order to accommodate the three saccharide ligands. The branching unit is covalently attached to the remaining tethered portions of the ligand and the nucleic acid. The branching unit may comprise a branched aliphatic group comprising groups selected from alkyl, amide, disulphide, polyethylene glycol, ether, thioether and hydroxyamino groups. The branching unit may comprise groups selected from alkyl and ether groups.

The branching unit A may have a structure selected from: 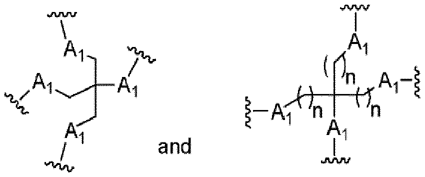
wherein each A₁ independently represents O, S, C=O or NH; and
each n independently represents an integer from 1 to 20.

The branching unit may have a structure selected from: 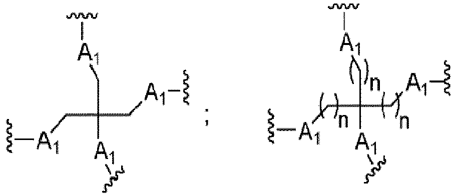 and 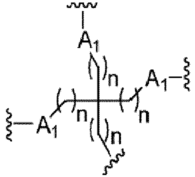
wherein each A₁ independently represents O, S, C=O or NH; and
each n independently represents an integer from 1 to 20.

The branching unit may have a structure selected from: 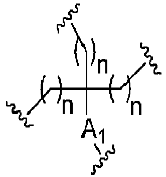 and 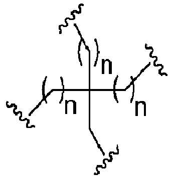
wherein A₁ is O, S, C=O or NH; and
each n independently represents an integer from 1 to 20.

The branching unit may have the structure:

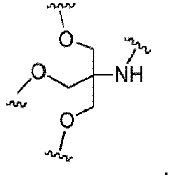

The branching unit may have the structure:

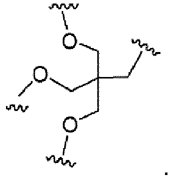

The branching unit may have the structure:

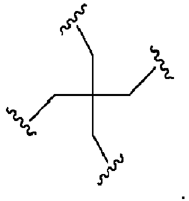

Optionally, the branching unit consists of only a carbon atom.

The "X³" portion is a bridging unit. The bridging unit is linear and is covalently bound to the branching unit and the nucleic acid.

X³ may be selected from -C₁-C₂₀ alkylene-, -C₂-C₂₀ alkenylene-, an alkylene ether of formula -(C₁-C₂₀ alkylene)-O-(C₁-C₂₀ alkylene)-, -C(O)-C₁-C₂₀ alkylene-, -C₀-C₄ alkylene(Cy)C₀-C₄ alkylene- wherein Cy represents a substituted or unsubstituted 5 or 6 membered cycloalkylene, arylene, heterocyclylene or heteroarylene ring, -C₁-C₄ alkylene-NHC(O)-C₁ C₄ alkylene-, -C₁-C₄ alkylene-C(O)NH-C₁-C₄ alkylene-, -C₁-C₄ alkylene-SC(O)-C₁-C₄ alkylene-, -C₁-C₄ alkylene-C(O)S-C₁-C₄ alkylene-, -C₁-C₄ alkylene-OC(O)-C₁-C₄ alkylene-, -C₁-C₄ alkylene-C(O)O-C₁-C₄ alkylene-, and -C₁-C₆ alkylene-S-S-C₁-C₆ alkylene-.

X³ may be an alkylene ether of formula -(C₁-C₂₀ alkylene)-O-(C₁-C₂₀ alkylene)-. X³ may be an alkylene ether of formula -(C1-C20 alkylene)-O-(C₄-C₂₀ alkylene)-, wherein said (C4-C20 alkylene) is linked to Z. X³ may be selected from the group consisting of -CH₂-O-C₃H₆-, - CH₂-O-C₄H₈-, -CH₂-O-C₆H₁₂- and -CH₂-O-C₈H₁₆-, especially -CH₂-O-C₄H₈-, -CH₂-O-C₆H₁₂- and -CH₂-O-C₈H₁₆-, wherein in each case the -CH2- group is linked to A.

The ligand may comprise a compound of formula (II):

[S-X¹-P-X²]₃-A-X³- (II)

wherein:
S represents a saccharide;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is C₁-C₈ alkylene;
A is a branching unit selected from: 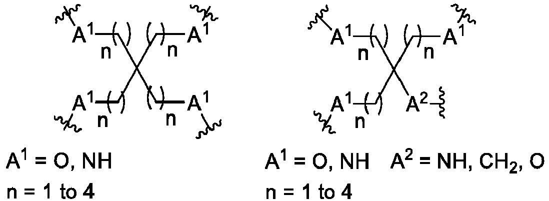
X³ is a bridging unit;.
wherein a nucleic acid according to the present invention is conjugated to X³ via a phosphate or modified phosphate (preferably a thiophosphate)

Branching unit A may have the structure:

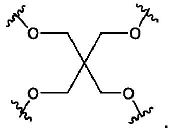

Branching unit A may have the structure: 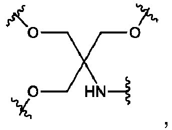 wherein X³ is attached to the nitrogen atom.

X³ may be C1-C20 alkylene. Preferably, X³ is selected from the group consisting of -C₃H₆-, - C₄H₈-, -C₆H₁₂- and -C₈H₁₆-, especially -C₄H₈-, -C₆H₁₂- and -C₈H₁₆-.

The ligand may comprise a compound of formula (III):

[S-X¹-P-X²]₃-A-X³- (III)

wherein:
S represents a saccharide;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is an alkylene ether of formula -C₃H₆-O-CH₂-;
A is a branching unit;
X³ is an alkylene ether of formula selected from the group consisting of -CH2-O-CH2-, - CH₂-O-C₂H₄-, -CH₂-O-C₃H₆-, -CH₂-O-C₄H₈-, -CH₂-O-C₅H₁₀-, -CH₂-O-C₆H₁₂-, -CH₂-O-C₇H₁₄-, and -CH₂-O-C₈H₁₆-, wherein in each case the -CH2- group is linked to A,
and wherein X³ is conjugated to a nucleic acid according to the present invention by a phosphate or modified phosphate (preferably a thiophosphate).

The branching unit may comprise carbon. Preferably, the branching unit is carbon.

X³ may be selected from the group consisting of -CH₂-O-C₄H₈-, -CH₂-O-C₅H₁₀-, -CH₂-O-C₆H₁₂-, -CH₂-O-C₇H₁₄-, and -CH₂-O-C₈H₁₆-. Preferably, X³ is selected from the group consisting of -CH₂-O-C₄H₈-, -CH₂-O-C₆H₁₂- and -CH₂-O-C₈H₁₆.

For any of the above aspects, when P represents a modified phosphate group, P can be represented by: 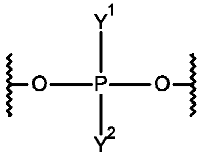 wherein Y¹ and Y² each independently represent =O, =S, -O⁻, -OH, -SH, -BH3, -OCH₂CO₂, -OCH₂CO₂R^{x}, -OCH₂C(S)OR^{x}, and -OR^{x}, wherein R^{x} represents C₁-C₆ alkyl and wherein 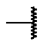 indicates attachment to the remainder of the compound.

By modified phosphate It is meant a phosphate group wherein one or more of the non-linking oxygens is replaced. Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulphur. One, each or both non-linking oxygens in the phosphate group can be independently any one of S, Se, B, C, H, N, or OR (R is alkyl or aryl).

The phosphate can also be modified by replacement of a linking oxygen with nitrogen (bridged phosphoroamidates), sulfur (bridged phosphorothioates) and carbon (bridged methylenephosphonates). The replacement can occur at a terminal oxygen. Replacement of the non-linking oxygens with nitrogen is possible.

For example, Y¹ may represent -OH and Y² may represent =O or =S; or
Y¹ may represent -O⁻ and Y² may represent =O or =S;
Y¹ may represent =O and Y² may represent -CH₃, -SH, -OR^{x}, or -BH₃
Y¹ may represent =S and Y² may represent -CH3, OR^{x} or -SH.

It will be understood by the skilled person that in certain instances there will be delocalisation between Y¹ and Y².

Preferably, the modified phosphate group is a thiophosphate group. Thiophosphate groups include bithiophosphate (i.e. where Y¹ represents =S and Y² represents -S⁻) and monothiophosphate (i.e. where Y¹ represents -O⁻ and Y² represents =S, or where Y¹ represents =O and Y² represents -S⁻). Preferably, P is a monothiophosphate. The inventors have found that conjugates having thiophosphate groups in replacement of phosphate groups have improved potency and duration of action *in vivo.* P may also be an ethylphosphate (i.e. where Y¹ represents =O and Y² represents OCH₂CH₃).

The saccharide may be selected to have an affinity for at least one type of receptor on a target cell. In particular, the receptor is on the surface of a mammalian liver cell, for example, the hepatic asialoglycoprotein receptor complex(ASGP-R).

For any of the above aspects, the saccharide may be selected from N-acetyl with one or more of galactosamine, mannose, galactose, glucose, glucosamine and fructose. Typically a ligand to be used in the present invention may include N-acetyl galactosamine (GalNAc). Preferably the compounds of the invention may have 3 ligands, which will each preferably include N-acetyl galactosamine.

"GalNAc" refers to 2-(Acetylamino)-2-deoxy-D- galactopyranose, commonly referred to in the literature as N-acetyl galactosamine. Reference to "GalNAc" or "N-acetyl galactosamine" includes both the β- form: 2-(Acetylamino)-2-deoxy-β-D-galactopyranose and the α-form: 2-(Acetylamino)-2-deoxy-α-D-galactopyranose. In certain embodiments, both the β-form: 2-(Acetylarnino)-2-deoxy-β-D-galactopyranose and α-form: 2-(Acetylamino)-2-deoxy-α-D-galactopyranose may be used interchangeably. Preferably, the compounds of the invention comprise the β-form, 2-(Acetylarnino)-2-deoxy-β-D-galactopyranose.

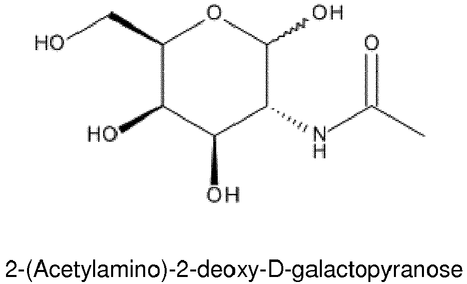

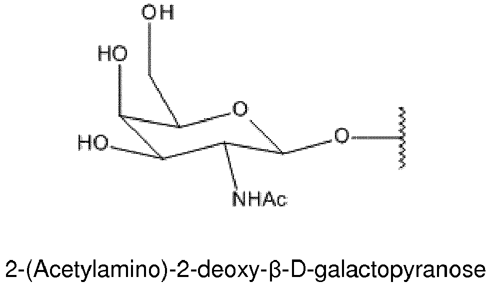

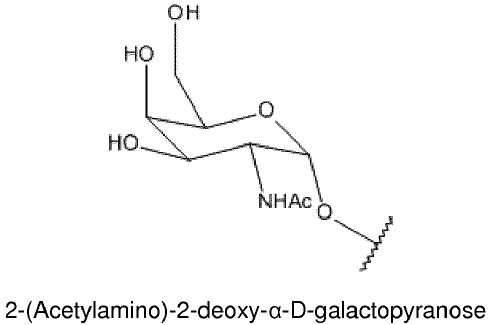

For any of the above compounds of formula (III), X¹ may be (-CH₂-CH₂-O)(-CH₂)₂-. X¹ may be (-CH₂-CH₂-O)₂(-CH₂)₂-. X¹ may be (-CH₂-CH₂-O)₃(-CH₂)₂-. Preferably, X¹ is (-CH₂-CH₂-O)₂(-CH₂)₂-. Alternatively, X¹ represents C₃-C₆ alkylene. X¹ may be propylene. X¹ may be butylene. X¹ may be pentylene. X¹ may be hexylene. Preferably the alkyl is a linear alkylene. In particular, X¹ may be butylene.

For compounds of formula (III), X² represents an alkylene ether of formula -C₃H₆-O-CH₂- i.e. C₃ alkoxy methylene, or -CH₂CH₂CH₂OCH₂-.

The invention provides a conjugated nucleic acid having one of the following structures: 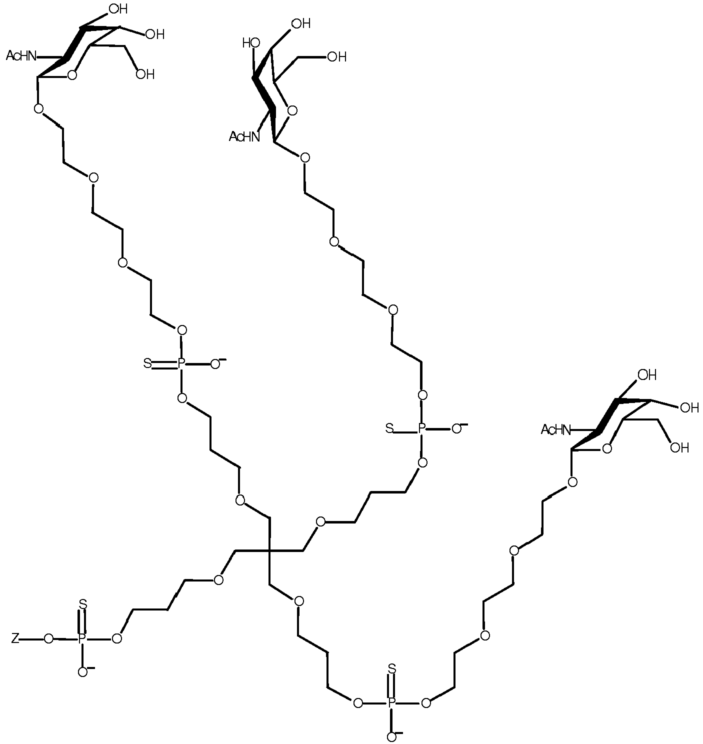 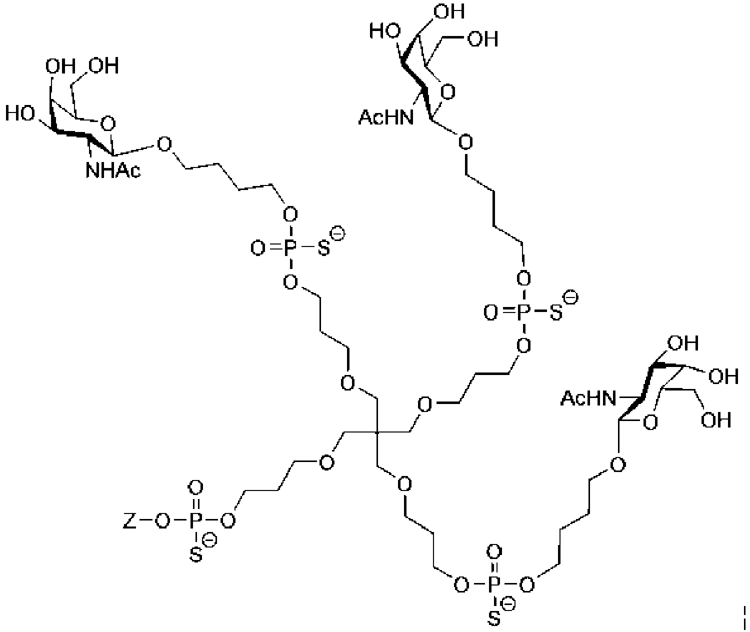 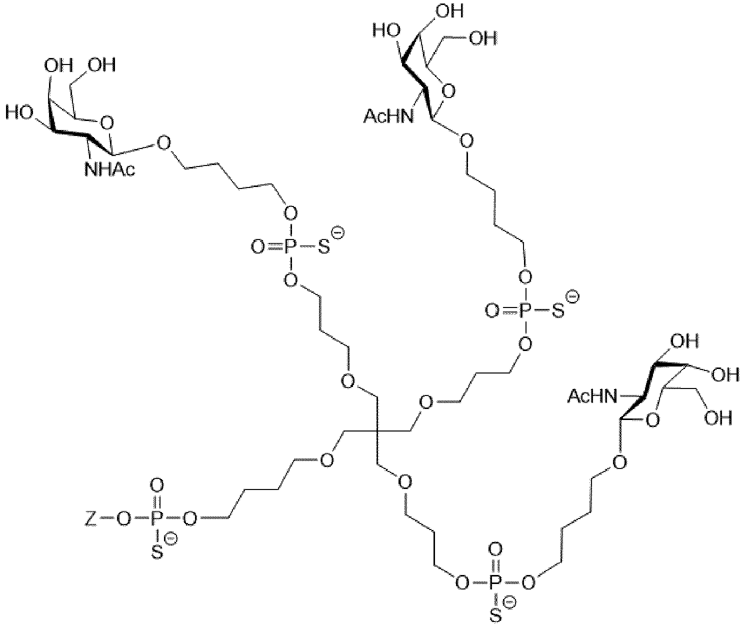 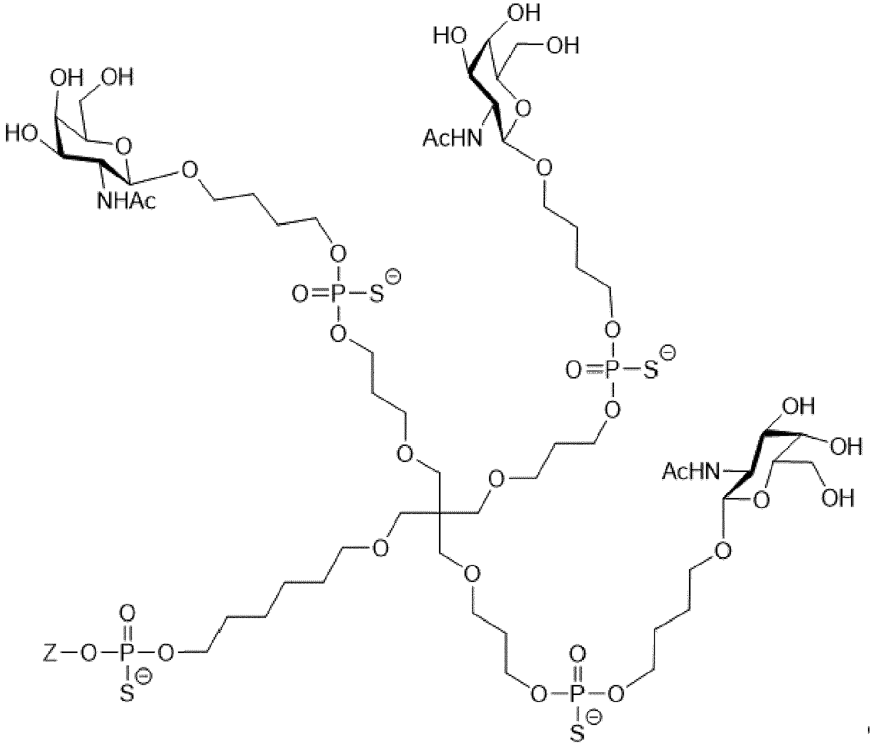 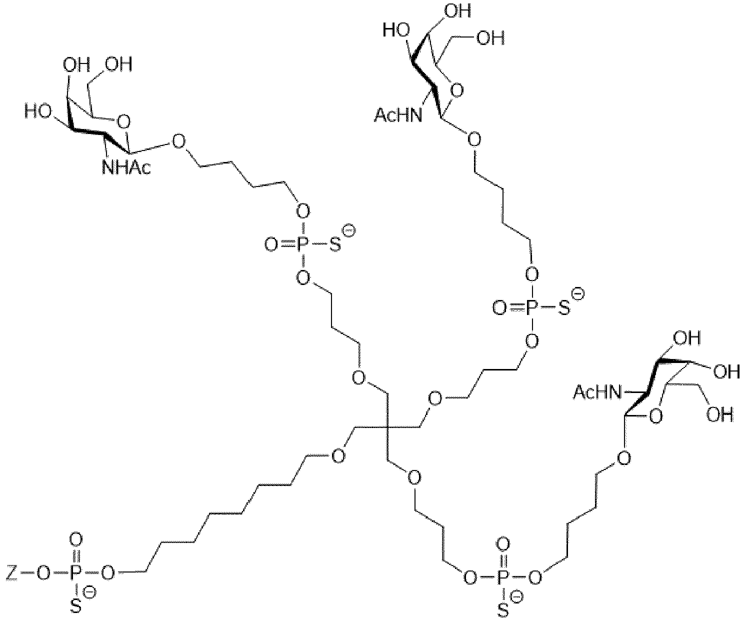 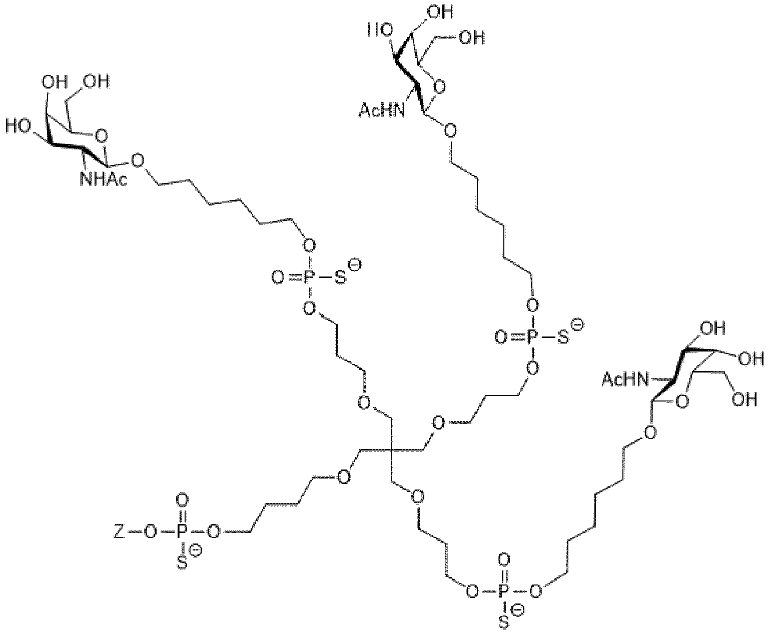 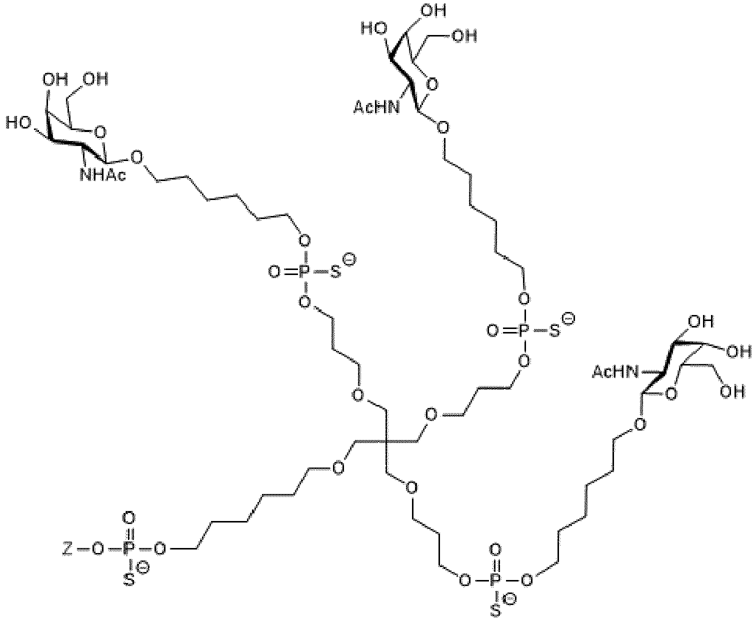 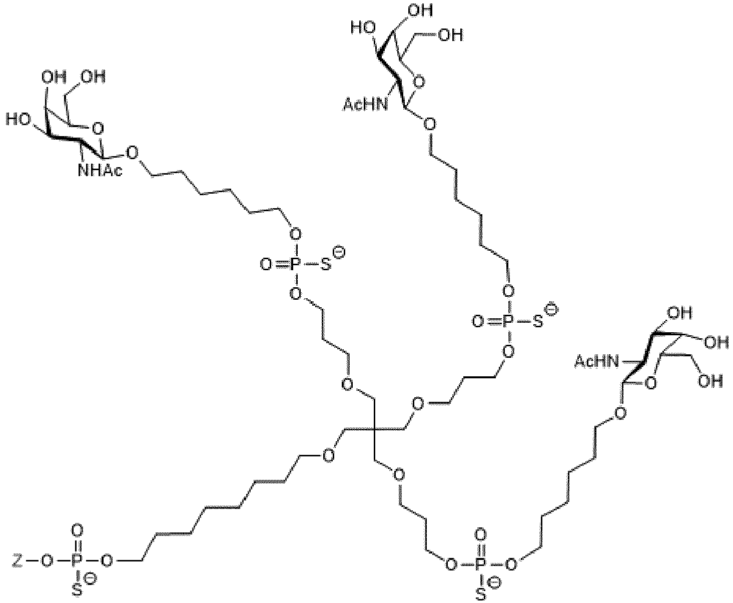 wherein Z is a nucleic acid as defined herein before..

The invention provides, as another aspect, a nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of a RNA transcribed from the *ALDH2* gene, wherein said first strand comprises a nucleotide sequence selected from the following sequences: SEQ ID NOs:1, 3, 5, 7, 9, 11, 13, 15, 17, 19, 21, 23, 25 or 27, wherein the nucleic acid is conjugated to a ligand. The second strand may comprise a nucleotide sequence of SEQ ID NO:2, 4, 6, 8, 10, 12, 14, 16, 18, 20, 22, 24, 26 or 28. The nucleotides of the first and/or second strand may be modified, as herein described.

Preferably, the nucleic acid comprises SEQ ID NO:3, SEQ ID NO:11 or SEQ ID NO:17 and SEQ ID NO:4, SEQ ID NO:12 or SEQ ID NO:18 conjugated to a ligand of formula I (as set out above), wherein the ligand is conjugated to the nucleic acid as described and wherein the first strand is modified with a 2'OMe modification on the odd numbered nucleotides, and modified with a 2'F on the even numbered nucleotides, and the second strand is modified with a 2'OMe on the even numbered nucleotides and modified with a 2'F on the odd numbered nucleotides.

More preferably, the nucleic acid comprises SEQ ID NO:3, SEQ ID NO:11 or SEQ ID NO:17 and SEQ ID NO:4, SEQ ID NO:12 or SEQ ID NO:18, wherein the nucleic acid is conjugated to a ligand of formula I (as set out above), and furthermore wherein the nucleic acid has a modification pattern as shown below which is an extract of Table 1 as herein provided.

| **Sequence ID No** | **siRNA ID** | **sequence** | **modifications** |
|---|---|---|---|
| 3 | ALDH2-hcm-2 | 5'-AAUGUUUUCCUGCUGACGG-3' | 6254515173547182748 |
| 4 | | 5'-CCGUCAGCAGGAAAACAUU-3' | 3745364728462627251 |
| 11 | ALDH2-hcm-6 | 5'-UCUUCUUAAACUGAGUUUC-3' | 5351715262718281517 |
| 12 | | 5'-GAAACUCAGUUUAAGAAGA-3' | 4626353645152646282 |
| 17 | ALDH2-hcm-9 | 5'-AUGUAGCCGAGGAUCUUCU-3' | 6181647382846171535 |
| 18 | | 5'-AGAAGAUCCUCGGCUACAU-3' | 2826461735384716361 |

wherein the specific modifications are depicted by numbers
1=2'F-dU,
2=2'-F-dA,
3=2'F-dC,
4=2'F-dG,
5=2'-OMe-rU;
6=2'-OMe-rA;
7=2'-OMe-rC;
8=2'-OMe-rG.

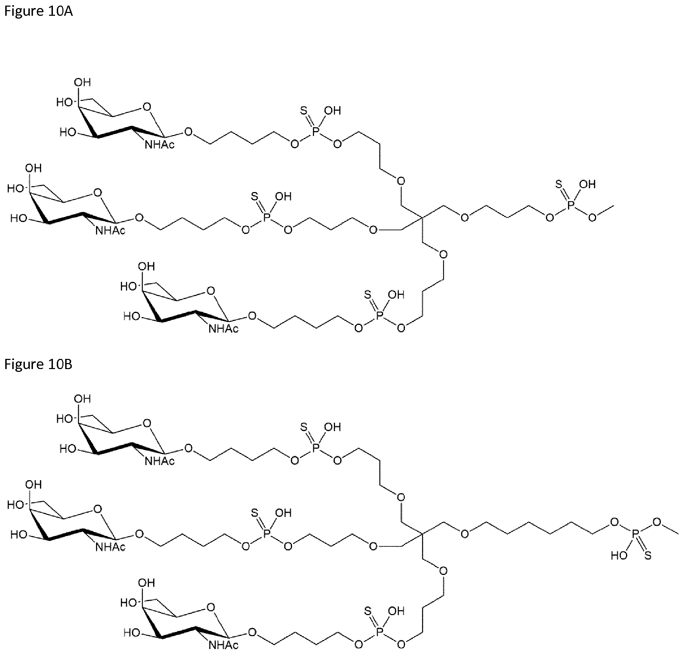

The ligand may comprise GalNac and Figure 10A or Figure 10B further illustrate examples of the present invention.

The present invention also provides pharmaceutical compositions comprising the nucleic acid or conjugated nucleic acid of the invention. The pharmaceutical compositions may be used as medicaments or as diagnostic agents, alone or in combination with other agents. For example, one or more nucleic acid conjugates of the invention can be combined with a delivery vehicle (e.g., liposomes) and excipients, such as carriers, diluents. Other agents such as preservatives and stabilizers can also be added. Methods for the delivery of nucleic acids are known in the art and within the knowledge of the person skilled in the art.

The nucleic acid or conjugated nucleic acid of the present invention can also be administered in combination with other therapeutic compounds, either administrated separately or simultaneously, e.g., as a combined unit dose. The invention also includes a pharmaceutical composition comprising one or more nucleic acids or conjugated nucleic acids according to the present invention in a physiologically/pharmaceutically acceptable excipient, such as a stabilizer, preservative, diluent, buffer, and the like.

Dosage levels for the medicament and pharmaceutical compositions of the invention can be determined by those skilled in the art by routine experimentation. In one embodiment, a unit dose may contain between about 0.01 mg/kg and about 100 mg/kg body weight of nucleic acid or conjugated nucleic acid. Alternatively, the dose can be from 10 mg/kg to 25 mg/kg body weight, or 1 mg/kg to 10 mg/kg body weight, or 0.05 mg/kg to 5 mg/kg body weight, or 0.1 mg/kg to 5 mg/kg body weight, or 0.1 mg/kg to1 mg/kg body weight, or 0.1 mg/kg to 0.5 mg/kg body weight, or 0.5 mg/kg to 1 mg/kg body weight. Dosage levels may also be calculated via other parameters such as, e.g., body surface area.

The pharmaceutical composition may be a sterile injectable aqueous suspension or solution, or in a lyophilized form.

The pharmaceutical compositions and medicaments of the present invention may be administered to a mammalian subject in a pharmaceutically effective dose. The mammal may be selected from a human, a non-human primate, a simian or prosimian, a dog, a cat, a horse, cattle, a pig, a goat, a sheep, a mouse, a rat, a hamster, a hedgehog and a guinea pig, or other species of relevance. On this basis, the wording "ALDH2" or *"ALDH2"* as used herein denotes nucleic acid or protein in any of the above mentioned species, if expressed therein naturally or artificially, but preferably this wording denotes human nucleic acids or proteins.

A further aspect of the invention relates to a nucleic acid or conjugated nucleic acid of the invention or the pharmaceutical composition comprising the nucleic acid or conjugated nucleic acid of the invention for use in the treatment of a disease, disorder or syndrome. The disease, disorder or syndrome may be alcohol use disorder which includes acute alcohol sensitivity (hangover), alcoholic neuropathy (alcohol-related polyneuropathy), alcohol dependence, alcohol abuse (alcoholism), or fetal alcohol syndrome (alcohol-related neurodevelopmental disorder) or any other pathology associated to acute or prolonged excessive alcohol consumption. The invention includes a pharmaceutical composition comprising one or more nucleic acids or conjugated nucleic acids according to the present invention in a physiologically/ pharmaceutically acceptable excipient, such as a stabiliser, preservative, diluent, buffer and the like.

The pharmaceutical composition may be a sterile injectable aqueous suspension or solution, or in a lyophilised form or adhered, absorbed or included to or into any other suitable galenic carrier substance such as pellets, tablets, capsules, nanoparticles, gels, tablets, beads or similar structures.

The nucleic acid described herein may be capable of inhibiting the expression of *ALDH2.* The nucleic acid described herein may be capable of partially inhibiting the expression of *ALDH2.* Inhibition may be complete, i.e. 0% compared of the expression level of *ALDH2* in the absence of the nucleic acid of the invention. Inhibition of *ALDH2* expression may be partial, i.e. it may be 15%, 20%, 30%, 40%, 50%, 60%, 70%, 75%, 80%, 85%, 90%, 95% or intermediate values of *ALDH2* expression in the absence of a nucleic acid of the invention. Inhibition may last 2 weeks, 3 weeks, 4 weeks, 5 weeks, 6 weeks, 7 weeks, 8 weeks, 10 weeks, 11 weeks, 12 weeks, 13 weeks, 14 weeks or up to 3 months, when used in a subject, such as a human patient. A nucleic acid or conjugated nucleic acid of the invention, or compositions including the same, may be for use in a regimen comprising treatments once or twice weekly, every week, every two weeks, every three weeks, every four weeks, every five weeks, every six weeks, every seven weeks, or every eight weeks, or in regimens with varying dosing frequency such as combinations of the before-mentioned intervals. The nucleic acid may be for use subcutaneously, intravenously or using any other application routes such as oral, rectal or intraperitoneal.

In cells and/or subjects treated with or receiving the nucleic acid or conjugated nucleic acid of the present invention, the *ALDH2* expression may be inhibited compared to untreated cells and/or subjects by a range from 15% up to 100% but at least about 30%, 35%, 40%, 45%, 50%, 55%, 60%, 65%, 70%, 75%, 80%, 85%, 90%, 95%, 98%, or 100% or intermediate values. The level of inhibition may allow treatment of a disease associated with *ALDH2* expression or overexpression, or may serve to further investigate the functions and physiological roles of the *ALDH2* gene product.

A further aspect of the invention relates to a nucleic acid or conjugated nucleic acid of the invention in the manufacture of a medicament for treating a disease, disorder or syndromes, such as those as listed above or additional pathologies associated with acute or prolonged excessive alcohol consumption, or additional therapeutic approaches where inhibition of *ALDH2* expression is desired.

Also included in the invention is a method of treating or preventing a disease, disorder or syndrome, such as those listed above, comprising administration of a pharmaceutical composition comprising a nucleic acid or conjugated nucleic acid as described herein, to an individual in need of treatment (to improve such pathologies). The nucleic acid composition may be administered in a regimen comprising treatments twice every week, once every week, every two weeks, every three weeks, every four weeks, every five weeks, every six weeks, every seven weeks, or every eight weeks or in regimens with varying dosing frequency such as combinations of the before-mentioned intervals. The nucleic acid or conjugated nucleic acid may be for use subcutaneously or intravenously or other application routes such as oral, rectal or intraperitoneal.

The nucleic acid or conjugated nucleic acid of the present invention can also be administered for use in combination with other therapeutic compounds, either administered separately or simultaneously, e.g., as a combined unit dose. A molecular conjugation to other biologically active molecular entities such as peptides, cellular or artificial ligands or small and large molecules is also possible.

The nucleic acid or conjugated nucleic acid of the present invention can be produced using routine methods in the art including chemical synthesis or expressing the nucleic acid either *in vitro* (e.g., run off transcription) or *in vivo.* For example, using solid phase chemical synthesis or using a nucleic acid-based expression vector including viral derivates or partially or completely synthetic expression systems. In one embodiment, the expression vector can be used to produce the nucleic acid of the invention *in vitro,* within an intermediate host organism or cell type, within an intermediate or the final organism or within the desired target cell. Methods for the production (synthesis or enzymatic transcription) of the nucleic acid described herein are known to persons skilled in the art.

The invention consists of chemical molecular entities that mediate *ALDH2* mRNA degradation by binding to the *ALDH2* gene transcripts through cellular RNA interference mechanisms. The molecular compounds invented may be used as conjugates with, but are not limited to an N-acetylgalactosamin (GalNAc) sugar moiety that ensures hepatocyte-specific cellular uptake, though specific binding to the asialoglycoprotein receptor complex (ASGPR). The invention may be linked to other different chemical structures conferring different properties as referred to in the following. The use of a chemical modification pattern of the nucleic acids confers nuclease stability in serum and makes for example subcutaneous application route feasible.

Low substrate specificity of known drugs inhibiting up to 20 different enzymes in addition to ALDH2 leads to severe undesired physiological effects in several tissues such as heart muscle and the central and peripheral nervous system even in non-drinking patients. The present invention aims at conferring assistance in alcohol dishabituation inducing alcohol aversion with a better safety profile than the currently available treatments. The invention is characterized by high specificity at the molecular and tissue-directed delivery level.

The invention will now be described with reference to the following non-limiting Figures and Examples.

### Figures

Figure 1 shows the results of a unconjugated RNAi molecule screen for inhibition of *ALDH2* expression transfecting human Hep3B cells;
Figure 2 shows the dose response of *ALDH2* siRNA molecules in human HepG2 cells;
Figure 3 shows mRNA and protein reduction by ALDH2-hcm-2 siRNA in human HepG2 cells as well as its effect on ALDH enzymatic activity in HepG2 total cell extracts;
Figure 4 shows the dose-response for GalNAc-coupled siRNAs targeting *Aldh2* mRNA in primary mouse hepatocytes;
Figure 5 shows the dose response for GalNAc-coupled siRNAs against *ALDH2* mRNA in primary human hepatocytes;
Figure 6 shows the reduction of *ALDH2* mRNA by 15nM of GalNAc-coupled ALDH2-hcm-2 siRNA in 1° human, cynomolgus and mouse hepatocytes;
Figure 7 shows the comparison in dose-response on *ALDH2* mRNA knockdown by GalNAc-conjugated *ALDH2* siRNAs hcm2, hcm6, hcm9 in conjugation to L1 linker vs. conjugation to the L6 GalNAc-linker in 1° human hepatocytes;
Figure 8a show *Aldh2* mRNA knockdown in mice *in vivo* 10 days post treatment, 8b the consequent reduction of Aldh2 protein and 8c the resulting hepatic acetaldehyde metabolite levels in the same mice;
Figure 9 shows the dose-dependent duration of the *Aldh2* mRNA knockdown up to 41 days upon a single dose application in mice using as an example GalNAc-ALDH2-hcm6 and hcm9 conjugated to the L6 linked GalNAc moiety; and
Figures 10a and 10b show the structure of the GalNac L1 and L6 ligands, respectively, to which the example oligonucleotides were conjugated.
Figure 11 shows different siRNA duplexes containing inverted RNA nucleotides at both 3'- ends tested for serum stability.
Figure 12 shows the influence on *ALDH2* mRNA knockdown of inverted A, U, C and G RNA nucleotides at 3'-overhang positions using derivatives of the siRNA ALDH2-hcm2. (see Table 3).
Figure 13 shows the influence on *ALDH2* mRNA knockdown of inverted A, U, C and G RNA nucleotides at terminal 3' positions using derivatives of the siRNA ALDH2-hcm2 (see Table 3).
Figure 14 shows different GalNAc-L6-linker conjugates of ALDH2-hcm2 containing inverted RNA nucleotides (see Table 4) tested for serum stability.
Figure 15 shows the dose response of inverted RNA nucleotides at terminal 3' positions on the efficacy of *ALDH2* mRNA knockdown using GalNac-L6-conjugated derivatives of the siRNA ALDH2-hcm2 (see Table 4) by ASGPR receptor complex-mediated uptake in mouse primary hepatocytes.
Figure 16 shows the results of an *in vivo* study in mice testing the influence of ivA at the first strand's 3'-end in GalNAc-L6 derivatives of ALDH2-hcm6 and ALDH2-hcm9 siRNA conjugates on the efficacy of *ALDH2* mRNA knockdown
Figure 17 shows the effect of different modified derivatives of the GalNAc-siRNA conjugates of ALDH2-hcm6 and ALDH2-hcm9 (Table 5) on knockdown efficacy on *ALDH2* mRNA expression levels *in vivo* in mice.

### Examples

### Example 1

Nucleic acids in accordance with the invention were synthesised, using the oligonucleotides as set out in the tables below.

The method of synthesis was as follows, using one of the sequences of the invention as an example:

### ALDH2-hcm-2

### GalNAc-ALDH2-hcm-2-L1:

First strand (SEQ ID NO: 3)
   5'OMeA(ps)-FA-(ps)-OMeU-FG-OMeU-FU-OMeU-FU-OMeC-FC-OMeU-FG-OMeC-FU-OMeG-FA-OMeC-(ps)-FG-(ps)-OMeG 3'
Second strand (SEQ ID NO: 4)
   5'[ST23 (ps)]3 long trebler (ps) FC-OMeC-FG-OMeU-FC-OMeA-FG-OMeC-FA-OMeG-FG-OMeA-FA-OMeA-FA-OMeC-FA-(ps)-OMeU-(ps)-FU 3'

### GalNAc-ALDH2-hcm-2-L6:

First strand (SEQ ID NO: 3)
   5'OMeA(ps)- FA-(ps)-OMeU-FG-OMeU-FU-OMeU-FU-OMeC-FC-OMeU-FG-OMeC-FU-OMeG-FA-OMeC-(ps)-FG-(ps)-OMeG 3'
Second strand (SEQ ID NO: 4)
   5'[ST23 (ps)]3 ST43 (ps) FC-OMeC-FG-OMeU-FC-OMeA-FG-OMeC-FA-OMeG-FG-OMeA-FA-OMeA-FA-OMeC-FA-(ps)-OMeU-(ps)-FU 3'
FN (N=A, C, G, U) denotes 2'Fluoro, 2' DeoxyNucleosides
   OMeN (N=A, C, G, U) denotes 2'O Methyl Nucleosides
   (ps) indicates a phosphorothioate linkage
ST23 and ST43 are as below.

### A further example is ALDH2-hcm-6

### GalNAc-ALDH2-hcm6-L1

First strand (SEQ ID NO: 11)
   5'OMeU-(ps)-FC-(ps)-OMeU-FU-OMeC-FU-OMeU-FA-OMeA-FA-OMeC-FU-OMeG-FA-OMeG-FU-OMeU-(ps)-FU-(ps)-OMeC
Second strand (SEQ ID NO: 12)
   5'[ST23 (ps)]3 long trebler (ps) FG-OMeA-FA-OMeA-FC-OMeU-FC-OMeA-FG-OMeU-FU-OMeU-FA-OMeA-FG-OMeA-FA-(ps)-OMeG-(ps)-FA 3'

### GalNAc-ALDH2-hcm6-L6

First strand (SEQ ID NO: 11)
   5'OMeU-(ps)-FC-(ps)-OMeU-FU-OMeC-FU-OMeU-FA-OMeA-FA-OMeC-FU-OMeG-FA-OMeG-FU-OMeU-(ps)-FU-(ps)-OMeC
Second strand (SEQ ID NO: 12)
   5'[ST23 (ps)]3 ST43 (ps) FG-OMeA-FA-OMeA-FC-OMeU-FC-OMeA-FG-OMeU-FU-OMeU-FA-OMeA-FG-OMeA-FA-(ps)-OMeG-(ps)-FA 3'
FN (N=A, C, G, U) denotes 2'Fluoro, 2' DeoxyNucleosides
   OMeN (N=A, C, G, U) denotes 2'O Methyl Nucleosides
   (ps) indicates a phosphorothioate linkage
ST23 and ST43 are as below.

### A further example is ALDH2-hcm-9

### GalNAc-ALDH2-hcm9-L1

First strand (SEQ ID NO: 17)
   5'OMeA-(ps)-FU-(ps)-OMeG-FU-OMeA-FG-OMeC-FC-OMeG-FA-OMeG-FG-OMeA-FU-OMeC-FU-OMeU-(ps)-FC-(ps)-OMeU 3'
Second strand (SEQ ID NO:18)
   5'[ST23 (ps)]3 long trebler (ps) FA-OMeG-FA-OMeA-FG-OMeA-FU-OMeC-FC-OMeU-FC-OMeG-FG-OMeC-FU-OMeA-FC-(ps)-OMeA-(ps)-FU 3'

### GalNAc-ALDH2-hcm9-L6

First strand (SEQ ID NO: 17)
   5'OMeA-(ps)-FU-(ps)-OMeG-FU-OMeA-FG-OMeC-FC-OMeG-FA-OMeG-FG-OMeA-FU-OMeC-FU-OMeU-(ps)-FC-(ps)-OMeU 3'
Second strand (SEQ ID NO:18)
   5'[ST23 (ps)]3 ST43 (ps) FA-OMeG-FA-OMeA-FG-OMeA-FU-OMeC-FC-OMeU-FC-OMeG-FG-OMeC-FU-OMeA-FC-(ps)-OMeA-(ps)-FU 3'

### GalNAc-ALDH2-hcm9-L4

First strand (SEQ ID NO: 17)
   5'OMeA-(ps)-FU-(ps)-OMeG-FU-OMeA-FG-OMeC-FC-OMeG-FA-OMeG-FG-OMeA-FU-OMeC-FU-OMeU-(ps)-FC-(ps)-OMeU 3'
Second strand (SEQ ID NO:18)
   5'[ST23 (ps)]3 ST41 (ps) FA-OMeG-FA-OMeA-FG-OMeA-FU-OMeC-FC-OMeU-FC-OMeG-FG-OMeC-FU-OMeA-FC-(ps)-OMeA-(ps)-FU 3'
FN (N=A, C, G, U) denotes 2'Fluoro, 2' DeoxyNucleosides
   OMeN (N=A, C, G, U) denotes 2'O Methyl Nucleosides
   (ps) indicates a phosphorothioate linkage
ST23, ST41 and ST43 are as below.
ST23 is a GalNac C4 phosphoramidite (structure components as below) 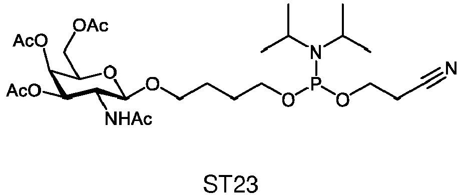 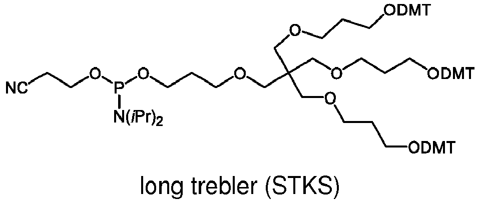
ST41 is as follows: 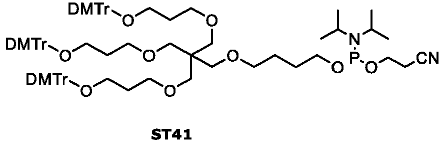
ST43 is as follows and as described in WO2017/174657:

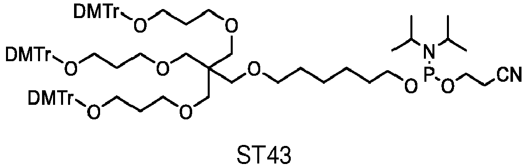

All oligonucleotides were either obtained from a commercial oligonucleotide manufacturer (Biospring, Frankfurt, Germany) or synthesized on an AKTA oligopilot synthesizer (in house) using standard phosphoramidite chemistry. Commercially available solid support and 2'O-Methyl RNA phosphoramidtes, 2'Fluoro DNA phosphoramidites (all standard protection) and commercially available long trebler phosphoramidite (Glen research) were used. Synthesis was performed using 0.1 M solutions of the phosphoramidite in dry acetonitrile and benzylthiotetrazole (BTT) was used as activator (0.3M in acetonitrile). All other reagents were commercially available standard reagents.

Conjugation of the respective GalNac synthon (e.g., ST23, ST41 or ST43) was achieved by coupling of the respective phosphoramidite to the 5' end of the oligochain under standard phosphoramidite coupling conditions. Phosphorothioates were introduced using standard commercially available thiolation reagents (EDITH, Link technologies).

The single strands were cleaved off the CPG by using methylamine (40% aqueous) and the resulting crude oligonucleotide was purified by Ion exchange chromatography (Resource Q, 6mL, GE Healthcare) on a AKTA Pure HPLC System using a Sodium chloride gradient. Product containing fractions were pooled, desalted on a size exclusion column (Zetadex, EMP Biotech) and lyophilised.

For annealing, equimolar amounts of the respective single strands were dissolved in water and heated to 80°C for 5min. After gradual cooling to RT the resulting duplex was lyophilised.

The sequences of the resulting nucleic acids (siRNAs) are set out in Table 1 below.

Table 1: nucleic acid sequences tested for inhibition of *ALDH2* expression. Nucleic acids were synthesized by Biospring, Frankfurt, Germany.

**Table 1**

| **Sequence ID No** | **siRNA ID** | **sequence** | **modifications** |
|---|---|---|---|
| 1 | ALDH2-h-1 | 5'-UUGUUUAUGAAAAUCUGGU-3' | 5181516182626171845 |
| 2 | | 5'-ACCAGAUUUUCAUAAACAA-3' | 2736461515361626362 |
| 3 | ALDH2-hcm-2 | 5'-AAUGUUUUCCUGCUGACGG-3' | 6254515173547182748 |
| 4 | | 5'-CCGUCAGCAGGAAAACAUU-3' | 3745364728462627251 |
| 5 | ALDH2-hc-3 | 5'-UUGAACUUCAGGAUCUGCA-3' | 5182635172846171836 |
| 6 | | 5'-UGCAGAUCCUGAAGUUCAA-3' | 1836461735462815362 |
| 7 | ALDH2-hc-4 | 5'-UUUCACUUCAGUGUAUGCC-3' | 5153635172818161837 |
| 8 | | 5'-GGCAUACACUGAAGUGAAA-3' | 4836163635462818262 |
| 9 | ALDH2-hc-5 | 5'-UUCUUAUGAGUUCUUCUGA-3' | 5171525464517153546 |
| 10 | | 5'-UCAGAAGAACUCAUAAGAA-3' | 1728264627172526462 |
| 11 | ALDH2-hcm-6 | 5'-UCUUCUUAAACUGAGUUUC-3' | 5351715262718281517 |
| 12 | | 5'-GAAACUCAGUUUAAGAAGA-3' | 4626353645152646282 |
| 13 | ALDH2-hc-7 | 5'-UCCUUGAUCAGGUUGGCCA-3' | 5371546172845184736 |
| 14 | | 5'-UGGCCAACCUGAUCAAGGA-3' | 1847362735461726482 |
| 15 | ALDH2-hc-8 | 5'-UUGAAGAACAGGGCGAAGU-3' | 5182646272848382645 |
| 16 | | 5'-ACUUCGCCCUGUUCUUCAA-3' | 2715383735451715362 |
| 17 | ALDH2-hcm-9 | 5'-AUGUAGCCGAGGAUCUUCU-3' | 6181647382846171535 |
| 18 | | 5'-AGAAGAUCCUCGGCUACAU-3' | 2826461735384716361 |
| 19 | ALDH2-hmr-10 | 5'-AUCUGGUUGCAGAAGACCU-3' | 6171845183646282735 |
| 20 | | 5'-AGGUCUUCUGCAACCAGAU-3' | 2845351718362736461 |
| 21 | ALDH2-hr-11 | 5'-AAAUCCACCAGGUAGGAGA-3' | 6261736372845284646 |
| 22 | | 5'-UCUCCUACCUGGUGGAUUU-3' | 1717352735481846151 |
| 23 | ALDH2-hm-12 | 5'-ACUCUCUUGAGGUUGCUGC-3' | 6353535182845183547 |
| 24 | | 5'-GCAGCAACCUCAAGAGAGU-3' | 4728362735362828281 |
| 25 | ALDH2-hcmr-13 | 5'-AUGUCCAAAUCCACCAGGU-3' | 6181736261736372845 |
| 26 | | 5'-ACCUGGUGGAUUUGGACAU-3' | 2735481846151846361 |
| 27 | ALDH2-hm-14 | 5'-CCAUGCUUGCAUCAGGAGC-3' | 7361835183617284647 |
| 28 | | 5'-GCUCCUGAUGCAAGCAUGG-3' | 4717354618362836184 |

Nucleotides modifications are depicted by the following numbers (column 4), 1=2'F-dU, 2=2'F-dA, 3=2'F-dC, 4=2'F-dG, 5=2'-OMe-rU; 6=2'-OMe-rA; 7=2'-OMe-rC; 8=2'-OMe-rG.

### Example 2

Screening for inhibition *ALDH2* mRNA expression in human Hep3B cells was carried out as follows.

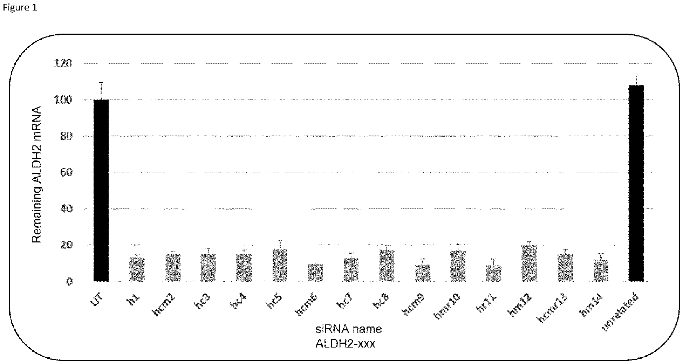

Cells were plated at a density of 150,000 cells per well in 6-well format. 24 hours post seeding, cells were transfected with 20 nM of indicated non-conjugated siRNAs using 1 µg/ml of AtuFECT as a liposomal transfection reagent. 48 hours after transfection, RNA was isolated using the Spin Cell Mini Kit 250 (Stratec). *ALDH2* mRNA levels were determined by qRT-PCR relative to PTEN mRNA expression as housekeeper. Values were normalized to the amount of *ALDH2* in untreated cells (UT). "Unrelated" denotes an siRNA directed against Firefly-Luciferase as non-targeting control. Means and SD of normalized triplicate measurements are shown. Results are shown in Figure 1.

Table 2: Sequences of *ALDH2, ApoB, beta-Actin* and *PTEN* qPCR amplicon sets that were used to measure mRNA levels are shown below.

**Table 2:**

| **SEQ ID NO::** | **Gene** | **Species** | **Sequences** |
|---|---|---|---|
| 29 | ALDH2 (upper) | human, cynomolgus | 5' GGCAAGCCCTATGTCATCTCCT 3' |
| 30 | ALDH2 (lower) | | 5' GGATGGTTTTCCCGTGGTACTT 3' |
| 31 | ALDH2 (probe) | | 5' TGGTCCTCAAATGTCTCCGGTATTATGCC 3' |
| 32 | ALDH2 (upper) | mouse | 5' GGCAAGCCTTATGTCATCTCGT 3' |
| 33 | ALDH2 (lower) | | 5' GGAATGGTTTTCCCATGGTACTT 3' |
| 34 | ALDH2 (probe) | | 5' TGAAATGTCTCCGCTATTACGCTGGCTG 3' |
| 35 | ApoB (upper) | human | 5' TCATTCCTTCCCCAAAGAGACC 3' |
| 36 | ApoB (lower) | | 5' CACCTCCGTTTTGGTGGTAGAG 3' |
| 37 | ApoB (probe) | | 5' CAAGCTGCTCAGTGGAGGCAACACATTA 3' |
| 38 | ApoB (upper) | cynomolgus | 5' TCATTCCTTCCCCAAAGAAACC 3' |
| 39 | ApoB (lower) | | 5' CACCTCCGTTTTGGTGGTAGAG 3' |
| 40 | ApoB (probe) | | 5' TCAAGCTGTTAAGTGGCAGCAACACGTT 3' |
| 41 | beta-Actin (upper) | human | 5' GCATGGGTCAGAAGGATTCCTAT 3' |
| 42 | beta-Actin (lower) | | 5' TGTAGAAGGTGTGGTGCCAGATT 3' |
| 43 | beta-Actin (probe) | | 5' TCGAGCACGGCATCGTCACCAA 3' |
| 44 | beta-Actin (upper) | cynomolgus | 5' AAGGCCAACCGCGAGAAG 3' |
| 45 | beta-Actin (lower) | | 5' AGAGGCGTACAGGGACAGCA 3' |
| 46 | beta-Actin (probe) | | 5' TGAGACCTTCAACACCCCAGCCATGTAC 3' |
| 47 | beta-Actin (upper) | mouse | 5' CCTAAGGCCAACCGTGAAAAG 3' |
| 48 | beta-Actin (lower) | | 5' AGGCATACAGGGACAGCACAG 3' |
| 49 | beta-Actin (probe) | | 5' TGAGACCTTCAACACCCCAGCCATGTAC 3' |
| 50 | PTEN (upper) | human | 5' CACCGCCAAATTTAACTGCAGA 3' |
| 51 | PTEN (lower) | | 5' AAGGGTTTGATAAGTTCTAGCTGT 3' |
| 52 | PTEN (probe) | | 5' TGCACAGTATCCTTTTGAAGACCATAACCCA 3' |

Table 3: Non-conjugated derivatives of ALDH2-hcm2 with the indicated modification pattern are shown:

**Table 3**

| **Duplex ID** | **sequence and chemistry top: first strand, bottom: second strand, both 5'-3'** |
|---|---|
| ALD01 | mA(ps)fA(ps)mUfGmUfUmUfUmCfCmUfGmCfUmGfAmC(ps)fG(ps)mG |
| | fC(ps)mC(ps)fGmUfCmAfGmCfAmGfGmAfAmAfAmCfA(ps)mU(ps)fU |
| ALD02 | mA(ps)fA(ps)mUfGmUfUmUfUmCfCmUfGmCfUmGfAmCfGmG ivA |
| | fC(ps)mC(ps)fGmUfCmAfGmCfAmGfGmAfAmAfAmCfAmUfU ivA |
| ALD03 | mA(ps)fA(ps)mUfGmUfUmUfUmCfCmUfGmCfUmGfAmCfGmG ivU |
| | fC(ps)mC(ps)fGmUfCmAfGmCfAmGfGmAfAmAfAmCfAmUfU ivU |
| ALD04 | mA(ps)fA(ps)mUfGmUfUmUfUmCfCmUfGmCfUmGfAmCfGmG ivC |
| | fC(ps)mC(ps)fGmUfCmAfGmCfAmGfGmAfAmAfAmCfAmUfU ivC |
| ALD05 | mA(ps)fA(ps)mUfGmUfUmUfUmCfCmUfGmCfUmGfAmCfGmG ivG |
| | fC(ps)mC(ps)fGmUfCmAfGmCfAmGfGmAfAmAfAmCfAmUfU ivG |
| ALD06 | mA(ps)fA(ps)mUfGmUfUmUfUmCfCmUfGmCfUmGfAmCfG ivA |
| | fC(ps)mC(ps)fGmUfCmAfGmCfAmGfGmAfAmAfAmCfAmU ivA |
| ALD07 | mA(ps)fA(ps)mUfGmUfUmUfUmCfCmUfGmCfUmGfAmCfG ivU |
| | fC(ps)mC(ps)fGmUfCmAfGmCfAmGfGmAfAmAfAmCfAmU ivU |
| ALD08 | mA(ps)fA(ps)mUfGmUfUmUfUmCfCmUfGmCfUmGfAmCfG ivC |
| | fC(ps)mC(ps)fGmUfCmAfGmCfAmGfGmAfAmAfAmCfAmU ivC |
| ALD09 | mA(ps)fA(ps)mUfGmUfUmUfUmCfCmUfGmCfUmGfAmCfG ivG |
| | fC(ps)mC(ps)fGmUfCmAfGmCfAmGfGmAfAmAfAmCfAmU ivG |

| | |
|---|---|
| mA, mU, mC, mG - 2'-OMe RNA fA, fU, fC, fG - 2'-F RNA ivA, ivU, ivC, ivG - inverted RNA (3'-3') (ps) - phosphorothioate | |

Table 4: L6-GalNac linker conjugated derivatives of ALDH2-hcm2 with the indicated modification pattern are shown

**Table 4**

| **Duplex ID** | **sequence and chemistry top: first strand, bottom: second strand, both 5'-3'** |
|---|---|
| STS22002L6 | mA(ps)fA(ps)mUfGmUfUmUfUmCfCmUfGmCfUmGfAmC(ps)fG(ps)mG |
| | ST23(ps)3 ST 43(ps)fCmCfGmUfCmAfGmCfAmGfGmAfAmAfAmCfA(ps)mU(ps)fU |
| STS22002V1 L6 | mA(ps)fA(ps)mUfGmUfUmUfUmCfCmUfGmCfUmGfAmC(ps)fG(ps)mG |
| | ST23(ps)3 ST 43(ps)fCmCfGmUfCmAfGmCfAmGfGmAfAmAfAmCfAmUivA |
| STS22002V2L6 | mA(ps)fA(ps)mUfGmUfUmUfUmCfCmUfGmCfUmGfAmC(ps)fG(ps)mG |
| | ST23(ps)3 ST 43(ps)fCmCfGmUfCmAfGmCfAmGfGmAfAmAfAmCfAmUivG |
| STS22002V3L6 | mA(ps)fA(ps)mUfGmUfUmUfUmCfCmUfGmCfUmGfAmCfGmGivA |
| | ST23(ps)3 ST 43(ps)fCmCfGmUfCmAfGmCfAmGfGmAfAmAfAmCfA(ps)mU(ps)fU |
| STS22002V4L6 | mA(ps)fA(ps)mUfGmUfUmUfUmCfCmUfGmCfUmGfAmCfGmGivG |
| | ST23(ps)3 ST 43(ps)fCmCfGmUfCmAfGmCfAmGfGmAfAmAfAmCfA(ps)mU(ps)fU |

| | |
|---|---|
| mA, mU, mC, mG - 2'-OMe RNA fA, fU, fC, fG - 2'-F RNA ivA - inverted RNA (3'-3') (ps) - phosphorothioate | |

Table 5: L6-GalNac linker conjugated derivatives of ALDH2-hcm6 and ALDH2-hcm9 with the indicated modification pattern tested *in vivo* in mice are shown

**Table 5**

| **Duplex ID** | **sequence and chemistry top: first strand, bottom: second strand, both 5'-3'** |
|---|---|
| STS22006L6 | mU(ps)fC(ps)mUfUmCfUmUfAmAfAmCfUmGfAmGfUmU(ps)fU(ps)mC |
| | ST23(ps)3 ST 43(ps)fGmAfAmAfCmUfCmAfGmUfUmUfAmAfGmAfAmGfA |
| STS22006V1L6 | mU(ps)fC(ps)mUfUmCfUmUfAmAfAmCfUmGfAmGfUmU(ps)fU(ps)mC |
| | ST23(ps)3 ST 43(ps)mGmAmAmAmCmUfCfAfGmUmUmUmAmAmGmAmAmGivA |
| STS22009L6 | mA(ps)fU(ps)mGfUmAfGmCfCmGfAmGfGmAfUmCfUmU(ps)fC(ps)mU |
| | ST23(ps)3 ST 43(ps)fAmGfAmAfGmAfUmCfCmUfCmGfGmCfUmAfCmAfU |
| STS22009V1L6 | mA(ps)fU(ps)mGfUmAfGmCfCmGfAmGfGmAfUmCfUmU(ps)fC(ps)mU |
| | ST23(ps)3 ST 43(ps)mAmGmAmAmGmAfUfCfCmUmCmGmGmCmUmAmCmAivA |
| STS22009V2L6 | mA(ps)fU(ps)mGmUmAmGmCmCmGmAmGmGmAfUmCmUmU(ps)mC(ps)mU |
| | ST23(ps)3 ST 43(ps)mAmGmAmAmGmAfUfCfCmUmCmGmGmCmUmAmCmAivA |

| | |
|---|---|
| mA, mU, mC, mG - 2'-OMe RNA fA, fU, fC, fG - 2'-F RNA ivA - inverted RNA (3'-3') (ps) - phosphorothioate | |

### Example 3

Dose responses of ALDH2-siRNA molecules in human hepatocarinoma HepG2 cells were measured as follows.

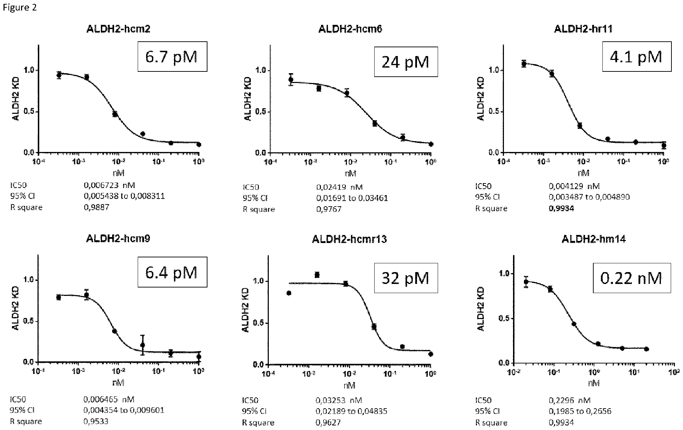

Cells were plated at a density of 150,000 cells per well in 6-well format. 24 hours post seeding, cells were transfected with indicated amounts of non-conjugated siRNAs using 1 µg/ml of AtuFECT as liposomal transfection reagent. 48 hours after transfection, RNA was isolated using the Spin Cell Mini Kit 250 (Stratec). *ALDH2* mRNA levels were determined by qRT-PCR relative to the geometric mean of *PTEN* and *ACTIN* mRNA expression as housekeepers. Values were normalized to the amount of *ALDH2* mRNA in untreated cells. Means and SD of normalized triplicate measurements are shown. IC₅₀ values, 95% confidence intervals and coefficients of determination are given. Results are shown in Figure 2.

### Example 4

*ALDH2* mRNA and protein levels and reduction of ALDH enzyme activity by ALDH2-hcm-2 siRNA in human HepG2-cells were investigated as follows.

Cells were seeded at 500,000 cells per well in 6-cm dishes. 24 hours post seeding, cells were transfected in triplicate with 1nM of ALDH2-hcm-2 siRNA or an siRNA against *firefly* luciferase as a non-targeting control (Fluc) using 1µg/ml of Atufect as liposomal transfection reagent. Three days post transfection, cells were lysed using InviTrap Spin Cell RNA Mini Kit (Stratec). ALDH2 mRNA levels were determined by qRT-PCR relative to actin expression as housekeeper. Mean and SD of normalized values are shown.

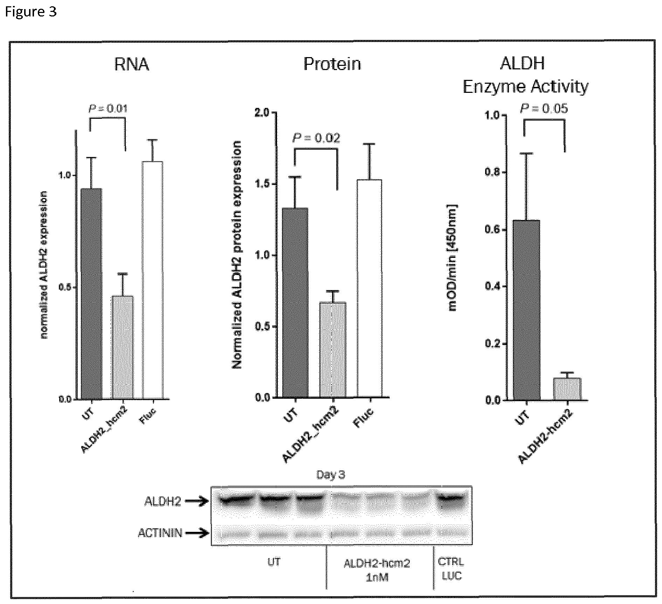

For Western Blotting, Cells were lysed in 500 µl lysis buffer (Tris/HCI, 15% Glycerin, 1% Triton). Protein amount was determined using the Pierce BCA Protein Assay Kit (Thermo Scientific), and 30 µg per sample were used for electrophoresis in Laemmli buffer. Membranes were probed with antibodies against ALDH2 (Abcam #194587, 1:1200) and alpha-ACTININ (NEB #3134, 1:1000). Quantification was performed Using a Stella Imaging System (Raytest). Aldehyde-Dehydrogenase activity assay was performed with total protein (12.2 micro g) extract using the Mitochondrial Aldehyde Dehydrogenase Activity Assay Kit (Abcam) according to manufacturer's protocol. Results are shown in Figure 3.

### Example 5

Dose-response for GalNAc- L1-linker-coupled siRNAs against *Aldh2* in primary mouse hepatocytes was measured as follows.

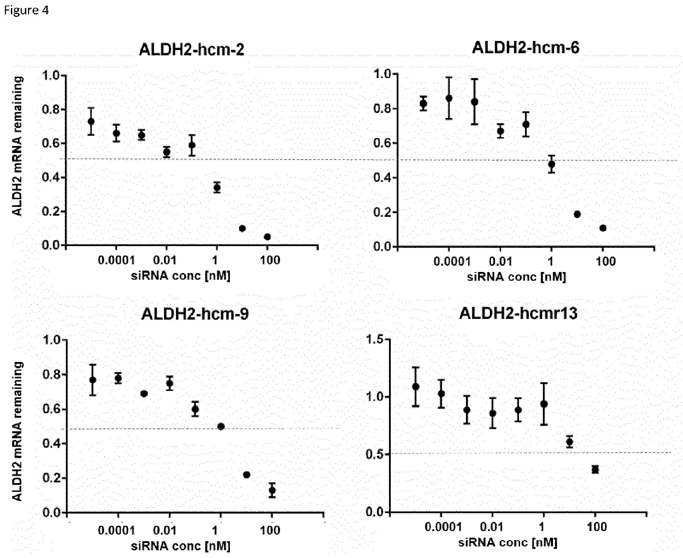

Mouse hepatocytes were plated at a density of 20.000 cells per well on collagen-coated 96-well plates (Life Technologies). 3 hours after plating, cells were rinsed once with PBS and L1-GalNAc-conjugated siRNAs were added at the indicated concentrations in triplicates. 24 hours post treatment, cells were lysed with InviTrap Spin Cell RNA Mini Kit (Stratec). *Aldh2* mRNA levels were determined by qRT-PCR relative to *Actin* mRNA expression as housekeeper. Values were normalized to the amount of *Aldh2* in untreated cells. Means and SD of normalized triplicate values are shown. Results are shown in Figure 4.

### Example 6

Dose-response for GalNAc- L1-linker-coupled siRNAs against ALDH2 in primary human hepatocytes was measured as follows.

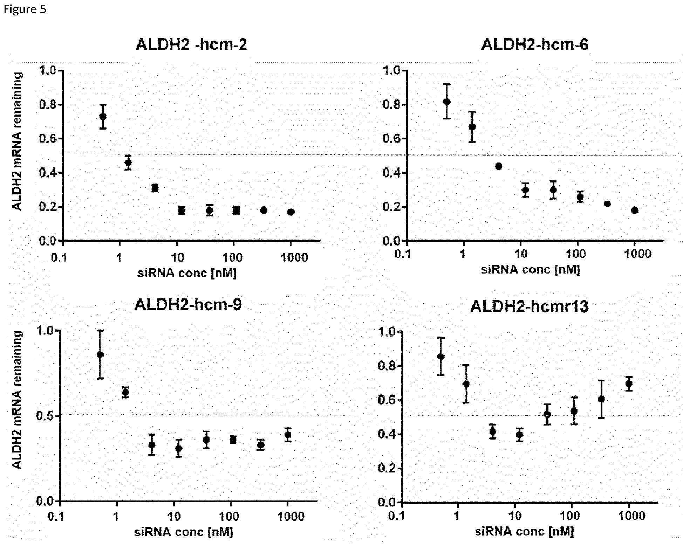

Human hepatocytes (Thermo Fisher) were plated at a density of 30.000 cells per well on collagen-coated 96-well plates (Life Technologies). GalNAc-L1 linker conjugated siRNAs were added at the indicated concentrations in triplicates immediately after plating. 24 hours post treatment, cells were lysed with InviTrap Spin Cell RNA Mini Kit (Stratec). ALDH2 mRNA levels were determined by qRT-PCR relative to *ACTIN* mRNA expression as housekeeper. Values were normalized to the amount of *ALDH2* in untreated cells. Means and SD of normalized triplicate values are shown. Results are shown in Figure 5.

### Example 7

Reduction of *ALDH2-mRNA* by 15 nM of GalNAc-L1 linker-coupled ALDH2-hcm-2 siRNA in primary hepatocytes of different species indicated 6 days post treatment was measured as follows.

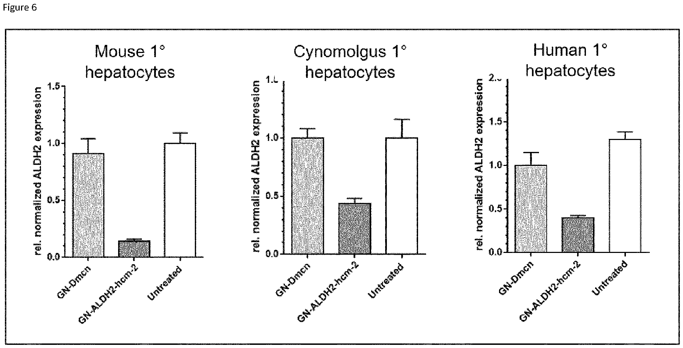

Hepatocytes (Thermo Fisher) were plated at a density of 250.000 (human and cynomolgus) or 150.000 (mouse) cells per well on collagen-coated 24-well plates (Life Technologies). L1-GalNAc conjugated ALDH2-hcm-2 or non-silencing control siRNA were added at 15 nM concentration in triplicates immediately after plating. 6 days post treatment, cells were lysed with InviTrap Spin Cell RNA Mini Kit (Stratec). *ALDH2* mRNA levels of the different species were determined by qRT-PCR relative to geometric means of *ACTIN* and *APOB* expression (human and cynomolgus) or to Actin alone (mouse) as housekeeper. Means and SD of normalized triplicate values are shown. Results are shown in Figure 6.

### Example 8

Comparison of *ALDH2* mRNA knockdown by GalNAc-conjugated ALDH2 siRNAs with linkers L1 and L6 in human primary hepatocytes was measured as follows.

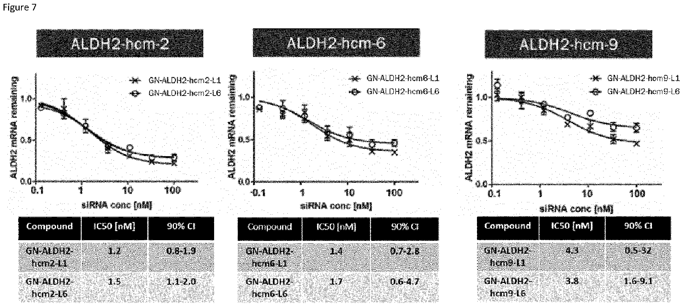

Human hepatocytes (Thermo Fisher) were seeded at 30.000 cells per well in collagen-coated 96-well plates (Life Technologies). Immediately after seeding, GalNac-conjugated siRNAs were added as indicated in graphs. 24 hours post treatment, cells were lysed with with InviTrap Spin Cell RNA Mini Kit (Stratec). *ALDH2* mRNA levels were determined by qRT-PCR relative to geometric means of *ACTIN* and *APOB* expression as housekeepers. Values were normalized to the amount of *ALDH2* in untreated cells. Means and SD of normalized triplicate values are shown. IC50 values and 90% confidence intervals were calculated. Results are shown in Figure 7.

### Example 9

Activity of GalNAc-siRNAs against ALDH2 in vivo was measured as follows.

Male mice (C57BI/6J) were treated subcutaneously with 3 or 10 mg/kg of the indicated compounds (6 animals per group). At necropsy 10 days post treatment, liver was harvested. Tissue was homogenized in a Tissue Lyser II machine (Qiagen).

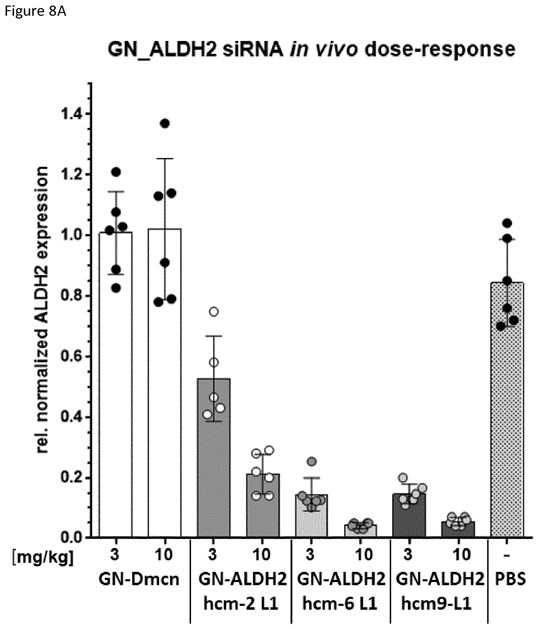

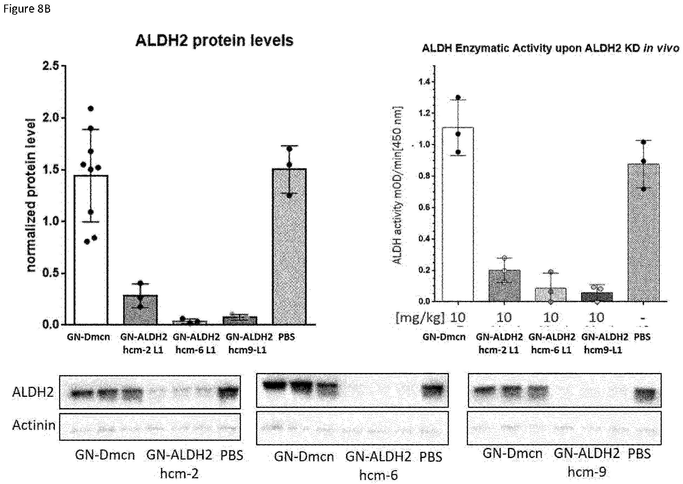

A -RNA was extracted from 10 mg of liver tissue using an InviTrap Spin Tissue RNA Mini Kit (Stratec). *Aldh2* mRNA levels were determined by qRT-PCR relative to the geometric means of *Actin* and *ApoB* (mouse) as housekeepers. Values were normalized to the amount of *Aldh2* in mice treated with the negative control siRNA (GN-Dmcn). Means and SD of normalized values are shown as well as values for individual animals. Measurements of untreated control mice (PBS) is shown. Results are shown in Figure 8A. B - Protein was extracted from 10 mg of liver tissue in RIPA buffer (Cell Signaling) from three animals treated with 10 mg/kg of the indicated compounds per group. Protein amount was determined using the Pierce BCA Protein Assay Kit (Thermo Scientific), and 30 µg per sample were used for Electrophoresis in Laemmli buffer. Membranes were probed with antibodies against ALDH2 (Abcam #194587, 1:1200) and alpha-ACTININ (NEB #3134, 1:1000) and developed with HRP secondary antbody. Visualizations of the membranes are shown in the lower panel. Quantification (upper left panel) was performed Using a Stella Imaging System (Raytest). Aldehyde-Dehydrogenase activity assay (upper right panel) was performed using liver protein extracts of three mice treated with the indicated siRNAs or PBS using the Mitochondrial Aldehyde Dehydrogenase Activity Assay Kit (Abcam) according to manufacturer's protocol. Results are shown in Figure 8B.

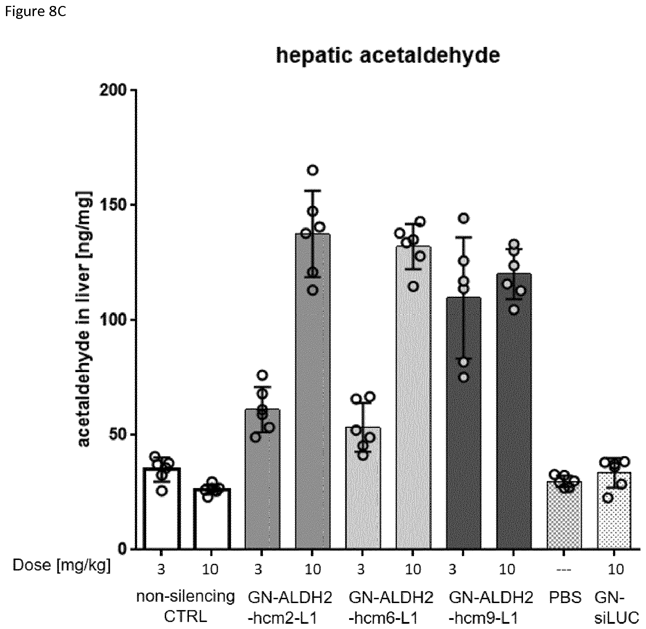

C - Livers from mice untreated (PBS) or treated with the indicated GalNAc-siRNAs (n=6) at the indicated doses for 10 days were analysed for its amount of hepatic acetaldehyde. Acetaldehyde content was measured in whole liver tissue homogenates (100 mg) in aqueous solution using an Agilent gas chromatography (GC) 6890 and 7890 series system coupled with a 5975 C MS detector (Agilent), MSD Chemstation (Agilent) and Maestro software for data analyses. Internal standard was 2-propanol and the following settings were applied: Incubation: 70°C for 30 min; Syringe temp.: 80°C; Injector temp.: 200°C; Injection mode: SHS, Split 1/5; Injection volume: 1 ml (gas syringe); Column: DB-WAX MS, 30m x 0.25 mm; 1µm ; Helium Flow: 1 ml/min; Oven : 37°C for 5 min then 30°C/min to 120°C for 1 min; MS Transfer Line: 240°C; Incubation solvent: Purified Water; MS detection: EI - Scan from 10 to 300 uma. Individual measurements as well as mean +/-SD are depicted. Results are shown in Figure 8C.

### Example 10

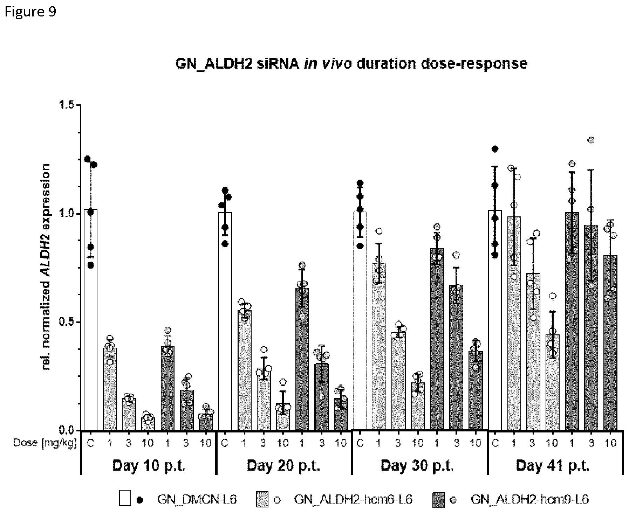

Activity of GalNAc-siRNAs against *Aldh2 in vivo* - duration and dose-response. Male mice (C57BI/6J) were treated once subcutaneously with 1, 3 or 10 mg/kg of the indicated compounds (5 animals per group). At necropsy at 10, 20, 30, and 41 days post treatment, liver was harvested. Tissue was homogenized in a Tissue Lyser II machine (Qiagen). Total RNA was extracted from 10 mg of liver tissue using an InviTrap Spin Tissue RNA Mini Kit (Stratec). *Aldh2* mRNA levels were determined by qRT-PCR relative to the geometric means of Actin and ApoB expression (mouse) as housekeepers. Values were normalized to the amount of *Aldh2* in mice treated with the non-silencing control siRNA. Means and SD of normalized values are shown as well as values for individual animals. Results are shown in Figure 9.

### Example 11

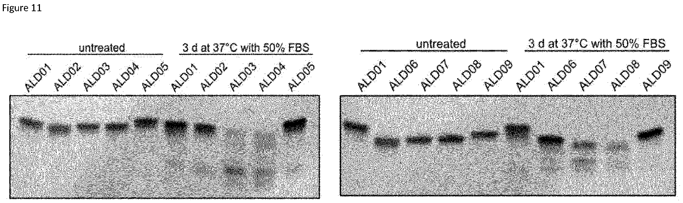

Different siRNA duplexes containing inverted RNA nucleotides at both 3'-ends were tested for serum stability. ALD02-ALD05 (Table 3) contain inverted RNA in addition to the last nucleotide in the first strand and second strand. ALD06-ALD09 contain inverted RNA instead of the last nucleotide in first strand and second strand. All inverted RNA nucleotides substitute for terminally used phosphorothioates. In both designs, ivA and ivG confer higher stability to the tested sequence than ivU and ivC. "UT" indicates untreated samples. "FBS" indicates siRNA duplexes which were incubated at 37 °C at 5 µM concentration with 50 % FBS for 3 days, before being phenol/chloroform-extracted and precipitated with Ethanol. Samples were analyzed on 20% TBE polyacrylamide gels in native gel electrophoresis. UV pictures of the ethidium bromide-stained gels are shown in Figure 11.

### Example 12:

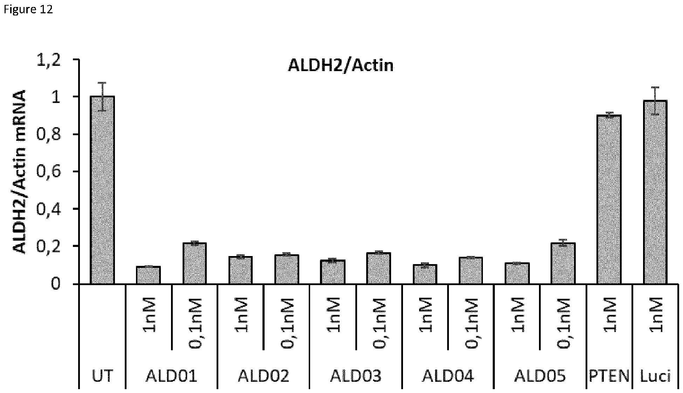

The influence of inverted A, U, C and G RNA nucleotides at 3'-overhang positions on the efficacy of *ALDH2* mRNA knockdown was analyzed using derivatives of the siRNA ALDH2-hcm2 (see Table 3). ALD01 contains phosphorothioates at all termini, whereas ALD02-ALD05 contain ivA (ALD01), ivU (ALD03), ivC (ALD04) and ivG (ALD05) at the 3'-end of the first strand and at the 3'-end of the second strand. Both inverted nucleotides are present in addition to the terminal nucleotide of the respective strands and substitute for terminal phosphorothioates. A non-related siRNA *(PTEN)* and a control siRNA targeting firefly luciferase (Luci) were included. All tested variants show comparable activity under the tested conditions. The experiment was conducted in the human hepatocarcinoma cell line Hep3B. Cells were seeded at a density of 150,000 cells per 6-well, transfected with 0.1 nM and 1 nM siRNA, respectively, using 1 µg/ml Atufect as a liposomal transfection reagent after 24 h. Cells were lysed after 48 h. Total RNA was extracted and *ALDH2* and *ACTIN* mRNA levels were determined by Taqman qRT-PCR. Each bar represents mean ± SD of three technical replicates normalized to the house keeping gene expression (*ACTIN*). Results are shown in Figure 12.

### Example 13

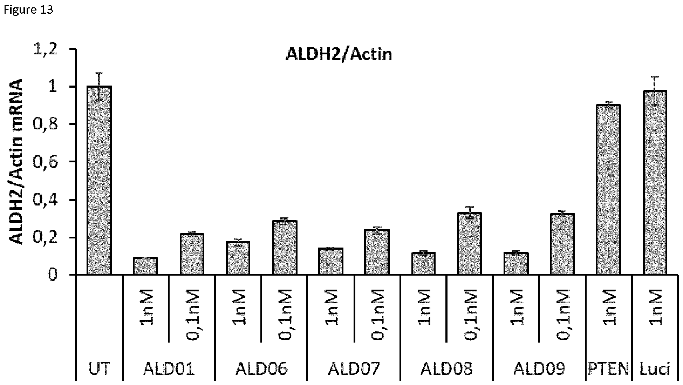

The influence of inverted A, U, C and G RNA nucleotides at terminal 3' positions on the efficacy of *ALDH2* mRNA knockdown was analyzed using derivatives of the siRNA ALDH2-hcm2 (see Table 3). ALD01 contains phosphorothioates at all termini, whereas ALD06-ALD09 contain ivA (ALD06), ivU (ALD07), ivC (ALD08) and ivG (ALD09) at the 3'- end of the first strand and at the 3'-end of the second strand. Both inverted nucleotides replace the terminal nucleotide of the respective strands and substitute for terminal phosphorothioates used in other derivatives for stabilization. A non-related siRNA (*PTEN*) and a control siRNA targeting firefly luciferase (Luci) were included. All tested variants show comparable activity under the tested conditions. The experiment was conducted in the human hepatocarcinoma cell line Hep3B. Cells were seeded at a density of 150,000 cells per 6-well, transfected with 0.1 nM and 1 nM siRNA, respectively, using and 1 µg/ml Atufect as a liposomal transfection reagent after 24 h. Cells were lysed after 48 h. Total RNA was extracted and *ALDH2* and *ACTIN* mRNA levels were determined by Taqman qRT-PCR. Each bar represents mean ± SD of three technical replicates normalized to the house keeping gene expression (*ACTIN*). Results are shown in Figure 13.

### Example 14

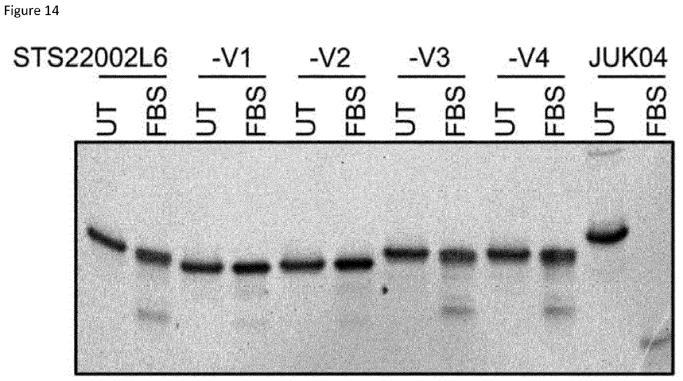

Different GalNAc-L6-linker conjugates of ALDH2-hcm2 containing inverted RNA nucleotides (see Table 4) were tested for serum stability. STS22002L6 contains phosphorothioates at all non-conjugated ends, whereas STS22002V1-L6 and STS22002V2-L6 contain inverted RNA nucleotides at the second strand's 3'-end, where the respective inverted nucleotide is present instead of the last nucleotide. STS22002V3-L6 and STS22002V4-L6 contain inverted RNA nucleotides at the first strand's 3'-end, whereby the respective inverted nucleotide is present in addition to the last nucleotide. ivA was used in STS22002V1-L6 and STS22002V3-L6, whereas ivG was used in STS22002V2-L6 and STS22002V4-L6. All inverted RNA nucleotides substitute for terminal phosphorothioates used in other derivatives for stabilization. STS22002V1-L6 and STS22002V2-L6 are slightly more stable than the other variants tested here. "UT" indicates untreated samples. JUK04 represents a control compound not serum stabilized as a positive control. "FBS" indicates GalNAc-siRNA conjugates which were incubated at 37 °C at 5 µM concentration with 50 % FBS for 3 days before being phenol/chloroform-extracted and precipitated with Ethanol. Samples were analyzed on 20% TBE polyacrylamide gels in native gel electrophoresis. UV pictures of the ethidium bromide-stained gels are shown in Figure 14.

### Example 15

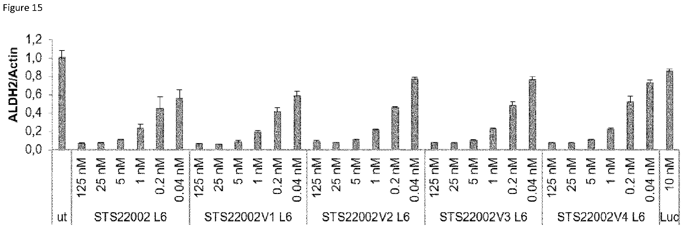

The dose response of inverted RNA nucleotides at terminal 3' positions on the efficacy of *ALDH2* mRNA knockdown was analyzed using GalNac-L6-conjugated derivatives of the siRNA ALDH2-hcm2 (see Table 4) by ASGPR receptor complex-mediated uptake in mouse primary hepatocytes. STS22002L6 contains phosphorothioates at all non-conjugated ends, whereas STS22002V1-L6 and STS22002V2-L6 contain inverted RNA nucleotides at the second strand's 3'-end, where the respective inverted nucleotide is present instead of the last nucleotide. STS22002V3-L6 and STS22002V4-L6 contain inverted RNA nucleotides at the first strand's 3'-end, whereby the nucleotide is present in addition to the last nucleotide. ivA was used in STS22002V1-L6 and STS22002V3-L6, whereas ivG was used in STS22002V2-L6 and STS22002V4-L6. All inverted RNA nucleotides substitute for terminal phosphorothioates used in other derivatives for stabilization. All tested variants show comparable activity. The experiment was conducted in mouse primary hepatocytes. Cells were seeded at a density of 20,000 cells per 96-well, treated with the indicated doses from 125 nM to 0.04 nM of the respective indicated siRNA conjugates directly after plating. Non-treated cells and a control siRNA targeting firefly luciferase at 10 nM (Luc) were included. Cells were lysed after 24 h. Total RNA was extracted and *Aldh2* and *Actin* mRNA levels were determined by Taqman qRT-PCR. Each bar represents mean ± SD of three biological replicates normalized to the house keeping gene expression (*Actin*). Results are shown in Figure 15.

### Example 16

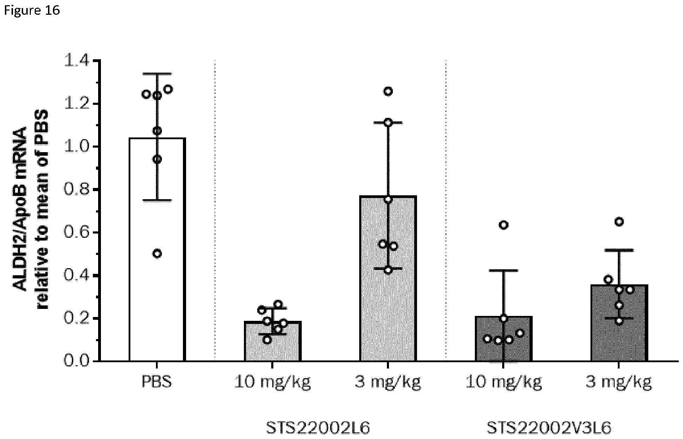

The influence of ivA at the first strand's 3'-end on the efficacy of *ALDH2* mRNA knockdown was analyzed *in vivo.* Therefore, GalNAc-L6 derivatives of ALDH2-hcm6 and ALDH2-hcm9 siRNA conjugates targeting *Aldh2* were used in an in vivo efficacy study. STS22002L6 contains phosphorothioates at all non-conjugated termini, whereas STS22002V3-L6 contains an ivA at the first strand's 3'-end in addition to the last nucleotide. C57BL/6 male mice (n=6) were treated subcutaneously with vehicle (PBS), 10 mg/kg and 3 mg/kg of the indicated GalNAc-L6 conjugate, respectively. Seven days after treatment, at necropsy liver tissue was harvested and total RNA was extracted. *Aldh2* and *ApoB* mRNA levels were analyzed by Taqman qRT-PCR. Each bar represents mean normalized *(ApoB)* expression ± SD of six animals. Individual values normalized to the house keeping gene expression *(ApoB)* are plotted, additionally. Results are shown in Figure 16.

### Example 17

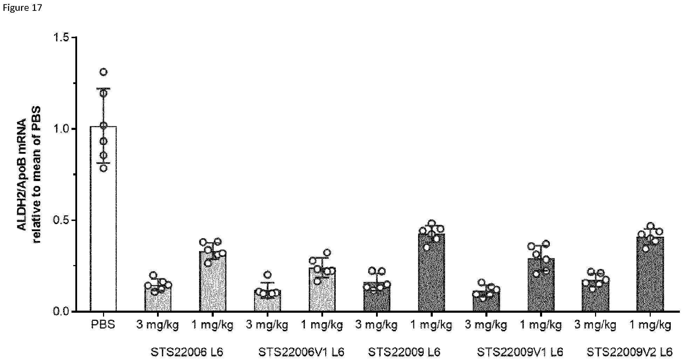

Different modified derivatives of the GalNAc-siRNA conjugates of ALDH2-hcm6 and ALDH2-hcm9 (Table 5) were analyzed for knockdown efficacy on *ALDH2* mRNA expression levels *in vivo* in mice. "V1" variants contain a different 2'-OMe/2'-F modification pattern in the second strand and an ivA nucleotide at the 3'-end of the second strand, substituting for the last nucleotide and for terminal phosphorothioates at this end. "V2" variants additionally contain a different 2'-OMe/2'-F modification pattern in the first strand as specified in Table 5. C57BL/6 male mice (n=6) were subcutaneously treated with vehicle (PBS) or 3 mg/kg and 1 mg/kg GalNAc-L6 conjugate derivatives, respectively. Nine days after treatment, at necropsy liver tissue was harvested and total RNA was extracted. *Aldh2* and *ApoB* mRNA levels were analyzed by Taqman qRT-PCR. Each bar represents mean normalized *(ApoB)* expression ± SD of six animals. Individual values normalized to the house keeping gene expression *(ApoB)* are plotted, additionally. Statistical analysis is based on Kruskal-Wallis test with Dunn's multiple comparisons test respective to the control group (PBS). Results are shown in Figure 17.

### Statements of Invention

1. A nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of RNA transcribed from the *ALDH2* gene, wherein said first strand comprises a nucleotide sequence selected from the following sequences: SEQ ID NOs: 11, 1, 3, 5, 7, 9, 13, 15, 17, 19, 21, 23, 25 or 27.
2. A nucleic acid of statement 1, wherein the second strand comprises a nucleotide sequence of SEQ ID NO: 12, 2, 4, 6, 8, 10, 14, 16, 18, 20, 22, 24, 26 or 28.
3. A nucleic acid of statement 1 or statement 2, wherein said first strand comprises a nucleotide sequence of SEQ ID NO:11, SEQ ID NO:3 or SEQ ID NO:17.
4. A nucleic acid of any one of statements 1 to 3, wherein said second strand comprises the nucleotide sequence of SEQ ID NO:12, SEQ ID NO:4 or SEQ ID NO:18.
5. A nucleic acid according to any one of statements 1 to 4, wherein said first strand and/or said second strand are each from 17-35 nucleotides in length.
6. A nucleic acid of any one of statements 1 to 5, wherein the at least one duplex region consists of 19-25 consecutive nucleotide base pairs.
7. A nucleic acid of any preceding statement, which
a) is blunt ended at both ends; or
b) has an overhang at one end and a blunt end at the other; or
c) has an overhang at both ends.
8. A nucleic acid according to any preceding statement, wherein one or more nucleotides on the first and / or second strand are modified, to form modified nucleotides.
9. A nucleic acid of statement 8, wherein one or more of the odd numbered nucleotides of the first strand are modified.
10. A nucleic acid according to statement 9, wherein one or more of the even numbered nucleotides of the first strand are modified by at least a second modification, wherein the at least second modification is different from the modification of statement 9.
11. A nucleic acid of statement 10, wherein at least one of the one or more modified even numbered nucleotides is adjacent to at least one of the one or more modified odd numbered nucleotides.
12. A nucleic acid of any one of statements 9 to 11, wherein a plurality of odd numbered nucleotides are modified.
13. A nucleic acid of statement 10 to 12, wherein a plurality of even numbered nucleotides are modified by a second modification.
14. A nucleic acid of any of statements 8 to 13, wherein the first strand comprises adjacent nucleotides that are modified by a common modification.
15. A nucleic acid of any of statements 9 to 14, wherein the first strand comprises adjacent nucleotides that are modified by a second modification that is different to the modification of statement 9.
16. A nucleic acid of any of statements 9 to 15, wherein one or more of the odd numbered nucleotides of the second strand are modified by a modification that is different to the modification of statement 9.
17. A nucleic acid according to any of statements 9 to 15, wherein one or more of the even numbered nucleotides of the second strand are modified by the modification of statement 9.
18. A nucleic acid of statement 16 or 17, wherein at least one of the one or more modified even numbered nucleotides of the second strand is adjacent to the one or more modified odd numbered nucleotides of the second strand.
19. A nucleic acid of any of statements 16 to 18, wherein a plurality of odd numbered nucleotides of the second strand are modified by a common modification.
20. A nucleic acid of any of statements 16 to 19, wherein a plurality of even numbered nucleotides are modified by a modification according to statement 9.
21. A nucleic acid of any of statements 16 to 20, wherein a plurality of odd numbered nucleotides on the second strand are modified by a second modification, wherein the second modification is different from the modification of statement 9.
22. A nucleic acid of any of statements 16 to 21, wherein the second strand comprises adjacent nucleotides that are modified by a common modification.
23. A nucleic acid of any of statements 16 to 22, wherein the second strand comprises adjacent nucleotides that are modified by a second modification that is different from the modification of statement 9.
24. A nucleic acid according to any one of statements 8 to 23, wherein each of the odd numbered nucleotides in the first strand and each of the even numbered nucleotides in the second strand are modified with a common modification.
25. A nucleic acid of statement 24, wherein each of the even numbered nucleotides are modified in the first strand with a second modification and each of the odd numbered nucleotides are modified in the second strand with a second modification.
26. A nucleic acid according to any one of statements 8 to 25, wherein the modified nucleotides of the first strand are shifted by at least one nucleotide relative to the unmodified or differently modified nucleotides of the second strand.
27. A nucleic acid according to any one of statements 8 to 26, wherein the modification and/or modifications are each and individually selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification.
28. A nucleic acid according to any one of statements 8 to 27, wherein the modification is any one of a locked nucleotide, an abasic nucleotide or a non-natural base comprising nucleotide.
29. A nucleic acid according to any one of statements 8 to 28, wherein at least one modification is 2'-O-methyl.
30. A nucleic acid according to any one of statements 8 to 29, wherein at least one modification is 2'-F.
31. A nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of a RNA transcribed from the *ALDH2* gene, wherein said first strand comprises a nucleotide sequence selected from the following sequences: SEQ ID NOs: 11, 1, 3, 5, 7, 9, 13, 15, 17, 19, 21, 23, 25 or 27, wherein the nucleotides of first strand are modified by first modification on the odd numbered nucleotides, and modified by a second modification on the even numbered nucleotides, and nucleotides of the second strand are modified by a third modification on the even numbered nucleotides and modified by a fourth modification on the odd numbered nucleotides, wherein at least the first modification is different to the second modification and the third modification is different to the fourth modification.
32. A nucleic acid of statement 31, wherein second sequence comprises a nucleotide sequence of SEQ ID NO: 12, 2, 4, 6, 8, 10, 14, 16, 18, 20, 22, 24, 26 or 28.
33. A nucleic acid of statement 31 or 32, wherein the fourth modification and the second modification are the same.
34. A nucleic acid of any one of statements 31 to 33, wherein the first modification and the third modification are the same.
35. A nucleic acid of any one of statements 31 to 34, wherein the first modification is 2'O-Me and the second modification is 2'F.
36. A nucleic acid of any one of statements 31 to 35, wherein the first strand comprises the nucleotide sequence of SEQ ID NO:11, SEQ ID NO:3 or SEQ ID NO:17 and the second strand comprises the nucleotide sequence of SEQ ID NO:12, SEQ ID NO:4 or SEQ ID NO:18.
37. A nucleic acid of any one of statements 31 to 36, comprising a sequence and modifications as shown below:

| **Sequence ID No** | **siRNA ID** | **sequence** | **modifications** |
|---|---|---|---|
| 3 | ALDH2-hcm-2 | 5'-AAUGUUUUCCUGCUGACGG-3' | 6254515173547182748 |
| 4 | | 5'-CCGUCAGCAGGAAAACAUU-3' | 3745364728462627251 |
| 11 | ALDH2-hcm-6 | 5'-UCUUCUUAAACUGAGUUUC-3' | 5351715262718281517 |
| 12 | | 5'-GAAACUCAGUUUAAGAAGA-3' | 4626353645152646282 |
| 17 | ALDH2-hcm-9 | 5'-AUGUAGCCGAGGAUCUUCU-3' | 6181647382846171535 |
| 18 | | 5'-AGAAGAUCCUCGGCUACAU-3' | 2826461735384716361 |

wherein, the specific modifications are depicted by numbers
1=2'F-dU,
2=2'F-dA,
3=2'F-dC,
4=2'F-dG,
5=2'-OMe-rU;
6=2'-OMe-rA;
7=2'-OMe-rC;
8=2'-OMe-rG.
38. A nucleic acid according to any one of statements 1 to 37, conjugated to a ligand.
39. A nucleic acid according to any one of statements 1 to 38, comprising a phosphorothioate linkage between the terminal one, two or three 3' nucleotides and/or 5' nucleotides of the first and/or the second strand.
40. A nucleic acid according to any one of statements 1 to 39 comprising two phosphorothioate linkage between each of the three terminal 3' and between each of the three terminal 5' nucleotides on the first strand, and two phosphorothioate linkages between the three terminal nucleotides of the 3' end of the second strand.
41. A nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of a RNA transcribed from the *ALDH2* gene, wherein said first strand comprises a nucleotide sequence selected from the following sequences: SEQ ID NOs: 11, 1, 3, 5, 7, 9, 13, 15, 17, 19, 21, 23, 25 or 27, and wherein the nucleic acid is conjugated to a ligand.
42. A nucleic acid according to statement 41, wherein the ligand comprises (i) one or more N-acetyl galactosamine (GalNac) moieties and derivatives thereof, and (ii) a linker, wherein the linker conjugates the GalNac moieties to a nucleic acid as defined in statement 41.
43. A nucleic acid according to any of statements 41 or 42, wherein linker may be a bivalent or trivalent or tetravalent branched structure.
44. A nucleic acid of any of statements 41 to 43, wherein the nucleotides are modified as defined in any preceding statements.
45. A nucleic acid of any preceding statement, which is conjugated to a ligand comprising the formula I:

[S-X¹-P-X²]₃-A-X³- (I)

wherein:
S represents a saccharide, wherein the saccharide is N-acetyl galactosamine;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is alkylene or an alkylene ether of the formula (-CH₂)ₙ-O-CH₂- where n = 1- 6;
A is a branching unit;
X³ represents a bridging unit;
wherein a nucleic acid as defined in any of statements 1 to 40 is conjugated to X³ via a phosphate or modified phosphate (preferably a thiophosphate).
46. A conjugated nucleic acid having one of the following structures 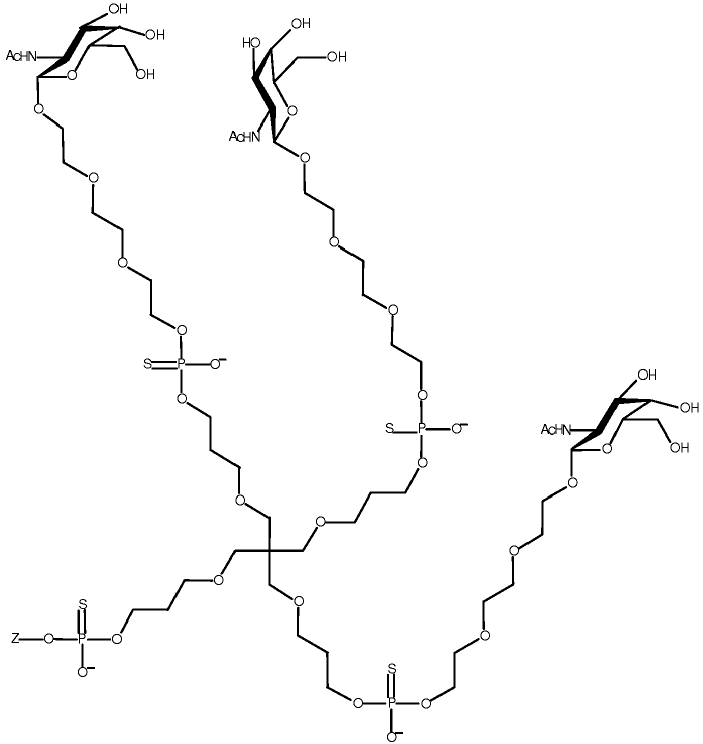 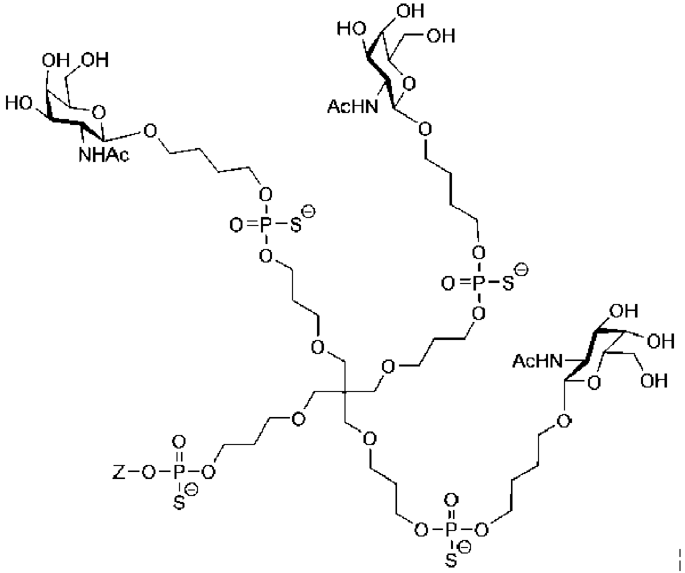 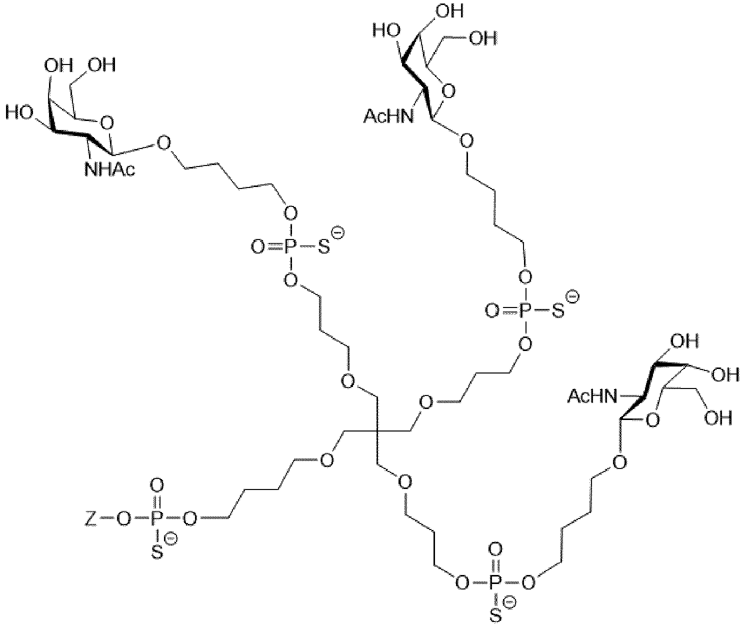 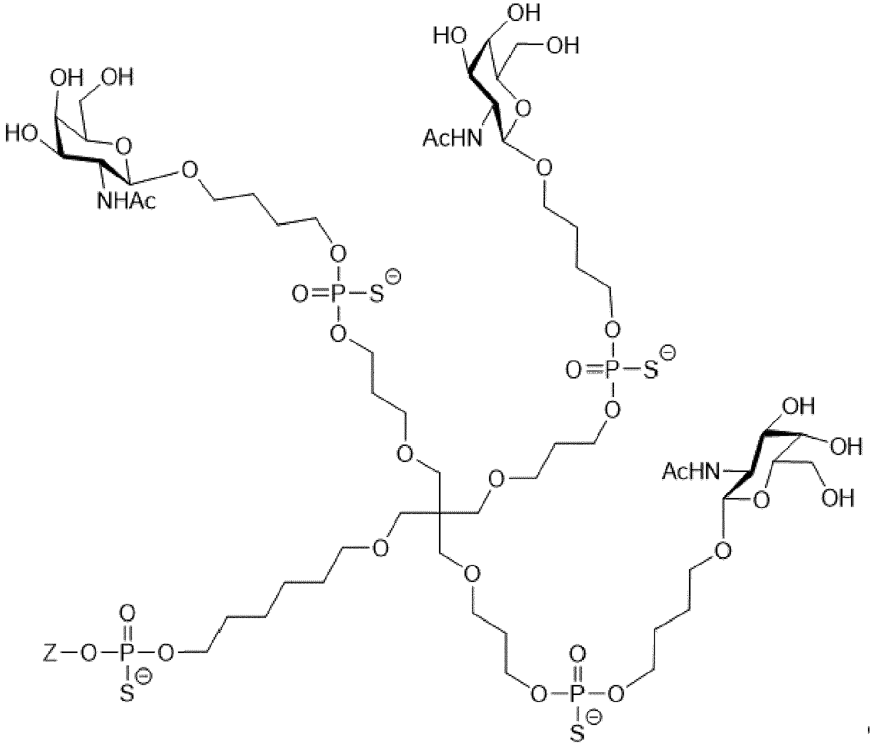 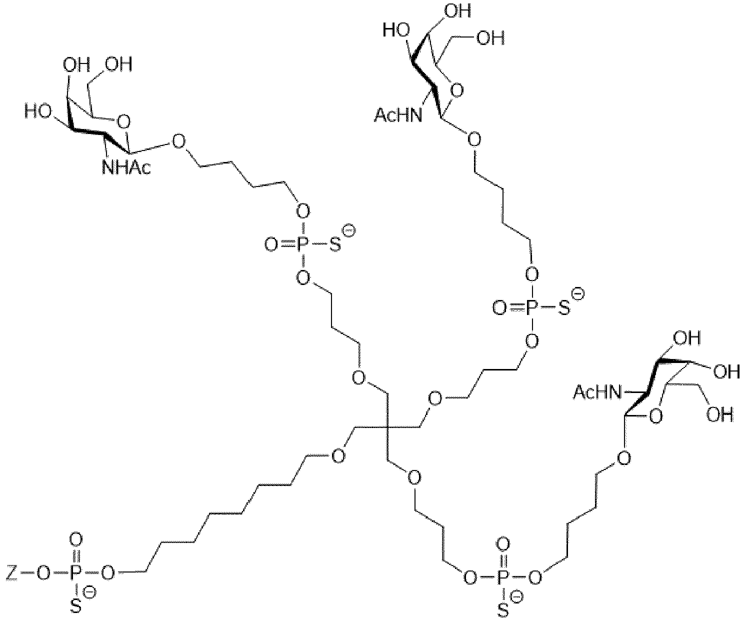 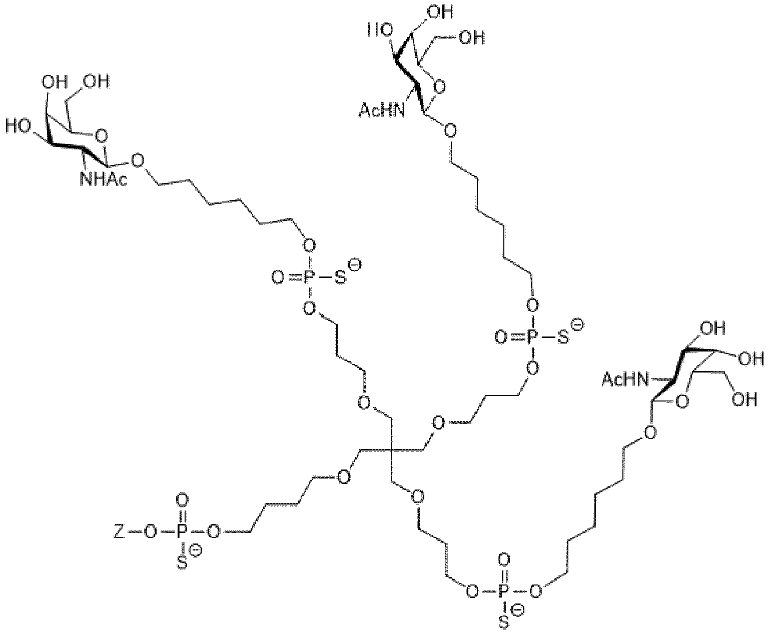 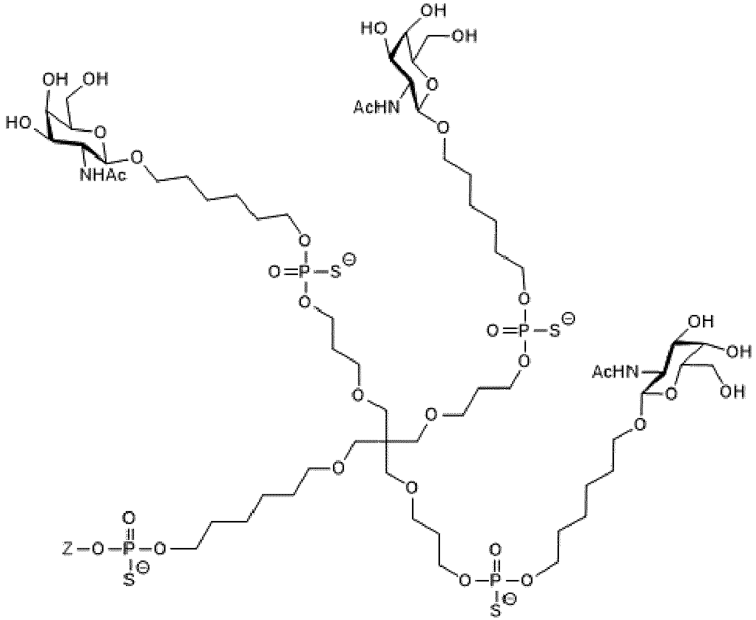 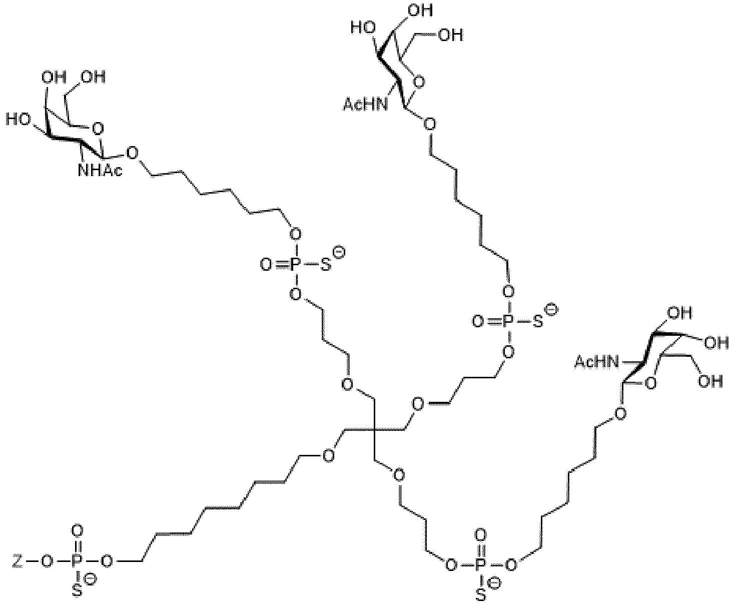 wherein Z is a nucleic acid according to any of statements 1 to 40.
47. A nucleic acid according to any of statements 1 to 40, which is conjugated to a ligand of the following structure 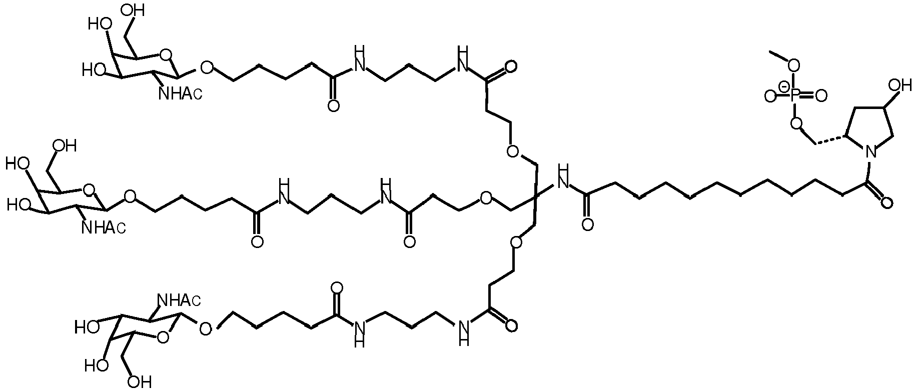 48. A nucleic acid or conjugated nucleic acid of any preceding statement, wherein the duplex comprises separate strands.
49. A nucleic acid or conjugated nucleic acid of any preceding statement, wherein the duplex comprises a single strand comprising a first strand and a second strand.
50. A composition comprising a nucleic acid or conjugated nucleic acid of any preceding statement and a physiologically acceptable excipient.
51. A nucleic acid or conjugated nucleic acid according to any preceding statement for use in the prevention or treatment of a disease, disorder or syndrome.
52. Use of a nucleic acid or conjugated nucleic acid according to any preceding statement in the manufacture of a medicament for preventing or treating a disease, disorder or syndrome.
53. A method of preventing or treating a disease, disorder or syndrome comprising administration of a composition comprising a nucleic acid or conjugated nucleic acid according to any preceding statement to an individual in need of treatment.
54. The method of statement 54, wherein the nucleic acid or conjugated nucleic acid is administered to the subject subcutaneously, intravenously or using any other application routes such as oral, rectal or intraperitoneal.
55. A use or method according to statements 51 to 54, wherein said disease or disorder is alcohol use disorder.
56. A use or method according to statement 55, wherein the alcohol use disorder is acute alcohol sensitivity, alcoholic neuropathy, alcohol abuse or fetal alcohol disorder or other pathologies associated to acute or prolonged excessive alcohol consumption.
57. A process for making a nucleic acid or conjugated nucleic acid of any one of statements 1 to 49.
58. A nucleic acid for inhibiting expression of *ALDH2,* comprising at least one duplex region that comprises at least a portion of a first strand and at least a portion of a second strand that is at least partially complementary to the first strand, wherein said first strand is at least partially complementary to at least a portion of RNA transcribed from the *ALDH2* gene, wherein the expression of *ALDH2* is reduced to levels which are at least 15% lower than expression levels observed in same test conditions but in the absence of the nucleic acid or conjugated nucleic acid or in the presence of a non-silencing control.
59. A nucleic acid for inhibiting expression of *ALDH2,* comprising at least one duplex region that comprises at least a portion of a first strand and at least a portion of a second strand that is at least partially complementary to the first strand, wherein said first strand is at least partially complementary to at least a portion of a RNA transcribed from the *ALDH2* gene, wherein said first strand comprises a nucleotide sequence selected from the following sequences: SEQ ID NOs: 11, 1, 3, 5, 7, 9, 13, 15, 17, 19, 21, 23, 25 or 27, wherein the expression of *ALDH2* is reduced to levels which are at least 15% lower than expression levels observed in same test conditions but in the absence of the nucleic acid or conjugated nucleic acid or in the presence of a non-silencing control.

## Claims

1. A nucleic acid for inhibiting expression of *ALDH2* in a cell, comprising at least one duplex region that comprises at least a portion of a first strand and at least a portion of a second strand that is at least partially complementary to the first strand, wherein said first strand is at least partially complementary to at least a portion of RNA transcribed from the *ALDH2* gene, wherein said first strand comprises a nucleotide sequence selected from the following sequences: SEQ ID NOs: 11, 1, 3, 5, 7, 9, 13, 15, 17, 19, 21, 23, 25 or 27.

2. A nucleic acid of claim 1, wherein the second strand comprises a nucleotide sequence of SEQ ID NO: 12, 2, 4, 6, 8, 10, 14, 16, 18, 20, 22, 24, 26 or 28.

3. A nucleic acid according to claims 1 or 2, wherein one or more nucleotides on the first and / or second strand are modified, to form modified nucleotides.

4. A nucleic acid of claim 3, wherein one or more of the odd numbered nucleotides of the first strand are modified.

5. A nucleic acid according to claim 4, wherein one or more of the even numbered nucleotides of the first strand are modified by at least a second modification, wherein the at least second modification is different from the modification of claim 4.

6. A nucleic acid of any of claims 4 to 5, wherein one or more of the odd numbered nucleotides of the second strand are modified by a modification that is different to the modification of claim 4.

7. A nucleic acid according to any of claims 4 to 5, wherein one or more of the even numbered nucleotides of the second strand are modified by the modification of claim 4.

8. A nucleic acid of any one of claims 3 to 7, comprising a sequence and modifications as shown below:
| **Sequence ID No** | **siRNA ID** | **sequence** | **modifications** |
|---|---|---|---|
| 11 | ALDH2-hcm-6 | 5'-UCUUCUUAAACUGAGUUUC-3' | 5351715262718281517 |
| 12 | | 5'-GAAACUCAGUUUAAGAAGA-3' | 4626353645152646282 |
| 3 | ALDH2-hcm-2 | 5'-AAUGUUUUCCUGCUGACGG-3' | 6254515173547182748 |
| 4 | | 5'-CCGUCAGCAGGAAAACAUU-3' | 3745364728462627251 |
| 17 | ALDH2-hcm-9 | 5'-AUGUAGCCGAGGAUCUUCU-3' | 6181647382846171535 |
| 18 | | 5'-AGAAGAUCCUCGGCUACAU-3' | 2826461735384716361 |
wherein, the specific modifications are depicted by numbers
1=2'F-dU,
2=2'F-dA,
3=2'F-dC,
4=2'F-dG,
5=2'-OMe-rU;
6=2'-OMe-rA;
7=2'-OMe-rC;
8=2'-OMe-rG.

9. A nucleic acid according to any one of claims 1 to 8 comprising two phosphorothioate linkage between each of the three terminal 3' and between each of the three terminal 5' nucleotides on the first strand, and two phosphorothioate linkages between the three terminal nucleotides of the 3' end of the second strand.

10. The nucleic acid according to claims 1 to 9, conjugated to a ligand.

11. A nucleic acid according to claim 10, wherein the ligand comprises (i) one or more N-acetyl galactosamine (GalNac) moieties and derivatives thereof, and (ii) a linker, wherein the linker conjugates the GalNac moieties to the nucleic acid.

12. A conjugated nucleic acid having one of the following structures 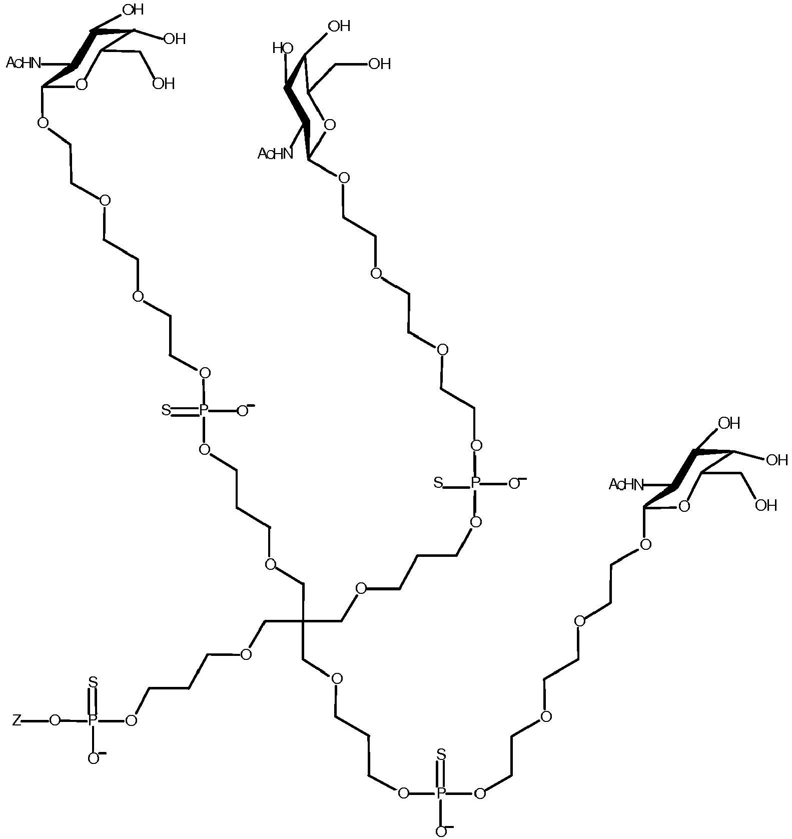 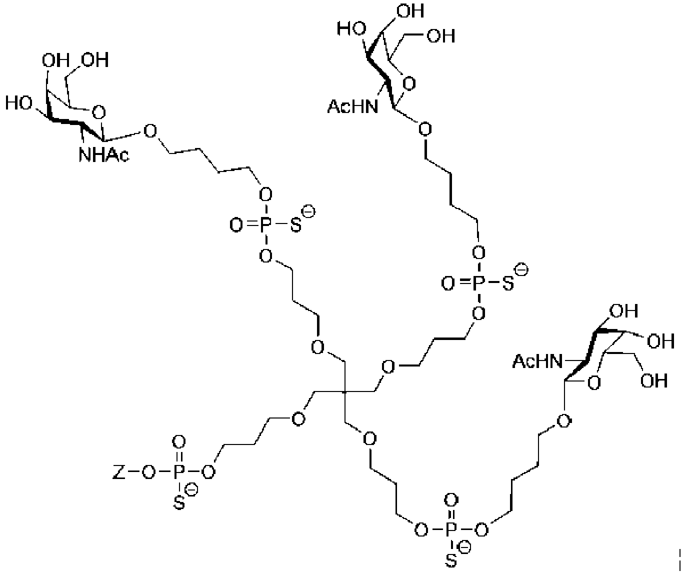 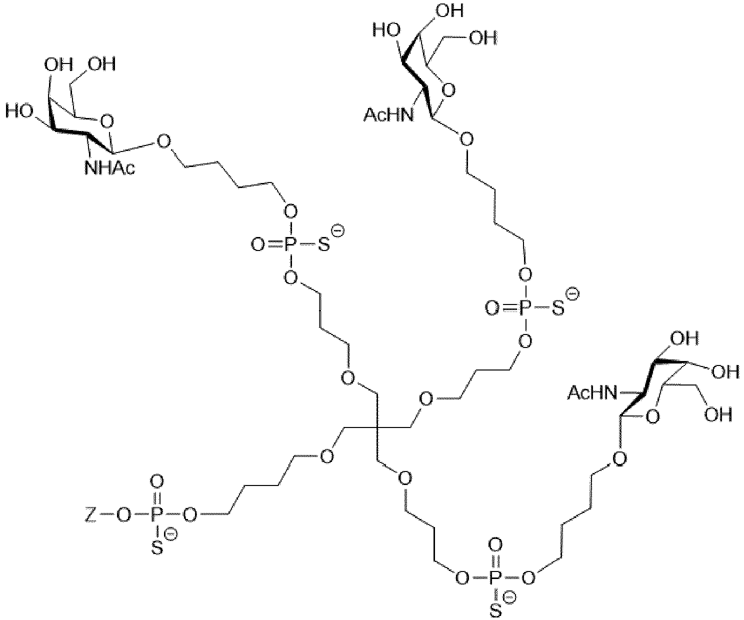 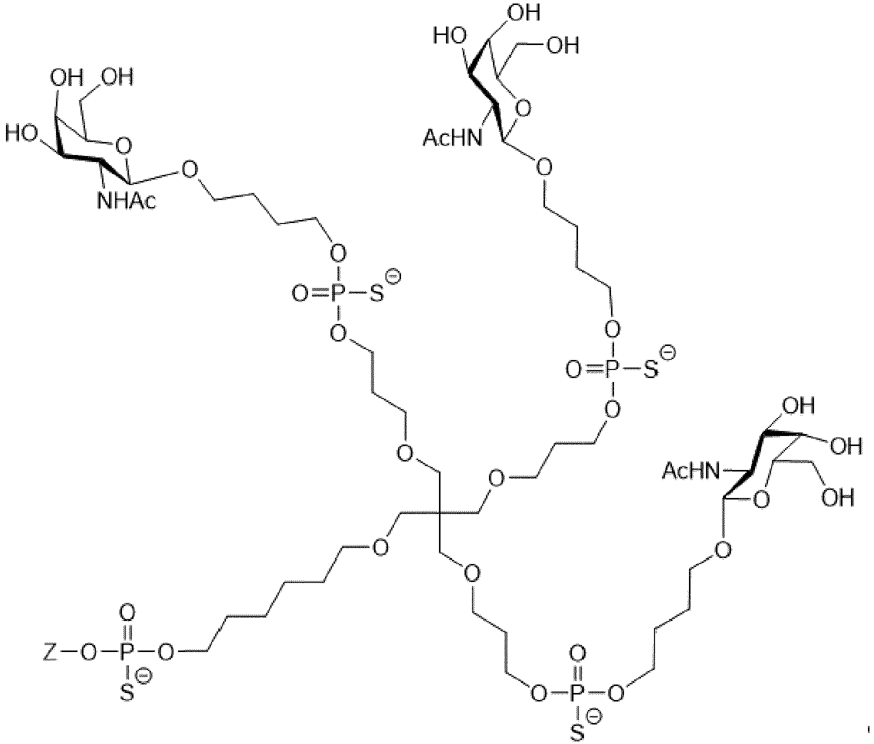 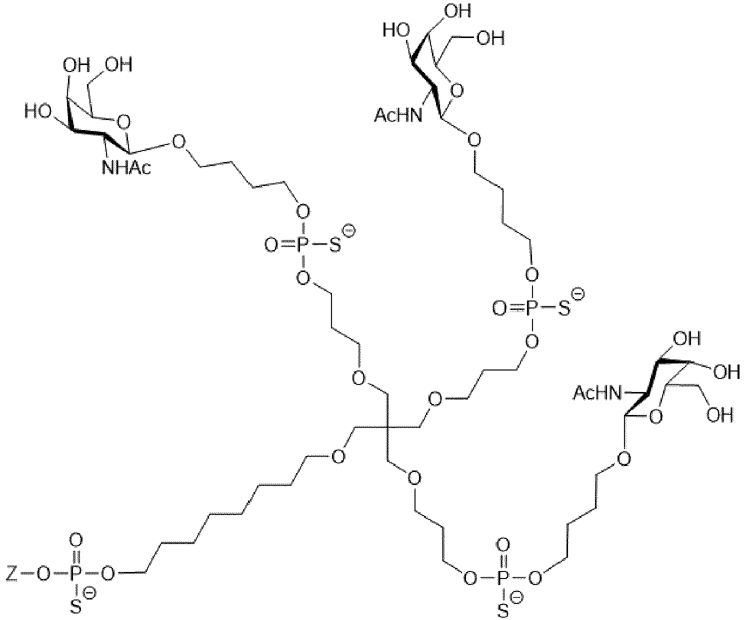 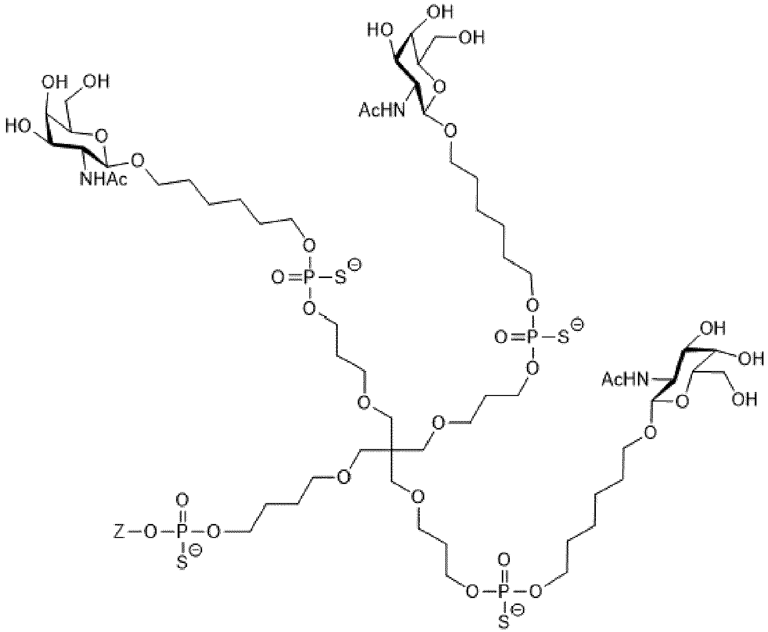 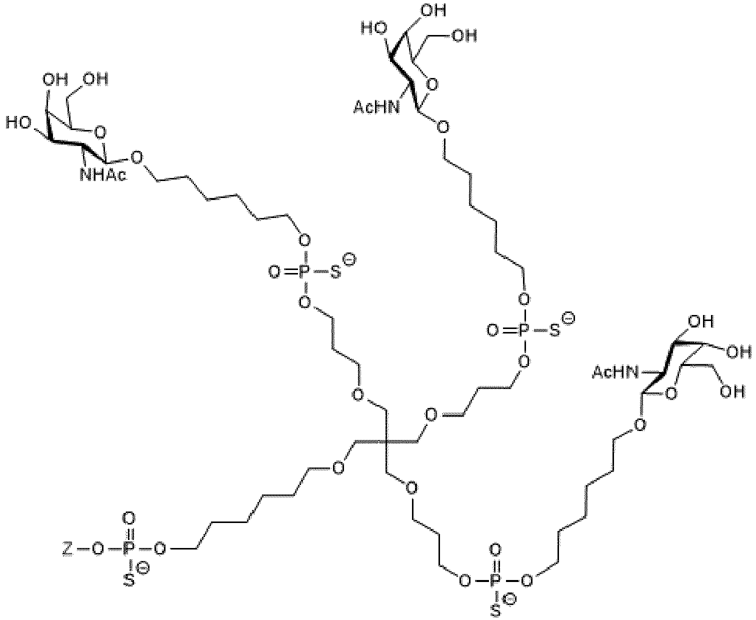 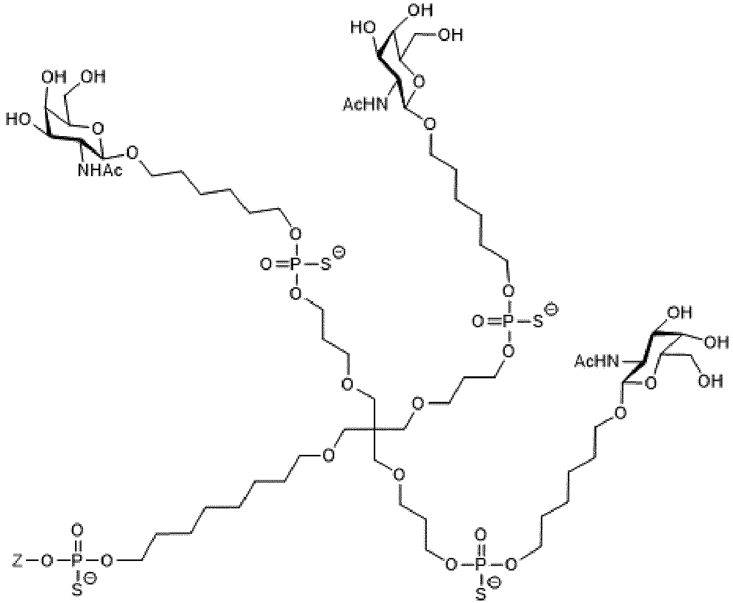 wherein Z is a nucleic acid according to any of claims 1 to 9.

13. A composition comprising a nucleic acid or conjugated nucleic acid of any preceding claim and a physiologically acceptable excipient.

14. A nucleic acid or conjugated nucleic acid according to any preceding claim for use in the prevention or treatment of a disease, disorder or syndrome.

15. A process for making a nucleic acid or conjugated nucleic acid of any one of claims 1 to 14, the process comprising the steps of:
i) synthesising the nucleic acid, and optionally
ii) conjugating the nucleic acid to a ligand
